(19) Europäisches Patentamt
European Patent Office
Office européen des brevets

(11) EP 2 839 267 B1

(12) EUROPÄISCHE PATENTSCHRIFT

(45) Veröffentlichungstag und Bekanntmachung des Hinweises auf die Patenterteilung:
**04.12.2019 Patentblatt 2019/49**

(21) Anmeldenummer: **13716323.4**

(22) Anmeldetag: **17.04.2013**

(51) Int Cl.:
***G01N 21/84*** *(2006.01)*

(86) Internationale Anmeldenummer:
**PCT/EP2013/057999**

(87) Internationale Veröffentlichungsnummer:
**WO 2013/156526 (24.10.2013 Gazette 2013/43)**

# (54) VERFAHREN UND VORRICHTUNG ZUR BESTIMMUNG EINER ANALYTKONZENTRATION IN BLUT

METHOD AND DEVICE FOR DETERMINING THE CONCENTRATION OF AN ANALYTE IN THE BLOOD

PROCÉDÉ ET DISPOSITIF DE DÉTERMINATION D'UNE CONCENTRATION D'ANALYTE DANS LE SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.04.2012 EP 12164805**

(43) Veröffentlichungstag der Anmeldung:
**25.02.2015 Patentblatt 2015/09**

(73) Patentinhaber:
- **F. Hoffmann-La Roche AG**
  **4070 Basel (CH)**
  Benannte Vertragsstaaten:
  **AL AT BE BG CH CY CZ DK EE ES FI FR GB GR HR HU IE IS IT LI LT LU LV MC MK MT NL NO PL PT RO RS SE SI SK SM TR**
- **Roche Diabetes Care GmbH**
  **68305 Mannheim (DE)**
  Benannte Vertragsstaaten:
  **DE**

(72) Erfinder:
- **BALDUS, Susanne**
  **64646 Heppenheim (DE)**
- **SCHULAT, Jochen**
  **68305 Mannheim (DE)**
- **TRICK, Sebastian**
  **68239 Mannheim (DE)**

(74) Vertreter: **Altmann Stößel Dick Patentanwälte PartG mbB**
**Dudenstrasse 46**
**68167 Mannheim (DE)**

(56) Entgegenhaltungen:
**EP-A1- 2 259 058**
**EP-A1- 2 325 624**
**WO-A1-2008/114060**
**US-A- 5 049 487**
**US-A- 5 246 858**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 839 267 B1

## Beschreibung

Gebiet der Erfindung

Die Erfindung betrifft ein Verfahren und eine Vorrichtung zur Bestimmung mindestens einer Konzentration mindestens eines Analyten in Blut. Weiterhin betrifft die Erfindung eine Verwendung eines Benetzungssprungs im zeitlichen Verlauf einer an einem Testelement erfassten optischen Messgröße zur Bestimmung einer Störgröße in Blut. Derartige Verfahren, Vorrichtungen oder Verwendungen werden insbesondere zur Bestimmung einer Blutglukosekonzentration eingesetzt. Grundsätzlich ist jedoch, alternativ oder zusätzlich, auch die Bestimmung eines oder mehrerer anderer Arten von Analyten möglich, insbesondere die Bestimmung eines oder mehrerer Metabolite.

Stand der Technik

Aus dem Stand der Technik sind zahlreiche verschiedene Vorrichtungen und Verfahren zur Bestimmung eines oder mehrerer Analyte in Körperflüssigkeiten, wie beispielsweise Blut, Urin, Speichel, bekannt. Die nachfolgende Erfindung beschreibt insbesondere eine Analytmessung in Blut.

Zur schnellen und einfachen Durchführung derartiger Messungen sind aus dem Stand der Technik insbesondere Testelemente bekannt, welche auf der Verwendung mindestens einer Testchemie basieren. Derartige Testchemien können eingerichtet sein, um bei Anwesenheit des mindestens einen nachzuweisenden Analyten mindestens eine detektierbare Nachweisreaktion durchzuführen, insbesondere eine spezifische Nachweisreaktion, beispielsweise eine optisch detektierbare Nachweisreaktion und/oder eine elektrochemisch detektierbare Nachweisreaktion. Für mögliche Testchemien, die auch im Rahmen der vorliegenden Erfindung eingesetzt werden können, kann beispielsweise auf J. Hönes et al.: Diabetes Technology and Therapeutics, Vol. 10, Supplement 1, 2008, S-10 to S-26 verwiesen werden. Weiterhin kann beispielsweise auf WO 2010/094426 A1 oder auf WO 2010/094427 A1 verwiesen werden. Weiterhin sind aus dem Stand der Technik zahlreiche verschiedene Arten von Testelementen, die die Testchemie umfassen, bekannt. Diesbezüglich kann beispielsweise auf EP 0 302 287 A2, auf EP 0 547 710 A2 oder auf EP 1 593 434 A2 verwiesen werden. Auch andere Arten von Testelementen und/oder Testchemien sind einsetzbar.

EP 2 325 624 A1 beschreibt ein Verfahren und eine Vorrichtung zur Untersuchung einer Körperflüssigkeit. Die Anmeldung setzt sich zum Ziel, ein Übertragungsverhalten des optischen Übertragungssystems einer Vorrichtung durch eine Messung bei zwei verschiedenen Wellenlängen zu kontrollieren. Das Verfahren beinhaltet eine Erfassung von Remissionskurven bei zwei unterschiedlichen Wellenlängen. Diese Messkurven werden einem Fit unterzogen, so dass zwei Fitkurven generiert werden. Aus einem Schnittpunkt der beiden Fitkurven (im Abschnitt zwischen t1 und t2 in Figur 5) wird auf einen Offset geschlossen, und es wird, wie beispielsweise in Absatz [0030] beschrieben wird, eine Offset-Korrektur der Messwerte durchgeführt. Diese Offset-Korrektur ist, wie beispielsweise im Absatz [0023] beschrieben wird, erforderlich, da der Startzeitpunkt der Probenapplikation unsicher bzw. ungenau ist und da, wie sich beispielsweise aus den Absätzen [0005] und [0006] ergibt, sich das Übertragungsverhalten des optischen Übertragungssystems durch das Aufbringen der Körperflüssigkeit ändern kann. Eine Änderung des Remissionsverhaltens während einer Benetzung der Testchemie mit der Probe wird, wie sich beispielsweise aus Absatz [0030] ausdrücklich ergibt, vernachlässigt, und das Remissionsverhalten während der Benetzung wird als konstant angesehen.

US 5,246,858 beschreibt eine Vorrichtung und ein Verfahren zur Bestimmung der Remission einer Testchemie, die mit einer Komponente einer Körperflüssigkeit reagiert. Dabei wird die Testchemie mit einer Strahlenquelle bestrahlt, und Remissionen der Strahlung aus der Chemie werden bestimmt. Wie sich beispielsweise aus Spalte 18, Zeilen 18 ff. ergibt, können dabei zur Auswertung der Kurve Schwellwertverfahren herangezogen werden.

US 5,049,487 offenbart ein Verfahren zur Bestimmung der Anwesenheit eines Analyten in einer Flüssigkeit. Dabei wird eine Reflexionsmessung an einer Reagenz-Matrix durchgeführt.

Insbesondere bei optischen Nachweisverfahren, jedoch grundsätzlich auch bei anderen Nachweisverfahren wie beispielsweise elektrochemischen Nachweisverfahren, tritt dabei in der Praxis häufig die Problematik auf, dass die eigentliche Nachweisreaktion zwar eine hohe Spezifität aufweist, also lediglich bei Anwesenheit des nachzuweisenden Analyten abläuft, nicht hingegen bei anderen Arten von Analyten. Die Detektion dieser Nachweisreaktion, welche beispielsweise anhand einer Remissionsänderung eines die Testchemie enthaltenden Testfeldes abläuft, wird jedoch in vielen Fällen von einer oder mehreren Störgrößen beeinflusst, wie insbesondere den Anteil an roten Blutkörperchen in einer Blutprobe, also den Hämatokrit. So lassen sich in vielen Fällen in der Praxis bei optischen Nachweisreaktionen, insbesondere bei Blutglukosemessungen unter Verwendung optischer Methoden, Abhängigkeiten der Messwerte vom Hämatokrit feststellen. Auch bei elektrochemischen Systemen sind derartige Abhängigkeiten feststellbar, welche jedoch grundsätzlich beispielsweise durch eine Leitfähigkeitsmessung und damit eine direkte Messung des Hämatokrits korrigierbar sind.

Aus US 2010/0159570 A1 sind elektrochemische Verfahren und Vorrichtungen zur Analytdetektion bekannt, welche einen Hämatokrit berücksichtigen. Dabei wird aus einer Füllzeit eines Teststreifens auf eine Viskosität des Blutes

und daraus wiederum auf einen Hämatokrit geschlossen.

In ähnlicher Weise beschreibt JP 2007/303968 A eine Analytmessung in einer Blutprobe, welche ebenfalls eine Hämatokritkorrektur beinhaltet. Dabei wird wiederum ein Testelement mit einem Füllkanal verwendet, und eine Füllgeschwindigkeit des Füllkanals wird bestimmt. Aus dieser Füllgeschwindigkeit wird wiederum auf den Hämatokrit geschlossen.

In WO2006/138226A2 wird eine Anordnung zur Messung der Konzentration eines Analyten beschrieben. Weiterhin wird ein Verfahren beschrieben, bei welchem aus einem zeitlichen Verlauf einer Änderung eines Detektorsignals auf einen Hämatokrit geschlossen wird. Entsprechend wird dann ein Korrekturfaktor in Abhängigkeit des Hämatokrit gewählt, um eine Glukosekonzentration zu berechnen.

WO 2008/114060 A1 beschreibt ein Verfahren und eine Vorrichtung zur Bestimmung einer Zielsubstanz in einer Plasmakomponente einer Vollblutprobe, ohne das Erfordernis, dass die roten Blutzellen vor dem Test aus dem Plasma entfernt werden. Aus einem Offset einer optischen Dichte wird dabei auf die Hämoglobinkonzentration in der Probe geschlossen.

EP 2 259 058 A1 beschreibt ein Verfahren zur Messung eines Hämatokritwerts und eine entsprechende Vorrichtung. Dabei wird mit einer ersten Messwellenlänge Hämoglobin bestimmt und mit einer zweiten Messwellenlänge eine Analyt-abhängige Farbreaktion überwacht.

US 4,935,346 beschreibt ein Verfahren zur Bestimmung der Anwesenheit eines Analyten in einer flüssigen Probe. Dabei wird, nach einem durch eine Benetzung bestimmten Startzeitpunkt, zu einem fest vorgegebenen ersten Messzeitpunkt mittels einer ersten Leuchtdiode und einer ersten Wellenlänge von 700 nm eine Hintergrundmessung zur Korrektur eines Hämatokrits durchgeführt. Zu einem zweiten Zeitpunkt wird dann mittels einer zweiten Leuchtdiode bei 635 nm eine Messung des Glukosegehalts durchgeführt, wobei diese Messung durch den Hämatokrit korrigiert wird.

Das in US 4,935,346 beschriebene Verfahren ist jedoch in der Praxis vergleichsweise aufwendig und erfordert einen vergleichsweise hohen apparativen Aufwand. Insbesondere müssen optische Messungen bei unterschiedlichen Wellenlängen vorgenommen werden, bei welchen das Licht in unterschiedlicher Weise durch die eigentliche Nachweisreaktion und durch den Hämatokrit beeinflusst wird. Weiterhin werden beide optische Messungen bei den verschiedenen Wellenlängen jedoch jeweils sowohl durch den Hämatokrit als auch durch den Blutglukosegehalt beeinflusst. Insofern weisen derartige einfache Messungen eines Hintergrundsignals nach wie vor eine vergleichsweise hohe Messunsicherheit auf.

Die bei herkömmlichen Systemen und Verfahren feststellbare Hämatokritabhängigkeit insbesondere photometrischer Systeme führt, trotz des hohen operativen Aufwands bei bekannten Korrekturverfahren, nach wie vor somit dazu, dass der Hämatokritbereich, innerhalb dessen die bekannten Verfahren und Systeme einsetzbar sind, vergleichsweise gering ist.

## Aufgabe der Erfindung

Es ist daher Aufgabe der vorliegenden Erfindung, Verfahren und Vorrichtungen anzugeben, welche die Nachteile bekannter Verfahren und Vorrichtungen der oben beschriebenen Art zumindest weitgehend vermeiden. Insbesondere sollen Verfahren und Vorrichtungen zur Bestimmung mindestens einer Konzentration mindestens eines Analyten in Blut angegeben werden, welche eine Störgrößenkorrektur mit im Vergleich zu bekannten Vorrichtungen und Verfahren verringertem apparativem Aufwand und dennoch erhöhter Präzision ermöglichen.

## Offenbarung der Erfindung

Diese Aufgabe wird durch ein Verfahren, eine Vorrichtung und eine Verwendung gemäß den unabhängigen Patentansprüchen gelöst. Vorteilhafte Weiterbildungen der Erfindung, welche einzeln oder in beliebiger Kombination realisierbar sind, sind in den abhängigen Patentansprüchen dargestellt.

Allgemein wird darauf hingewiesen, dass die im Folgenden verwendeten Begriffe "aufweisen", "umfassen" oder auch "haben" sowohl abschließend als auch nichtabschließend verstanden werden können. So beinhaltet beispielsweise der Ausdruck "A weist B auf sowohl ausdrücklich die Option, dass A ausschließlich aus B besteht und keine weiteren Komponenten aufweist, als auch die Option, dass A, neben B, mindestens eine weitere Komponente und/oder mindestens einen weiteren Bestandteil aufweist.

In einem ersten Aspekt der vorliegenden Erfindung werden ein Verfahren und eine Vorrichtung zur Bestimmung mindestens einer Konzentration mindestens eines Analyten in Blut vorgeschlagen. Das Verfahren kann insbesondere unter Verwendung einer erfindungsgemäßen Vorrichtung in einer oder mehreren der beschriebenen Ausgestaltungen durchgeführt werden. Alternativ oder zusätzlich kann die Vorrichtung eingerichtet sein, beispielsweise durch Vorhandensein entsprechender Teil-Vorrichtung und/oder Einrichtungen, um ein erfindungsgemäßes Verfahren in einer oder mehreren der dargestellten Ausgestaltungen durchzuführen. Dementsprechend kann für mögliche Ausgestaltungen des Verfahrens auf die Beschreibung der Vorrichtung verwiesen werden oder umgekehrt. Auch andere Ausgestaltungen

des Verfahrens und/oder andere Ausgestaltungen der Vorrichtung sind jedoch grundsätzlich möglich.

Mittels des vorgeschlagenen Verfahrens und der vorgeschlagenen Vorrichtung wird die Konzentration mindestens eines Analyten in Blut bestimmt. Bei dem mindestens einen Analyten kann es sich insbesondere um mindestens einen Metaboliten handeln. Besonders bevorzugt kann es sich bei dem mindestens einen Analyten um Glukose handeln, so dass insbesondere ein Blutglukosenachweis erfolgen kann. Die Konzentration kann grundsätzlich in beliebigen Einheiten angegeben werden, beispielsweise in Einheiten von mg/dl. Auch andere Einheiten sind grundsätzlich denkbar.

Bei dem Verfahren wird mindestens ein Testelement verwendet, wobei das Testelement mindestens eine Testchemie aufweist. Die Testchemie ist eingerichtet, um bei Anwesenheit des Analyten mindestens eine optisch nachweisbare Nachweisreaktion durchzuführen.

Unter einem Testelement ist grundsätzlich ein beliebiges Element zu verstehen, mittels dessen ein qualitativer und/oder quantitativer Nachweis des mindestens einen Analyten möglich ist, in Alleinstellung oder unter Zusammenwirkung mit beispielsweise einem Testgerät. Das Testelement umfasst die mindestens eine Testchemie. Beispielsweise kann das Testelement mindestens ein Trägerelement aufweisen, mit welchem die mindestens eine Testchemie verbunden ist. Insbesondere kann es sich hierbei um ein streifenförmiges und/oder um ein bandförmiges und/oder um ein scheibenförmiges Trägerelement handeln. Das Trägerelement kann beispielsweise ganz oder teilweise aus einem Papiermaterial und/oder einem Kunststoffmaterial und/oder einem Keramikmaterial hergestellt sein. Das Trägerelement kann beispielsweise eine oder mehrere Schichten umfassen.

Insbesondere kann das Testelement ein Einweg-Testelement sein und/oder mindestens ein Einweg-Testelement umfassen, also ein Testelement, welches für genau einen einmaligen Nachweis des Analyten eingerichtet ist und welches anschließend entsorgt werden kann. Das Testelement kann insbesondere streifenförmig ausgestaltet sein, also beispielsweise als Teststreifen ausgestaltet sein. Auch andere Ausgestaltungen sind jedoch grundsätzlich möglich.

Das Testelement kann für das Verfahren oder auch in der weiter unten noch beschriebenen Vorrichtung einzeln durch einen Benutzer bereitgestellt werden, beispielsweise durch Eingeben des Testelements, beispielsweise des Teststreifens in ein Testgerät der Vorrichtung. Alternativ oder zusätzlich zu einer einzelnen, händischen Eingabe des Testelements in das Testgerät können das Verfahren und/oder das Testgerät auch eingerichtet sein, um eine Mehrzahl von Testelementen zu lagern und/oder bereit zu stellen, beispielsweise aus mindestens einem Magazin. Beispielsweise kann das Testgerät eingerichtet sein, um aus dem mindestens einen Magazin einzeln Testelemente in eine Applikationsposition zu bringen, beispielsweise mittels einer entsprechenden Aktorik.

In der Applikationsposition kann das Testelement beispielsweise derart angeordnet sein, dass mindestens eine Aufgabestelle und/oder Applikationsstelle des Testelements, an welcher das Blut, beispielsweise ein Blutstropfen, auf das Testelement aufgebracht werden kann, für einen Benutzer zugänglich ist. Auch andere Ausgestaltungen sind möglich. Das Testelement kann also weiterhin, wie oben erwähnt, mindestens eine Aufgabestelle oder Applikationsstelle aufweisen, an welcher mindestens eine Probe des Bluts auf das Testelement aufgebracht werden kann. Beispielsweise kann dies ein Feld sein, auf welches die Probe aufgebracht werden kann. Alternativ oder zusätzlich kann die Aufgabestelle auch eine Öffnung mindestens eines Kapillarelements, aufweisen. Verschiedene andere Ausgestaltungen sind möglich.

Unter einer Testchemie ist im Rahmen der vorliegenden Erfindung allgemein ein Material zu verstehen, welches eine oder mehrere Komponenten umfassen kann und welches eingerichtet ist, um bei Anwesenheit des Analyten mindestens eine optisch nachweisbare Nachweisreaktion durchzuführen. Bezüglich möglicher Ausgestaltungen der Testchemie kann beispielsweise auf den oben genannten Stand der Technik verwiesen werden. Insbesondere kann die Testchemie mindestens ein Enzym aufweisen, welches spezifisch mit dem nachzuweisenden Analyten reagieren kann. Beispielsweise kann dieses Enzym mindestens eine Oxidase und/oder mindestens eine Hydrogenase aufweisen, beispielsweise Glukoseoxidase und/oder Glukosedehydrogenase. Weiterhin kann die Testchemie mindestens ein Coenzym aufweisen, beispielsweise ein Coenzym ausgewählt aus der Gruppe bestehend aus NAD, cNAD, PQQ und FAD. Bezüglich cNAD kann beispielsweise auf A. v. Ketteler et al.: Fluorescence Properties of Carba Nicotinamide Adenine Dinucleotide for Glucose Sensing, ChemPhysChem 2012, 13, 1302-1306 sowie die darin genannte zusätzliche Literatur verwiesen werden. Weiterhin kann die Testchemie mindestens einen Mediator aufweisen. Weiterhin kann die Testchemie mindestens einen Farbstoff aufweisen, welcher den Ablauf der enzymatischen Nachweisreaktion indizieren kann, beispielsweise durch eine Remissionsmessung und/oder ein Fluoreszenzmessung.

Insbesondere kann die Testchemie mindestens ein Reagens enthalten, das den Analyten in einer Nachweisreaktion umsetzt. Beispielsweise kann das Nachweisreagens mindestens ein enzymatisches Nachweisreagens umfassen. Als Beispiele derartiger analytspezifischer enzymatischer Nachweisreagenzien sind Oxireduktase-Enzyme (z.B. GlucDor/PQQ), Dehydrogenase-Enzyme, Oxidase-Enzyme oder ähnliche Enzyme oder Kombinationen der genannten und/oder anderer Enzyme, insbesondere Glukoseoxidase (GOD) oder Glukosedehydrogenase (beispielsweise FAD-, $NAD^+$- oder PQQ-abhängig) zu nennen. Bei der mindestens einen nachweisbaren Reaktion kann es sich insbesondere um eine optisch und/oder elektrochemisch nachweisbare Reaktion handeln. Auch andere Arten von Reaktionen sind jedoch grundsätzlich möglich. Insbesondere kann es sich um eine Reaktion handeln, bei welcher bei Anwesenheit des mindestens einen Analyten mindestens ein Nachweisstoff gebildet wird. Dabei können auch mehrere Nachweisstoffe gebildet und/oder verwendet werden, die einzeln, in Gruppen oder alle nachgewiesen werden können. Nachweisstoffe

sind insbesondere Stoffe, die sich aufgrund der mindestens einen Nachweisreaktion bilden und/oder die an der mindestens einen Nachweisreaktion beteiligt sind und die direkt oder indirekt nachweisbar sind. Anhand des nachgewiesenen mindestens einen Nachweisstoffs kann beispielsweise der mindestens eine Analyt quantitativ und/oder qualitativ nachgewiesen werden. Für Beispiele für Ausformungen der Testchemie bzw. des Nachweisstoffes kann auf die WO 2010/052307 verwiesen werden. Weitere bevorzugte Ausgestaltungen der Testchemie werden unten noch näher erläutert.

Die Testchemie mit dem Nachweisreagens kann sich insbesondere in mindestens einer Testchemieschicht oder Nachweisschicht befinden. Die Begriffe der Testchemieschicht und der Nachweisschicht werden im Folgenden synonym verwendet. Die mindestens eine Testchemieschicht kann optional noch weitere Substanzen beinhalten, beispielsweise einen oder mehrere Füllstoffe, vorzugsweise umfassend eine oder mehrere Arten von Partikeln, beispielsweise anorganische Partikel. Die Partikel sind vorzugsweise nicht identisch mit dem Nachweisreagens oder zumindest nicht vollständig identisch mit dem Nachweisreagens. Das Nachweisreagens kann grundsätzlich auch eine Mischung mehrerer Nachweisreagenzien oder mehrere Stoffe umfassen, die zusammen das Nachweisreagens bilden können. Die Testchemieschicht kann beispielsweise analog zu der in EP 0 821 234 B1 beschriebenen, ersten Filmschicht des diagnostischen Testträgers ausgestaltet sein. Somit kann die Nachweisschicht bzw. die Testchemieschicht beispielsweise mindestens einen organischen Filmbildner umfassen. Beispielsweise kann dieser mindestens eine Filmbildner eine Polyvinylproprionat-Dispersion umfassen. Alternativ oder zusätzlich können jedoch auch anderer Filmbildner eingesetzt werden. Auch andere Aufbauten der Testchemieschicht sind jedoch grundsätzlich möglich.

Die Testchemie kann insbesondere Bestandteil eines Testchemiefeldes sein, und/oder das Testelement kann mindestens ein Testchemiefeld aufweisen, welches die mindestens eine Testchemie umfassen kann. Unter einem Testchemiefeld ist dabei eine zusammenhängende Menge, beispielsweise eine zusammenhängende Schicht, zu verstehen, welche die Testchemie umfasst oder welche vollständig aus der Testchemie besteht. Diese Schicht kann beispielsweise eine Dicke, insbesondere eine Trockenschichtdicke, von 0,5 $\mu$m bis 500 $\mu$m, insbesondere eine Dicke von 10 $\mu$m bis 100 $\mu$m, aufweisen. Das Testchemiefeld kann insbesondere mindestens eine Detektionsoberfläche und/oder mindestens ein Testfeldfenster aufweisen, welches von außen optisch zugänglich ist, welches also mittels mindestens eines optischen Nachweises beobachtbar ist, so dass die mindestens eine optisch nachweisbare Nachweisreaktion über diese Oberfläche detektierbar ist.

Unter einer optisch nachweisbaren Nachweisreaktion ist allgemein im Rahmen der vorliegenden Erfindung eine beliebige Reaktion zu verstehen, deren Ablauf von der Anwesenheit des nachzuweisenden Analyten abhängt oder zumindest beeinflusst wird, welche optional auch mehrere Teil-Reaktionen umfassen kann und welche durch mindestens ein optisches Messverfahren detektierbar ist. Insbesondere kann eine quantitative Detektion erfolgen. Beispielsweise kann der Begriff der optischen Nachweisbarkeit eine Nachweisbarkeit mittels eines Farbumschlages und/oder mittels einer Farbänderung und/oder mittels mindestens einer Fluoreszenzänderung und/oder mittels mindestens einer Änderung einer Reflektivität, insbesondere einer Änderung einer streuenden Reflektivität, also insbesondere eine Änderung eines Remissionswerts der Testchemie und insbesondere eines Testchemiefeldes umfassen.

Unter einer Remission ist dabei allgemein eine diffuse, also ungerichtete Reflektion von Licht im ultravioletten und/oder sichtbaren und/oder infraroten Spektralbereich zu verstehen. Insbesondere kann die Remission, beispielsweise in willkürlichen Einheiten, als Signal eines das diffus reflektierte Licht empfangenden Reflektors, beispielsweise einer Leuchtdiode, angegeben werden. Weiterhin kann die Remission auch beispielsweise als prozentuale relative Remission angegeben werden (im Folgenden mit % rR bezeichnet), wobei beispielsweise eine Änderung einer beliebigen Remission, beispielsweise des oben genannten Detektorsignals, zu einem Ausgangswert, beispielsweise vor Ablauf der Nachweisreaktion, ins Verhältnis gesetzt wird. Auf diese Weise lassen sich beispielsweise Remissionsänderungen in % rR angeben, welche durch einen Farbumschlag und/oder eine Farbänderung bedingt sind. Allgemein lässt sich die Remission somit in willkürlichen Einheiten, in absoluten Einheiten oder auch in relativen Einheiten angeben, was allgemein vom Begriff der Remission umfasst sein soll.

Alternativ oder zusätzlich zu einer Remission sind jedoch auch andere optische Messgrößen einsetzbar, beispielsweise analoge oder digitale elektrische Signale von optischen Detektoren, Fluoreszenzmesswerte oder ähnliche optische Messgrößen.

Das Verfahren umfasst die im Folgenden beschriebenen Verfahrensschritte. Diese Verfahrensschritte können vorzugsweise, jedoch nicht notwendigerweise, in der dargestellten Reihenfolge durchgeführt werden. Grundsätzlich ist auch eine andere Reihenfolge denkbar. Weiterhin könnten einzelne oder mehrere Verfahrensschritte zeitlich parallel und/oder zeitlich überlappend und/oder einzeln oder zu mehreren wiederholt durchgeführt werden. Weiterhin kann das Verfahren zusätzliche, nicht genannte Verfahrensschritte umfassen.

Bei dem Verfahren wird das Blut auf das Testelement aufgebracht. Beispielsweise kann eine Probe des Bluts, insbesondere ein Blutstropfen, an mindestens einer Aufgabestelle, beispielsweise gemäß der obigen Ausgestaltung, auf das Testelement aufgebracht werden. Besonders bevorzugt ist ein Aufbringen an einer Aufgabestelle in Form einer Öffnung eines Kapillarelements eines Testelements, insbesondere eines Teststreifens. Auch andere Ausgestaltungen sind jedoch grundsätzlich möglich, beispielsweise Ausgestaltungen, bei welchen eine Aufgabestelle auf einer der Detektionsoberfläche des Testchemiefeldes gegenüberliegenden Seite des Testelements angeordnet ist, so dass beispielsweise ein Teststreifen verwendet werden kann, welcher eine Applikationsseite und eine dieser Applikationsseite gegenüberliegende Detektionsseite mit der Detektionsoberfläche aufweist. Das Aufbringen des Bluts auf das Testelement kann allgemein manuell erfolgen oder auch beispielsweise automatisiert.

Weiterhin wird bei dem Verfahren ein zeitlicher Verlauf mindestens einer optischen Messgröße der Testchemie erfasst. Insbesondere kann es sich hierbei um mindestens eine optische Messgröße eines Testchemiefeldes handeln, gemäß der obigen Beschreibung. Unter einer optischen Messgröße ist allgemein im Rahmen der vorliegenden Erfindung eine quantitativ erfassbare Größe und/oder ein quantitativ erfassbares Signal zu verstehen, welches auf der Verwendung einer oder mehrerer optischer Messmethoden basiert. Die optische Messgröße kann insbesondere durch die mindestens eine durch die Nachweisreaktion beeinflussbare optische Eigenschaft der Testchemie beeinflusst werden. Insbesondere kann die optische Messgröße ausgewählt sein aus der Gruppe bestehend aus einer Remission der Testchemie, insbesondere eines die Testchemie umfassenden Testchemiefeldes, bei einer oder mehreren Wellenlängen und einem Fluoreszenzsignal mindestens einer Fluoreszenz der Testchemie. Im Folgenden wird die Erfindung insbesondere unter Verwendung mindestens einer optischen Messgröße in Form mindestens einer Remission eines die Testchemie umfassenden Testchemiefeldes beschrieben. Auch andere Ausgestaltungen sind jedoch grundsätzlich möglich.

Unter einem zeitlichen Verlauf der mindestens einen optischen Messgröße ist eine quantitative Erfassung der mindestens einen optischen Messgröße zu unterschiedlichen Zeitpunkten, also beispielswiese zu 2, 3, 4, 5 oder mehr Zeitpunkten und/oder eine kontinuierliche Erfassung der optischen Messgröße als Funktion der Zeit zu verstehen. Beispielsweise kann der erfasste zeitliche Verlauf eine Funktion umfassen, bei welcher jeweils optische Messgrößen der Testchemie, welche erfasst worden sind, den zugehörigen Messzeitpunkten, zu welchen dieser optischen Messgrößen erfasst wurden, kontinuierlich oder diskontinuierlich zugeordnet werden. Diese Zuordnung kann beispielsweise in Form einer Tabelle und/oder einer Matrix und/oder in Form einer kontinuierlichen Funktion erfolgen. Beispielsweise kann der zeitliche Verlauf als Kurve erfasst werden, wobei jeweils die optischen Messgrößen den jeweiligen Messzeitpunkten zugeordnet werden. Der zeitliche Verlauf kann insbesondere in einem flüchtigen und/oder nicht flüchtigen Datenspeicher abgespeichert werden.

Allgemein wird im Rahmen der vorliegenden Erfindung unter "Zeit" eine beliebige Variable verstanden, welche einen Fortschritt des Verfahrens charakterisieren kann. Diese "Zeit" kann dabei beispielsweise in Einheiten einer tatsächlichen Zeit, beispielsweise in Sekunden, angegeben werden, entsprechend einer realen Uhr. Alternativ oder zusätzlich kann die Zeit auch beispielsweise in anderen Einheiten angegeben werden, beispielsweise entsprechend einer internen "Clock" eines Messgeräts, welches für das Verfahren verwendet wird. Diese "Clock" kann beispielsweise eine regelmäßige Abfolge von Taktzyklen umfassen. Insbesondere kann die mindestens eine Variable, welche zur Charakterisierung der Zeit verwendet wird, linear mit einer realen Zeit und/oder einem Ablauf einer realen Zeit auf einer realen Uhr korrelieren. Allgemein kann die Zeit als absolute Zeit, beispielsweise als absoluter Zeitpunkt, angegeben werden. Alternativ oder zusätzlich kann die Zeit auch beispielsweise relativ angegeben werden, beispielsweise als Zeit ausgehend von einem bestimmten Ereignis und/oder Startzeitpunkt.

Der zeitliche Verlauf der mindestens einen optischen Messgröße kann insbesondere eine Datenmenge umfassen, in welcher, wie oben dargestellt, jeweils Messwerte der mindestens einen optischen Messgröße entsprechenden Messzeitpunkten, zu welchen die optischen Messgrößen erfasst wurden, zugeordnet sind.

Bei dem Verfahren wird insbesondere vorgeschlagen, dass der erfasste zeitliche Verlauf der mindestens einen optischen Messgröße in mindestens zwei Zeitabschnitte des zeitlichen Verlaufs der optischen Messgröße unterteilt wird. Diese mindestens zwei Zeitabschnitte können insbesondere vollständig oder teilweise verschieden und insbesondere disjunkt sein, so dass diese mindestens zwei Zeitabschnitte vorzugsweise nicht überlappen. Auch andere Ausgestaltungen sind jedoch grundsätzlich möglich. Unter einem Zeitabschnitt kann im Rahmen der vorliegenden Erfindung allgemein eine Menge an Messzeitpunkten verstanden werden, wobei die Menge jeweils mindestens zwei Messzeitpunkte umfasst, zu welchen jeweils mindestens eine optische Messgröße erfasst wurde. Beispielsweise kann der Zeitabschnitt eine endliche Menge mit mindestens zwei Messzeitpunkten umfassen. Alternativ kann der Zeitabschnitt auch derart gestaltet werden, so dass keine diskreten Messzeitpunkte enthalten sind, sondern dass während des Zeitabschnitts vollständig oder teilweise kontinuierlich optische Messgrößen erfasst werden, so dass letztendlich eine unendliche Menge an Messzeitpunkten entsteht. Verschiedene Ausgestaltungen sind möglich. Insbesondere kann die Menge an Messzeitpunkten ein Messeintervall umfassen, welches geschlossen, einseitig geschlossenen oder offen ausgestaltet sein kann.

Weiterhin wird vorgeschlagen, dass aus mindestens einem ersten Zeitabschnitt des zeitlichen Verlaufs der optischen Messgröße auf mindestens eine Störgröße des Bluts geschlossen wird. Weiterhin wird vorgeschlagen, dass aus mindestens einem zweiten Zeitabschnitt des zeitlichen Verlaufs auf die Konzentration des Analyten geschlossen wird.

Unter einer Störgröße des Bluts ist dabei allgemein im Rahmen der vorliegenden Erfindung eine beliebige Einflussgröße zu verstehen, welche eine Eigenschaft des Bluts ist, mit Ausnahme der Konzentration des nachzuweisenden Analyten, wobei die Eigenschaft des Bluts die Bestimmung der Konzentration des Analyten beeinflussen kann. Wie oben ausgeführt, kann es sich bei der Störgröße insbesondere um eine Einflussgröße handeln, welche je nach

Blutprobe variieren kann und welche eine Detektion und/oder Messung der mindestens einen optischen Messgröße beeinflusst. Alternativ oder zusätzlich kann es sich auch um eine Einflussgröße handeln, welche den Ablauf der Nachweisreaktion selbst beeinflussen kann, beispielsweise indem durch die Einflussgröße Diffusionsgeschwindigkeiten einer oder mehrerer an der Nachweisreaktion beteiligter Stoffe in dem Blut oder in Bestandteilen des Bluts beeinflusst werden. Insbesondere kann die mindestens eine Störgröße eine Konzentration mindestens einer Störkomponente umfassen, welche in dem Blut enthalten ist. Unter einer Störkomponente ist allgemein ein Bestandteil des Bluts zu verstehen, abgesehen von dem mindestens einen nachzuweisenden Analyten, welcher die Bestimmung der Konzentration des Analyten beeinflussen kann. Vorzugsweise handelt es sich jedoch bei dieser Störkomponente um mindestens eine Komponente, welche an der Nachweisreaktion zum Nachweis des Analyten nicht beteiligt ist.

Insbesondere kann es sich bei dieser Störkomponente um rote Blutkörperchen handeln, so dass die Störgröße insbesondere ein Hämatokrit des Bluts sein kann. Im Rahmen der vorliegenden Erfindung wird unter einem Hämatokrit allgemein ein Anteil roter Blutzellen im Vollblut verstanden. Insbesondere kann es sich bei diesem Anteil um einen Volumenanteil handeln, beispielsweise einen Volumenprozentsatz der roten Blutzellen im Vollblut. Alternativ oder zusätzlich können jedoch auch andere Störkomponenten und/oder Störgrößen erfasst werden.

Bei dem vorgeschlagenen Verfahren wird aus dem ersten Zeitabschnitt des zeitlichen Verlaufs der optischen Messgröße auf die mindestens eine Störgröße des Bluts geschlossen. Insbesondere können zu dieser Bestimmung der Störgröße mindestens zwei optische Messgrößen, die zu zwei unterschiedlichen Zeitpunkten während des ersten Zeitabschnitts erfasst wurden, herangezogen werden. Wie unten noch näher ausgeführt wird, kann dabei insbesondere ein Benetzungssprung erfasst werden und aus dem Benetzungssprung auf die Störgröße geschlossen werden, beispielsweise aus einer Remissionsänderung während des Benetzungssprungs.

Um aus dem zeitlichen Verlauf der optischen Messgröße während des ersten Zeitabschnitts auf die Störgröße zu schließen, kann beispielsweise aus dem zeitlichen Verlauf der optischen Messgröße während des ersten Zeitabschnitts mindestens eine charakteristische Größe bestimmt werden. Wie oben ausgeführt, kann es sich bei dieser charakteristischen Größe beispielsweise um eine Remissionswertänderung während eines Benetzungssprungs oder während eines Teils des Benetzungssprungs handeln. Zwischen den Begriffen der Remission und des Remissionswertes wird dabei hier und im Folgenden nicht weiter unterschieden. Auch andere charakteristische Größen sind grundsätzlich, alternativ oder zusätzlich, einsetzbar.

Allgemein wird im Rahmen der vorliegenden Erfindung unter einem Benetzungssprung eine sprunghafte Veränderung der mindestens einen optischen Messgröße verstanden, welche auf eine Benetzung der Testchemie mit dem Blut zurückzuführen ist. Insbesondere kann die sprunghafte Veränderung derart ausgestaltet sein, dass sich die mindestens eine optische Messgröße durch die Benetzung der Testchemie mit dem Blut um mindestens 1%, vorzugsweise mindestens 2% oder sogar um mindestens 5%, mindestens 10%, mindestens 15% oder sogar mindestens 20% ändert. Der Benetzungssprung ist allgemein derart ausgestaltet, dass durch die Benetzung mit dem Blut sich die mindestens eine optische Messgröße von einem Anfangswert, welcher vor der Benetzung der Testchemie mit dem Blut verzeichnet wird, sprunghaft auf einen Endwert nach der Benetzung der Testchemie mit dem Blut ändert, wobei nach Erreichen des Endwerts eine weitere, langsamere Veränderung der mindestens einen optischen Messgröße eintreten kann, beispielsweise durch Reaktion des Bluts oder von Bestandteilen des Bluts mit der Testchemie. Als Benetzungssprung kann somit das Auftreten der sprunghaften Veränderung der mindestens einen optischen Messgröße selbst bezeichnet werden. Auch eine Differenz zwischen dem Endwert und dem Anfangswert oder ein Betrag dieser Differenz kann an sich als Benetzungssprung bezeichnet werden.

Wie oben ausgeführt, wird der zeitliche Verlauf der mindestens einen optischen Messgröße erfasst, beispielsweise während des ersten Zeitabschnitts. Der erste Zeitabschnitt kann derart gewählt werden, dass der Benetzungssprung vollständig oder zumindest teilweise innerhalb des ersten Zeitabschnitts liegt.

Ein Beginn des Benetzungssprungs, welcher auch als Benetzungszeitpunkt bezeichnet wird, kann beispielsweise durch Vergleich der optischen Messgröße oder einer Änderung derselben mit mindestens einem Schwellwert erkannt werden. Beispielsweise kann die optische Messgröße bei mindestens einer ersten Wellenlänge erfasst werden, wobei aus einer starken Änderung der optischen Messgröße nach dem Aufbringen des Bluts auf das Testelement auf den Benetzungszeitpunkt und/oder den Start des Benetzungssprungs geschlossen werden kann, zu welchen das Blut die Testchemie erreicht und/oder benetzt. Beispielsweise kann darauf geschlossen werden, dass der Benetzungssprung begonnen hat, wenn die optische Messgröße eine zeitliche Änderung erfährt, welche mindestens einem vorgegebenen Schwellwert entspricht und/oder einen vorgegebenen Schwellwert überschreitet. Die mindestens eine optische Messgröße kann beispielsweise direkt oder nach einer Aufbereitung mit dem mindestens einen vorgegebenen Schwellwert verglichen werden. Die Aufbereitung kann beispielsweise eine Filterung und/oder Glättung der mindestens einen optischen Messgröße beinhalten, beispielsweise eine Filterung durch einen Tiefpassfilter und/oder eine Glättung durch Mittelung über vorgegebene Zeitabschnitte und/oder eine Datenreduktion. Auf diese Weise können beispielsweise beim Vergleich der mindestens einen optischen Messgröße mit dem mindestens einen vorgegebenen Schwellwert Rauschen und/oder kurzfristige Artefakte unberücksichtigt bleiben. Wird beispielsweise als optische Messgröße eine Remission der Testchemie und/oder eines die Testchemie umfassenden Testfeldes erfasst, so kann beispielsweise ein Benetzungszeitpunkt festgestellt werden, wenn sich die Remission innerhalb einer Zeitdauer von beispielsweise 1 s um mehr als einen vorgegebenen Schwellwert ändert, beispielsweise um mehr als 0,1-10 %, insbesondere um mehr als 1-5 %. Allgemein können Schwellwerte zur Erkennung des Benetzungszeitpunkts also beispielsweise 0,1 bis 20% betragen, insbesondere 0,1 bis 10 %. Auch andere Schwellwerte, beispielsweise höhere Schwellwerte, kommen grundsätzlich in Betracht.

Auf das Ende des Benetzungssprungs, auch als Endzeitpunkt zu bezeichnen, kann beispielsweise wiederum durch einen Vergleich der mindestens einen optischen Messgröße oder einer Änderung derselben, beispielsweise einer zeitlichen Änderung derselben, mit mindestens einem Schwellwert geschlossen werden. Beispielsweise kann darauf geschlossen werden, dass das Ende des Benetzungssprungs erreicht wird, wenn eine zeitliche Änderung der mindestens einen optischen Messgröße einen weiteren vorgegebenen Schwellwert erreicht oder unterschreitet, nachdem zuvor höhere zeitliche Änderungen erfasst wurden. Wird beispielsweise festgestellt, dass, nachdem vorher ein Beginn des Benetzungssprungs erkannt wurde, die zeitliche Änderung der Remission auf Werte unterhalb eines vorgegebenen Schwellwertes sinkt, so kann auf das Ende des Benetzungssprungs geschlossen werden. Beispielsweise können als Schwellwerte wiederum Änderungsraten von 0,1-10%, insbesondere von 1-5% Remission pro Sekunde angesetzt werden. Der Zeitpunkt, zu welchem die Änderungsrate den vorgegebenen Schwellwert unterschreitet, kann als Endzeitpunkt erkannt werden, selbst wenn danach noch eine langsamere Änderung der Remission, also mit geringeren Änderungsraten, möglich ist.

Als charakteristische Größe des Benetzungssprungs kann dann beispielsweise eine Differenz zwischen der Remission im Benetzungszeitpunkt und der Remission im Endzeitpunkt verwendet werden. Auch andere charakteristische Größen, welche den Benetzungssprung charakterisieren, können grundsätzlich alternativ oder zusätzlich eingesetzt werden.

Um aus der charakteristischen Größe auf die mindestens eine Störgröße zu schließen, kann beispielsweise ein vorgegebener und/oder bestimmbarer Zusammenhang zwischen der charakteristischen Größe und der Störgröße verwendet werden. Beispielsweise kann, wie unten exemplarisch noch näher ausgeführt wird, auf empirischem Wege ein Zusammenhang zwischen einer Remissionswertänderung während eines Benetzungssprungs und der Störgröße, insbesondere einem Hämatokrit des Bluts, erfasst und beispielsweise gespeichert werden, beispielsweise in einer Datenverarbeitungsvorrichtung und/oder einem Datenspeicher eines Testgeräts. Dieser Zusammenhang kann somit beispielsweise durch einfache Messungen bestimmt werden, bei welchen die Störgröße auf anderem Wege, beispielsweise durch klassische Erfassung eines Hämatokrits, bestimmt wird und bei welcher die jeweils zugehörige charakteristische Größe aus dem ersten Zeitabschnitt des zeitlichen Verlaufs der optischen Messgröße abgeleitet und diesem Wert der Störgröße zugeordnet wird. Auf diese Weise kann beispielsweise eine Tabelle, insbesondere eine elektronische Tabelle, erstellt werden, in welcher jeweils charakteristische Größen zugehörigen Störgröße zugeordnet sind oder umgekehrt.

Alternativ oder zusätzlich zur Ermittlung einer oder mehrerer charakteristischer Größen aus dem ersten Zeitabschnitt des zeitlichen Verlaufs der optischen Messgröße sind auch andere Verfahren zur Bestimmung der Störgröße aus dem ersten Zeitabschnitt des zeitlichen Verlaufs der optischen Messgröße möglich. Beispielsweise kann hierfür mindestens ein Mustererkennungsverfahren herangezogen werden, mittels dessen der erste Zeitabschnitt des zeitlichen Verlaufs ausgewertet wird. Auf diese Weise kann beispielsweise der erste Zeitabschnitt des zeitlichen Verlaufs der optischen Messgröße mit einem oder mehreren Vergleichsmuster verglichen werden und entsprechend diesem Vergleich beispielsweise auf die Störgröße geschlossen werden.

Beispielsweise können mehrere Vergleichsmuster in einem Testgerät abgespeichert sein, wobei jedes Vergleichsmuster bekanntermaßen, beispielsweise entsprechend empirischer Messungen bei bekannter Störgröße, einer bestimmten Störgröße zuzuordnen sind. Diese Vergleichsmuster können, beispielsweise sequentiell oder gleichzeitig, mit dem zeitlichen Verlauf der optischen Messgröße in dem ersten Zeitabschnitt verglichen werden, beispielsweise durch ein oder mehrere Korrelationsverfahren und/oder Mustervergleichsverfahren, die dem Fachmann grundsätzlich bekannt sind. Entsprechend dem Ergebnis dieses Vergleichs kann das am besten zum zeitlichen Verlauf der optischen Messgröße während des ersten Zeitabschnitts passende Vergleichsmuster ausgewählt werden und die diesem Vergleichsmuster entsprechende Störgröße verwendet werden.

Wiederum alternativ oder zusätzlich zu einem Mustervergleich des zeitlichen Verlaufs der optischen Messgröße während des ersten Zeitabschnitts kann auch beispielsweise eine analytische Auswertung des zeitlichen Verlaufs der optischen Messgröße während des ersten Zeitabschnitts erfolgen, um über diese analytische Auswertung auf die Störgröße zu schließen. Beispielsweise können ein oder mehrere Anpassfunktionen (Fitfunktionen) vorgegeben werden, mit einem oder mehreren anzupassenden Parametern, wobei die Anpassfunktion an den zeitlichen Verlauf der optischen Messgröße während des ersten Zeitabschnitts angepasst wird. Zu diesem Zweck kann beispielsweise die Methode der kleinsten Fehlerquadrate verwendet werden. Auch andere Anpassmethoden sind denkbar. Entsprechend der auf diese Weise bestimmten Anpassfunktion, beispielsweise entsprechend der auf diese Weise bestimmten einen oder mehreren Anpassparametern, kann dann auf die Störkomponente geschlossen werden. Beispielsweise können wiederum ein oder mehrere Tabellen vorgegeben oder bestimmbar sein, in welchen zu einem oder mehreren Anpassparametern die jeweils zugehörigen Störgrößen hinterlegt sind. Beispielsweise kann während eines Benetzungssprungs eine Anpassung des

zeitlichen Verlaufs der optischen Messgröße in dem ersten Zeitabschnitt an eine Exponentialfunktion erfolgen, welche auch beispielsweise einen Offset aufweisen kann, wobei aus den angepassten Parametern der Exponentialfunktion beispielsweise auf den Hämatokrit und/oder eine andere Störgröße geschlossen werden kann. Auch andere Arten von Anpassfunktionen sind einsetzbar.

Weiterhin wird, wie oben ausgeführt, bei dem vorgeschlagenen Verfahren aus mindestens einem zweiten Zeitabschnitt des zeitlichen Verlaufs der optischen Messgröße auf die Konzentration des nachzuweisenden Analyten geschlossen. Dabei kann in dem zweiten Zeitabschnitt der zeitliche Verlauf derselben optischen Messgröße betrachtet werden, welche auch während des ersten Zeitabschnitts zur Bestimmung der Störgröße herangezogen wird. Insbesondere kann eine optische Messgröße herangezogen werden, welche bei derselben Wellenlänge erfasst wird, wie die optische Messgröße während des ersten Zeitabschnitts. Grundsätzlich sind jedoch auch Verfahren denkbar, bei welchen während des ersten Zeitabschnitts und während des zweiten Zeitabschnitts unterschiedliche optische Messgrößen zur Bestimmung der Störgröße bzw. zur Bestimmung der Analytkonzentration verwendet werden. Während des zweiten Zeitabschnitts wird aus dem zeitlichen Verlauf der optischen Messgröße auf die Konzentration des Analyten geschlossen. Hierzu kann wiederum ein vorgegebener und/oder bekannter und/oder bestimmbarer Zusammenhang zwischen dem zeitlichen Verlauf der optischen Messgröße während des zweiten Zeitabschnitts und der Konzentration des Analyten verwendet werden. Ein derartiger Zusammenhang kann beispielsweise wiederum analytisch und/oder empirisch und/oder semiempirisch bestimmbar sein. Wiederum kann, wie oben beschrieben, dieser Zusammenhang beispielsweise dadurch gegeben sein, dass aus dem zeitlichen Verlauf der optischen Messgröße während des zweiten Zeitabschnitts mindestens eine charakteristische Größe bestimmt wird, welche auch als zweite charakteristische Größe (zur Unterscheidung von der oben beschriebenen, zur Bestimmung der Störgröße verwendbaren charakteristischen Größe) bezeichnet werden kann. Diese zweite charakteristische Größe wird im Folgenden auch als "weitere" charakteristische Größe bezeichnet. Der Begriff "zweite" oder "weitere" charakteristische Größe wird dabei unabhängig davon verwendet, ob eine erste charakteristische Größe vorhanden ist oder nicht und wird als reine Bezeichnung verstanden. Insbesondere kann es sich bei dieser zweiten charakteristischen Größe um mindestens eine optische Messgröße während des zweiten Zeitabschnitts handeln, welche beispielsweise zu einem oder mehreren vorgegebenen, beispielsweise fest vorgegebenen, oder zumindest bestimmbaren und/oder definierten Zeitpunkten innerhalb des zweiten Zeitabschnitts erfasst wurde. Alternativ oder zusätzlich kann die weitere charakteristische Größe auch mindestens einen optische Messgröße umfassen, welche zu mindestens einem Messzeitpunkt innerhalb des zweiten Zeitabschnitts erfasst wurde, zu welchem sich der zeitliche Verlauf der optischen Messgrößen nicht mehr oder im Wesentlichen nicht mehr ändert. Beispielsweise kann der zeitliche Verlauf der optischen Messgröße in dem zweiten Zeitabschnitt überwacht werden, und es könnten Änderungsraten der optischen Messgrößen festgestellt werden. Liegt beispielsweise die Änderungsrate, beispielsweise die Änderung der optischen Messgrößen innerhalb eines vorgegebenen Zeitraums, unterhalb einer vorgegebenen Schwelle, so kann festgestellt werden, dass sich der zeitliche Verlauf der optischen Messgröße im Wesentlichen nicht mehr ändert. Beispielsweise kann vorgegeben werden, dass sich eine relative Remission um nicht mehr als 0,1 bis 10 % pro 1 s ändern soll, insbesondere nicht mehr als 1 bis 5 % pro 1 s ändern soll, beispielsweise um nicht mehr als 2 % pro 1 s ändern soll, damit der zeitliche Verlauf der Remission sich im Sinne der Vorgaben im wesentlichen nicht mehr ändert. Ist diese Bedingung erfüllt, so kann beispielsweise ein anschließend erfasster optischer Messwert als weitere charakteristische Größe verwendet werden. Aus dieser weiteren charakteristischen Größe kann dann beispielsweise auf die Konzentration des Analyten geschlossen werden, beispielsweise indem diese charakteristische Größe, insbesondere ein optischer Messwert, über eine entsprechende vorgegebene, bekannte oder bestimmbare Beziehung in einer Analytkonzentration in dem Blut umgerechnet wird. Derartige Umrechnungsverfahren sind grundsätzlich bekannt, da beispielsweise bereits bei herkömmlichen Blutzuckermessgeräten Remissionswerte in entsprechende Glukosekonzentrationen umgewandelt werden.

Alternativ oder zusätzlich zur Verwendung einer oder mehrerer charakteristischer Größen zur Auswertung des zeitlichen Verlaufs der optischen Messgröße während des zweiten Zeitabschnitts kommen auch, analog zur Auswertung des ersten Zeitabschnitts, andere Verfahren in Betracht. So können beispielsweise wiederum, analog zur obigen Beschreibung des ersten Zeitabschnitts, ein oder mehrere Mustervergleiche und/oder eine Anpassung einer oder mehrerer Anpassfunktionen an den zeitlichen Verlauf während des zweiten Zeitabschnitts erfolgen. Wiederum kann beispielsweise aus einem Vergleich mit einer Mehrzahl von hinterlegten Mustern auf eine Konzentration des Analyten geschlossen werden, und/oder es kann aus ermittelten Anpassparametern einer oder mehrerer Anpassfunktionen, beispielsweise wiederum Exponentialfunktionen mit und/oder ohne Offset, auf die Konzentration des Analyten geschlossen werden. Wie auch bei der Auswertung des ersten Zeitabschnitts kann auch bei der Auswertung des zweiten Zeitabschnitts zu diesem Zweck eine Datenverarbeitungsvorrichtung verwendet werden, beispielsweise ein Microcomputer, welcher in einem Testgerät zur Verwendung in dem Verfahren eingesetzt wird, beispielsweise in einer Auswertevorrichtung eines Testgeräts.

Im Unterschied zu den oben beschriebenen, aus dem Stand der Technik bekannten statischen Messverfahren wird erfindungsgemäß somit ein Verfahren vorgeschlagen, bei welchem ein zeitlicher Verlauf einer optischen Messgröße der Testchemie erfasst wird und in mindestens zwei Zeitabschnitte unterteilt wird, wobei sowohl die Störgröße als auch

die Analytkonzentration aus dem zeitlichen Verlauf, jedoch in unterschiedlichen Zeitabschnitten, bestimmt werden. Das vorgeschlagene Verfahren kann insbesondere der häufig zu beobachtenden Tatsache Rechnung tragen, dass sich die Störgröße und die Konzentration des Analyten in unterschiedlichen Zeitabschnitten auf den zeitlichen Verlauf der optischen Messgröße auswirken können. So macht sich üblicherweise eine Störgröße, insbesondere die Konzentration einer Störkomponente und besonders ein Hämatokrit, im Blut überwiegend in einem anfänglichen ersten Zeitabschnitt des zeitlichen Verlaufs einer Remission bemerkbar, insbesondere in einem anfänglichen Benetzungssprung. Zu diesem anfänglichen Zeitabschnitt, zu welchem somit der Hämatokrit und/oder eine andere Art von Störgröße aus dem zeitlichen Verlauf der optischen Messgröße abgeleitet werden kann, ist jedoch üblicherweise die Nachweisreaktion, welche einen Rückschluss auf die Konzentration des Analyten ermöglicht, noch nicht oder lediglich in geringem Umfang abgelaufen, beispielsweise indem nicht mehr als 20 %, insbesondere nicht mehr als 10 % und besonders bevorzugt nicht mehr als 5 % eines gesamten möglichen Umsatzes der Nachweisreaktion stattgefunden hat. Zu einem späteren Zeitpunkt, während des zweiten Zeitabschnittes, ist dann die Nachweisreaktion vorzugsweise vollständig oder weitgehend vollständig, beispielsweise zu mindestens 80 %, insbesondere zu mindestens 90 % und besonders bevorzugt zu mindestens 95 %, abgelaufen, so dass beispielsweise mittels der optischen Messgröße eine optisch nachweisbare Veränderung der Testchemie nachgewiesen werden kann, über welche dann wiederum auf die Konzentration des Analyten abgeschlossen werden kann.

Beispielsweise kann innerhalb des ersten Zeitabschnitts die Änderung der mindestens einen optischen Messgröße im Wesentlichen auf physikalische Vorgänge wie beispielsweise auf Solvatationsvorgänge und/oder auf Diffusionsprozesse zurückzuführen sein. Insofern kann beispielsweise der erste Zeitabschnitt derart gewählt werden, dass während dieses ersten Zeitabschnitts keine oder lediglich eine vernachlässigbare Umsetzung der Testchemie und/oder kein oder lediglich ein vernachlässigbarer Ablauf der Nachweisreaktionen zu verzeichnen ist, beispielsweise eine Umsetzung von 5 % oder weniger, insbesondere keine enzymatische Umsetzung. Im zweiten Zeitabschnitt kann die mindestens eine optische Messgröße hingegen im Wesentlichen oder zusätzlich bedingt sein durch die analytische Nachweisreaktion der Testchemie. In diesem Fall kann beispielsweise der Ablauf der Nachweisreaktion, beispielsweise die Enzymumsetzung, durch die Konzentration des nachzuweisenden Analyten, beispielsweise Glukose, im Blut bestimmt sein. In diesem Fall kann die Umsetzung der Testchemie und/oder eines aktiven Bestandteils der Testchemie, beispielsweise eine enzymatische Umsetzung, vorzugsweise erheblich größer sein als die oben genannten Prozentzahlen, beispielsweise größer und insbesondere deutlich größer als 5%.

Diese Möglichkeit der zeitlichen Trennung des zeitlichen Verlaufs der optischen Messgröße in mehrere Zeitabschnitte, die in unterschiedlicher Weise durch die Störgröße und durch die Konzentration des Analyten beeinflusst werden, ermöglicht es beispielsweise, die Störgröße und die Konzentration des Analyten aus ein und derselben optischen Messgröße zu bestimmen. Im Gegensatz zu einer einmaligen, statischen Messung, wie beispielsweise im oben beschriebenen Stand der Technik, ermöglicht jedoch die Auswertung des zeitlichen Verlaufs der optischen Messgröße einen geringeren apparativen Aufwand und eine gute Präzision bei der Bestimmung der Störgröße und der Analytkonzentration. An Stelle oder zusätzlich der oben beschriebenen spektralen Trennung der Messungen der Störgröße und der Analytkonzentration kann erfindungsgemäß insbesondere eine zeitliche Trennung der Bestimmung der Störgröße und der Analytkonzentration erfolgen, durch Unterteilung des zeitlichen Verlaufs in den ersten Zeitabschnitt und den zweiten Zeitabschnitt.

Diesbezüglich wird allgemein angemerkt, dass diese Unterteilung vollständig oder teilweise erfolgen kann. Insbesondere kann es sich bei dem ersten Zeitabschnitt und bei dem zweiten Zeitabschnitt jeweils um eine zusammenhängende Menge von aufeinander folgenden Messzeitpunkten handeln, denen jeweils Messwerte der optischen Messgröße zugeordnet sind. Der erste Zeitabschnitt kann insbesondere vollständig oder teilweise vor dem zweiten Zeitabschnitt angeordnet sein. Zwischen dem ersten Zeitabschnitt und dem zweiten Zeitabschnitt kann eine zeitliche Lücke angeordnet sein. Alternativ können der erste Zeitabschnitt und der zweite Zeitabschnitt jedoch auch zumindest teilweise überlagert sein. Wiederum alternativ können der erste Zeitabschnitt und der zweite Zeitabschnitt auch beispielsweise zeitlich unmittelbar aneinander angrenzen.

Der erste Zeitabschnitt kann beispielsweise mit Beginn einer Messung gestartet werden, beispielsweise bei einem Start eines Testgeräts, welches zur Durchführung des Verfahrens verwendet wird. Alternativ kann der erste Zeitabschnitt auch beispielsweise beginnen, wenn ein Start eines Benetzungssprungs im Verlauf der optischen Messgröße erkannt wird, beispielsweise indem die optische Messgröße, welche zuvor beispielsweise näherungsweise konstant sein kann, eine starke zeitliche Änderung aufweist. Beispielsweise kann dieser Start des Benetzungssprungs wiederum durch Vergleich der optischen Messgröße mit einem Schwellwert bestimmt werden. Überschreitet die zeitliche Änderung der optischen Messgröße diesen Schwellwert erstmalig, so kann beispielsweise auf den Start des Benetzungssprungs geschlossen werden, und der jeweilige Zeitpunkt kann beispielsweise als Startzeitpunkt des ersten Zeitabschnitts gewählt werden.

Als Ende des ersten Zeitabschnitts kann beispielsweise ein Ende eines Benetzungssprungs verwendet werden. Üblicherweise weist der zeitliche Verlauf der optischen Messgröße, beispielsweise der Remission, am Ende des Benetzungssprungs einen Knick auf, welcher beispielsweise in einer Unstetigkeit der ersten Ableitung des zeitlichen Verlaufs erkannt werden kann. Dieser Knick lässt sich dadurch erklären, dass während des Benetzungssprungs die optische Messgröße überwiegend durch die Benetzung der Testchemie mit Blut oder Blutbestandteilen beeinflusst wird. Anschließend wird dann die optische Messgröße durch den Verlauf der Nachweisreaktion und die dadurch bestimmte Änderung mindestens einer optische nachweisbaren Eigenschaft der Testchemie bedingt. Am Übergang zwischen beiden Bereichen bildet sich üblicherweise der oben beschriebene Knickpunkt. Somit kann, beispielsweise wiederum durch Vergleich der optischen Messgröße und/oder einer Ableitung des zeitlichen Verlaufs der optischen Messgröße, der Zeitpunkt des Knicks bestimmt werden und beispielsweise als Endzeitpunkt für den ersten Zeitabschnitt gewählt werden. Gleichzeitig kann der Zeitpunkt, zu welchem dieser Knick auftritt, als Startzeitpunkt für den zweiten Zeitabschnitt gewählt werden. Als Endzeitpunkt für den zweiten Zeitabschnitt kann dann beispielsweise ein Zeitpunkt gewählt werden, zu welchem sich der zeitliche Verlauf der optischen Messgröße nicht mehr oder im Wesentlichen nicht mehr ändert, beispielsweise gemäß der oben beschriebenen Schwellwertbedingung, nach welcher sich innerhalb eines vorgegebenen Zeitintervalls die optische Messgröße nur noch maximal um einen vorgegebenen Schwellwert oder um weniger als einen vorgegebenen Schwellwert ändert.

Die Wahl des ersten Zeitabschnitts und des zweiten Zeitabschnitts kann somit insbesondere nachträglich erfolgen, im Rahmen einer Auswertung des zeitlichen Verlaufs der optischen Messgröße, welche insbesondere durch mindestens eine Datenverarbeitungsvorrichtung erfolgen kann. Die oben beschriebenen Verfahren lassen sich insbesondere leicht automatisieren, so dass beispielsweise automatisch im Rahmen einer Auswertung des zeitlichen Verlaufs der optischen Messgröße der Startzeitpunkt des ersten Zeitabschnitts als der Zeitpunkt des Starts eines Benetzungssprungs gewählt werden kann, als Endzeitpunkt des ersten Zeitabschnitts und als Startzeitpunkt des zweiten Zeitabschnitts das Ende des Benetzungssprungs, und als Endzeitpunkt des zweiten Zeitabschnitts ein Zeitpunkt, zu welchem sich die optische Messgröße im Wesentlichen nicht mehr ändert.

Nachdem diese Einteilung des zeitlichen Verlaufs der optischen Messgröße erfolgt ist, beispielsweise durch eine Datenverarbeitungsvorrichtung, können, beispielsweise gemäß einem oder mehreren der oben beschriebenen Algorithmen, aus den zeitlichen Verläufen während des ersten Zeitabschnitts und des zweiten Zeitabschnitts die Störgröße und die Analytkonzentration bestimmt werden. Als charakteristische Größe während des ersten Zeitabschnitts kann beispielsweise eine Differenz zwischen der optischen Messgröße zum Startzeitpunkt des ersten Zeitabschnitts und der optischen Messgröße zum Endzeitpunkt des ersten Zeitabschnitts gewählt werden, also beispielsweise eine Änderung der optischen Messgröße während des ersten Zeitabschnitts. Auch diese Bestimmung der charakteristischen Größe zur Ermittlung der Störgröße des Bluts, insbesondere des Hämatokrits, lässt sich leicht automatisieren und somit leicht durch eine Datenverarbeitungsvorrichtung durchführen. Als zweite charakteristische Größe zur Bestimmung der Analytkonzentration aus dem zweiten Zeitabschnitt des zeitlichen Verlaufs der optischen Messgröße lässt sich beispielsweise, wie oben ausgeführt, eine optische Messgröße zu einem Zeitpunkt verwenden, welcher einen fest vorgegebenen zeitlichen Abstand zu einem Startzeitpunkt des zweiten Zeitabschnitts aufweist und/oder zu welchem sich der zeitliche Verlauf der optischen Messgröße im Wesentlichen nicht mehr ändert.

Das vorgeschlagene Verfahren lässt sich auf verschiedene Weisen vorteilhaft weiterbilden. Wie oben beschrieben, kann der erste Zeitabschnitt insbesondere ein anfänglicher Zeitabschnitt des zeitlichen Verlaufs der optischen Messgröße sein. Dieser anfängliche Zeitabschnitt kann mit dem Aufbringen des Bluts auf das Testelement und/oder mit einem Einschalten eines Testgeräts und/oder mit einer Erkennung eines Starts eines Benetzungssprungs in dem zeitlichen Verlauf beginnen. Auch andere Startzeitpunkte sind möglich. Der zweite Zeitabschnitt kann insbesondere dem ersten Zeitabschnitt zeitlich nachgeordnet sein.

Der zeitliche Verlauf der optischen Messgröße in dem ersten Zeitabschnitt kann insbesondere, wie oben mehrfach beschrieben, einen Benetzungssprung der optischen Messgröße umfassen. Unter einem Benetzungssprung ist dabei im Rahmen der vorliegenden Erfindung eine zeitliche Veränderung der optischen Messgröße zu verstehen, welche durch eine Benetzung der Testchemie oder von Teilen der Testchemie mit Blut oder mit Blutbestandteilen bedingt ist. Da die Nachweisreaktion, welche zu einer optisch nachweisbaren Veränderung mindestens einer Eigenschaft der Testchemie führt, grundsätzlich erst nach einer Benetzung der Testchemie mit Blut oder Blutbestandteilen ablaufen kann, ist die Änderung der optischen Messgröße während des Benetzungssprungs üblicherweise unabhängig von der Analytkonzentration. Der Benetzungssprung ist üblicherweise einzig und alleine dadurch bedingt, dass eine trockene Testchemie in der Regel einen anderen Wert der optischen Messgröße aufweist als eine mit Blut oder Blutbestandteilen benetzte Testchemie, also eine "nasse" Testchemie. Da die Benetzung üblicherweise durch die Störgröße, insbesondere den Hämatokrit, stark beeinflusst werden kann, lässt sich typischerweise, wie oben ausgeführt, aus dem Benetzungssprung zumindest auch und/oder zumindest näherungsweise auf die Störgröße und insbesondere den Hämatokrit schließen. Der Benetzungssprung kann, wie oben ausgeführt, insbesondere durch eine Benetzung der Testchemie mit dem Blut und/oder mit Bestandteilen des Bluts verursacht werden, insbesondere bevor die optisch nachweisbare Nachweisreaktion in signifikanten Umfang abgelaufen ist.

Wie oben ausgeführt, können die mindestens eine optische Messgröße und deren zeitlicher Verlauf während des ersten Zeitabschnitts und des zweiten Zeitabschnitts insbesondere bei derselben Wellenlänge erfasst werden. Allgemein kann die optische Messgröße beispielsweise bei mindestens einer ersten Wellenlänge erfasst werden, wobei

aus einer starken Änderung der optischen Messgröße nach dem Aufbringen des Bluts auf das Testelement auf einen Benetzungszeitpunkt und/oder einen Start eines Benetzungssprungs geschlossen werden kann, zu welchen das Blut die Testchemie erreicht und/oder benetzt. Unter einer starken Änderung der optischen Messgröße kann beispielsweise eine Änderung verstanden werden, welche mindestens einen vorgegebenen Schwellwert entspricht und/oder mehr als ein vorgegebener Schwellwert ist. Die mindestens eine optische Messgröße kann beispielsweise direkt oder nach einer Aufbereitung mit dem mindestens einen vorgegebenen Schwellwert verglichen werden. Die Aufbereitung kann beispielsweise eine Filterung und/oder Glättung der mindestens einen optischen Messgröße beinhalten, beispielsweise eine Filterung durch einen Tiefpassfilter und/oder eine Glättung durch Mittelung über vorgegebene Zeitabschnitte und/oder eine Datenreduktion. Auf diese Weise können beispielsweise beim Vergleich der mindestens einen optischen Messgröße mit dem mindestens einen vorgegebenen Schwellwert Rauschen und/oder kurzfristige Artefakte unberücksichtigt bleiben. Wird beispielsweise als optische Messgröße eine Remission der Testchemie und/oder eines die Testchemie umfassenden Testfeldes erfasst, so kann beispielsweise ein Schwellwert von 1% bis 10 % Remissionsänderung vorgegeben sein, beispielsweise 7 % bis 8 %. Liegt also beispielsweise eine Änderung der Remission bei der ersten Wellenlänge oberhalb dieses Schwellwerts, so kann im Rahmen des vorliegenden Verfahrens auf eine Benetzung der Testchemie mit dem Blut, also auf einen Benetzungszeitpunkt, und damit auf den Beginn eines Benetzungssprungs geschlossen werden.

Wie oben beschrieben, kann auf diese Weise insbesondere auf einen Benetzungszeitpunkt und damit einen Start des Benetzungssprungs geschlossen werden. Dieser Zeitpunkt kann insbesondere, wie oben ausgeführt, als Startzeitpunkt des ersten Zeitabschnitts gewählt werden. Der erste Zeitabschnitt und der zweite Zeitabschnitt können zumindest teilweise dem Benetzungszeitpunkt zeitlich nachgeordnet sein. Besonders bevorzugt kann dann die optische Messgröße in dem ersten Zeitabschnitt mit dem zweiten Zeitabschnitt bei mindestens einer zweiten Wellenlänge erfasst werden, wobei vorzugsweise die zweite Wellenlänge gleich der ersten Wellenlänge sein kann.

Die optische Messgröße kann, wie oben ausgeführt, insbesondere eine Messgröße sein, welche anhand einer Remissionsmessung und/oder einer streuenden Reflektion an der Testchemie, insbesondere einer Testfeldoberfläche eines die Testchemie umfassenden Testfeldes, bestimmt wird. Dementsprechend kann die optische Messgröße insbesondere mindestens einen Remissionswert der Testchemie umfassen, welcher bei einer oder mehreren Wellenlängen erfasst werden kann.

Zur Erfassung der optischen Messgröße in dem ersten Zeitabschnitt und in dem zweiten Zeitabschnitt können insbesondere jeweils mittels mindestens einer Abfragelichtquelle mindestens ein Abfragelichtstrahl auf die Testchemie eingestrahlt werden. Weiterhin kann jeweils mittels mindestens eines Detektors mindestens ein von der Testchemie ausgehender Antwortlichtstrahl erfasst werden. So kann in dem ersten Zeitabschnitt beispielsweise mittels mindestens einer ersten Abfragelichtquelle mindestens ein erster Abfragelichtstrahl auf die Testchemie eingestrahlt werden und mittels mindestens eines ersten Detektors mindestens ein von der Testchemie ausgehender erster Antwortlichtstrahl erfasst werden. Während des zweiten Zeitabschnitts kann entsprechend mittels mindestens einer zweiten Abfragelichtquelle mindestens ein zweiter Abfragelichtstrahl auf die Testchemie eingestrahlt werden und mittels mindestens eines zweiten Detektors mindestens ein von der Testchemie ausgehender Antwortlichtstrahl erfasst werden. Dabei kann die erste Abfragelichtquelle identisch oder verschieden zu der zweiten Abfragelichtquelle sein. Der erste Detektor kann identisch oder verschieden von dem zweiten Detektor ausgestaltet sein. Der Abfragelichtstrahl und der Antwortlichtstrahl können in dem ersten Zeitabschnitt und/oder in dem zweiten Zeitabschnitt jeweils dieselbe Wellenlänge und/oder verschiedene Wellenlängen aufweisen. So kann der erste Abfragelichtstrahl während des ersten Zeitabschnitts dieselbe Wellenlänge aufweisen wie der erste Antwortlichtstrahl während des ersten Zeitabschnitts. Weiterhin kann während des zweiten Zeitabschnitts der zweite Abfragelichtstrahl dieselbe Wellenlänge aufweisen wie der zweite Antwortlichtstrahl. Weiterhin können der erste Abfragelichtstrahl und der zweite Abfragelichtstrahl dieselbe Wellenlänge oder verschiedene Wellenlängen aufweisen. Weiterhin können der erste Antwortlichtstrahl und der zweite Antwortlichtstrahl dieselbe Wellenlänge oder auch verschiedene Wellenlängen aufweisen.

Besonders bevorzugt ist es, wenn die Abfragelichtstrahlen in dem ersten Zeitabschnitt in dem zweiten Zeitabschnitt dieselbe Wellenlänge und/oder dieselbe spektralen Eigenschaften aufweisen. Dies kann insbesondere bedeuten, dass der optische Messwert während des ersten Zeitabschnitts und des zweiten Zeitabschnitts mit derselben Wellenlänge oder mit denselben Wellenlängen ermittelt werden. Beispielsweise können die Abfragelichtstrahlen in dem ersten Zeitabschnitt und dem zweiten Zeitabschnitt mit derselben Lichtquelle, beispielsweise mit einer oder mehreren Leuchtdioden, erzeugt werden. Weiterhin können die Antwortlichtstrahlen in dem ersten Zeitabschnitt und dem zweiten Zeitabschnitt dieselbe Wellenlänge und/oder dieselben spektralen Eigenschaften aufweisen. Unter dem Begriff spektrale Eigenschaften können dabei insbesondere eine spektrale Zusammensetzung und/oder ein Intensitätsverlauf der Antwortlichtstrahlen als Funktion der Wellenlänge oder der Frequenz verstanden werden, welcher auch normiert sein kann oder welcher als relativer Intensitätsverlauf erfasst werden kann. So können sich die Antwortlichtstrahlen in dem ersten Zeitabschnitt und in dem zweiten Zeitabschnitt zwar grundsätzlich in ihrer Intensität und/oder Amplitude unterscheiden, wobei jedoch die spektrale Zusammensetzung und/oder ein normiertes Spektrum der Antwortlichtstrahlen in dem ersten Zeitabschnitt und dem zweiten Zeitabschnitt vorzugsweise identisch sind oder beispielsweise eine Abweichung (beispielsweise ermittelt durch eine Korrelationsfunktion) von nicht mehr als 20 %, insbesondere von nicht mehr als 10 %, aufweisen. Insbesondere kann die Erfassung der mindestens einen optischen Eigenschaft in dem ersten Zeitabschnitt und dem zweiten Zeitabschnitt also bei gleicher Wellenlänge und/oder im gleichen Wellenlängenbereich erfolgen. Insbesondere kann eine Erfassung der Antwortlichtstrahlen während des ersten Zeitabschnitts und des zweiten Zeitabschnitts mittels desselben Detektors erfolgen.

Wie oben ausgeführt, kann der Antwortlichtstrahl in dem ersten Zeitabschnitt und/oder in dem zweiten Zeitabschnitt jeweils insbesondere durch Reflektion und/oder Streuung des Abfragelichtstrahls an der Testchemie gebildet werden. Auch andere Nachweisverfahren sind jedoch grundsätzlich möglich, wie oben ausgeführt, beispielsweise Fluoreszenzmessungen.

Der Abfragelichtstrahl in dem ersten Zeitabschnitt und/oder dem zweiten Zeitabschnitt kann insbesondere eine Wellenlänge aufweisen, ausgewählt aus der Gruppe bestehend aus: 635 nm, 660 nm, 770 nm, 815 nm und 880 nm. Auch andere Wellenlängen sind jedoch grundsätzlich einsetzbar. Bei den genannten Wellenlängen kann es sich insbesondere um Wellenlängen eines Spektrums des Abfragelichtstrahls handeln, beispielsweise um zentrale Wellenlängen und/oder Peak-Wellenlängen. Insbesondere können als Abfragelichtstrahl schmalbandige Lichtstrahlen verwendet werden, beispielsweise mit einer spektralen Halbwertsbreite (FWHM) von nicht mehr als 50 nm, insbesondere von nicht mehr als 40 nm und besonders bevorzugt von nicht mehr als 30 nm und sogar nicht mehr als 20 oder nicht mehr als 10 nm. Beispielsweise können als Abfragelichtstrahlen Lichtstrahlen von Lichtquellen mit einer FWHM von 5-60 nm, insbesondere von 10-20 nm, verwendet werden, insbesondere Leuchtdioden mit den genannten spektralen Eigenschaften.

Weitere Aspekte des vorgeschlagenen Verfahrens berücksichtigen insbesondere, dass die Störgröße nicht nur bestimmt werden kann, sondern dass unter Berücksichtigung der Störgröße auch eine Korrektur der Bestimmung der Analytkonzentration erfolgen kann. So kann mittels der Störgröße, insbesondere des Hämatokrits, insbesondere mindestens eine Korrektur ermittelt werden, beispielsweise eine Korrekturfunktion und/oder ein Korrekturfaktor und/oder ein Korrektur-Offset, wobei aus dem zweiten Zeitabschnitt unter Berücksichtigung der Korrektur eine korrigierte Konzentration des Analyten ermittelt wird. Die korrigierte Konzentration des Analyten kann dabei nachträglich ermittelt werden oder auch gleichzeitig mit der Bestimmung der eigentlichen Analytkonzentration. So kann beispielsweise zunächst aus dem zeitlichen Verlauf der optischen Messgröße während des zweiten Zeitabschnitts eine Roh-Konzentration des Analyten ermittelt werden, welche anschließend mittels der aus der Störgröße bestimmten Korrektur korrigiert wird. Alternativ oder zusätzlich kann die Korrektur jedoch auch bereits bei der Auswertung des zweiten Zeitabschnitts berücksichtigt werden. So kann insbesondere aus dem zeitlichen Verlauf der optischen Messgröße während des zweiten Zeitabschnitts unmittelbar unter Berücksichtigung der Korrektur eine korrigierte Konzentration des Analyten abgeleitet werden. Dies kann beispielsweise dadurch erfolgen, dass aus dem zeitlichen Verlauf der optischen Messgröße während des zweiten Zeitabschnitts eine "zweite" charakteristische Größe abgeleitet wird, welche dann mittels eines bekannten oder bestimmbaren Zusammenhangs in eine korrigierte Analytkonzentration umgewandelt wird, wobei in den Zusammenhang die zuvor bestimmte Störgröße einfließt. Auch andere Ausgestaltungen und/oder Kombinationen der genannten Ausgestaltungen der Berücksichtigung der Störgröße bei der Bestimmung einer korrigierten Analytkonzentration sind denkbar.

Allgemein ist diesbezüglich darauf hinzuweisen, dass unter einer "Korrektur" eine beliebige Manipulation, insbesondere eine beliebige Rechenoperation, zu verstehen ist, welche den Einfluss der mindestens einen Störgröße auf die Bestimmung der Analytkonzentration aus dem zeitlichen Verlauf der optischen Messgröße während des zweiten Zeitabschnitts verringert, minimiert oder vollständig eliminiert, insbesondere derart, dass erfindungsgemäß bestimmte Analytkonzentrationen bei unterschiedlichen Graden an Störeinfluss durch die Störgröße dennoch vergleichbar sind. Insbesondere kann die Korrektur dazu führen, dass auch bei stark unterschiedlichen Werten der Störgröße aus dem zeitlichen Verlauf der optischen Messgröße während des zweiten Zeitabschnitts im Wesentlichen dieselbe Analytkonzentration abgeleitet werden kann, wenn die tatsächliche Analytkonzentration identisch ist. Unter "im Wesentlichen dieselbe" kann beispielsweise eine Abweichung der auf diese Weise bestimmten Analytkonzentrationen verstanden werden, welche nicht mehr als 10 % beträgt, insbesondere nicht mehr als 5 %. Beispielsweise kann die Korrektur derart erfolgen, dass, bei ein und derselben tatsächlichen Analytkonzentration, insbesondere bei ein und derselben tatsächlichen Blutglukosekonzentration, jedoch bei einem Hämatokritunterschied von 10 %, die korrigierten Konzentrationen des Analyten, insbesondere die korrigierten Blutglukosekonzentrationen, um nicht mehr als 10 % voneinander abweichen, insbesondere um nicht mehr als 5 %.

Insbesondere kann die Korrektur derart erfolgen, dass die korrigierte Analytkonzentration einer theoretischen Analytkonzentration entspricht, die gemessen worden wäre, wenn die Störgröße einen vorgegebenen Wert zum Zeitpunkt der Messung gehabt hätte. Beispielsweise kann ein bestimmter Bezugswert der Störgröße vorgegeben werden, wobei die Korrektur derart durchgeführt wird, dass die Analytkonzentration in eine Analytkonzentration bei Vorliegen des Bezugswerts der Störgröße umgerechnet wird. Beispielsweise kann die korrigierte Analytkonzentration eine Analytkonzentration bei einem Bezugswert von 43 % Hämatokrit sein.

Die Korrektur kann allgemein beispielsweise wiederum in Form eines Zusammenhangs und/oder einer Tabelle und/oder in anderer Form hinterlegt sein, beispielsweise in einem Testgerät, insbesondere in einer Auswertevorrichtung

eines Testgeräts und besonders bevorzugt in einem Datenspeicher und/oder einer Datenverarbeitungsvorrichtung einer Auswertevorrichtung eines Testgeräts. Beispielsweise kann die Korrektur in Form einer oder mehrerer Korrekturfunktionen und/oder in Form einer oder mehrerer Korrekturfaktoren und/oder in Form eines oder mehrerer Korrektur-Offsets hinterlegt sein, welche beispielsweise eine stetige Funktion der zuvor ermittelten Störgröße sein können und/oder welche im Rahmen einer punktweisen Zuordnung, beispielsweise in einer Tabelle und insbesondere einer elektronischen Tabelle, entsprechend der ermittelten Störgröße bestimmt werden können. Unter einem Korrektur-Offset kann beispielsweise ein Fehlerbeitrag der Störgröße zu der Analytkonzentration und/oder zu der optischen Messgröße verstanden werden, beispielsweise ein additiver oder ein subtraktiver Beitrag.

So kann beispielsweise zunächst aus dem zeitlichen Verlauf der optischen Messgröße während des ersten Zeitabschnitts eine Bestimmung der mindestens einen Störgröße erfolgen. Anschließend kann, entsprechend der ermittelten Störgröße, beispielsweise entsprechend des ermittelten Hämatokrits, eine Wahl einer Korrektur, insbesondere eines Korrekturfaktors und/oder eines Korrektur-Offsets und/oder einer Korrekturfunktion, erfolgen, beispielsweise durch Auswahl eines der Störgröße zugeordneten Korrekturfaktors und/oder eines der Störgröße zugeordneten Korrektur-Offsets aus einer Tabelle und/oder Liste. Weiterhin kann, beispielsweise gemäß dem oben beschriebenen Verfahren, aus dem zeitlichen Verlauf der optischen Messgröße während des zweiten Zeitabschnitts, eine Roh-Analytkonzentration bestimmt werden. Anschließend kann die Roh-Analytkonzentration beispielsweise mit der Korrektur beaufschlagt werden. Beispielsweise kann dies dadurch erfolgen, dass die Roh-Analytkonzentration mit dem Korrekturfaktor multipliziert wird und/oder dass die Roh-Analytkonzentration um den Korrektur-Offset erhöht oder erniedrigt wird, so dass eine korrigierte Analytkonzentration bestimmt werden kann. Alternativ oder zusätzlich kann, wie oben beschrieben, die Korrektur auch bereits unmittelbar bei der Auswertung des zeitlichen Verlaufs der optischen Messgröße während des zweiten Zeitabschnitts erfolgen. Beispielsweise kann zunächst aus dem ersten Zeitabschnitt die Störgröße ermittelt werden. Anschließend kann, beispielsweise durch Auswahl aus einer Liste, ein Zusammenhang ausgewählt werden, mittels dessen aus dem zeitlichen Verlauf der optischen Messgröße während des zweiten Zeitabschnitts auf die Analytkonzentration geschlossen werden kann, wobei der Zusammenhang bereits die Störgröße berücksichtigt, so dass die auf diese Weise gewonnene Analytkonzentration bereits eine korrigierte Analytkonzentration ist. Beispielsweise können während des zweiten Zeitabschnitts, wie oben beschrieben, ein oder mehrere Remissionswerte erfasst werden, beispielsweise zu einem Endzeitpunkt der Nachweisreaktion. Dieser mindestens eine Remissionswert kann mittels einer Umrechnung, die bereits den Hämatokrit berücksichtigt, unmittelbar in eine korrigierte Analytkonzentration umgerechnet werden.

Die Korrektur kann, wie oben beschrieben, beispielsweise zuvor bestimmt und/oder bestimmbar sein und kann beispielsweise in einem Testgerät hinterlegt sein. Zur Bestimmung der Korrektur lassen sich insbesondere empirische oder semi-empirische Verfahren einsetzten. So kann beispielsweise eine Messreihe durchgeführt werden, mittels derer entsprechende Korrekturfaktoren und/oder Korrektur-Offsets und/oder andere Arten von Korrekturfunktionen bestimmt werden können. Diese Versuchsreihe kann beispielsweise derart ausgestaltet werden, dass Blutproben hergestellt werden, welche dieselbe tatsächliche Analytkonzentration, beispielsweise dieselbe tatsächliche Glukosekonzentration, aufweisen, jedoch unterschiedliche Hämatokrite. Der jeweilige Hämatokrit der einzelnen Proben kann unabhängig bestimmt werden, beispielsweise bekannter Zentrifugierverfahren. Soll der Einfluss anderer Arten von Störgrößen bestimmt werden, so können diese Störgrößen in der Regel entsprechend mittels unabhängiger Nachweisverfahren bestimmt werden. Anschließend kann mittels des Teststreifens jeweils ein Rohwert der Analytkonzentration bestimmt werden, und es kann für jede Störgröße der Messreihe, beispielsweise für jeden Hämatokrit, ein Korrekturfaktor und/oder ein Korrektur-Offset bestimmt werden, um die Roh-Analytkonzentration in die tatsächliche Analytkonzentration umzurechnen. Diese Kalibration oder Eichung stellt nur eine von vielen empirischen Möglichkeiten dar, um entsprechende Korrekturen empirisch oder semi-empirisch zu bestimmen. Auch theoretische Modelle zum Einfluss der Störgröße lassen sich grundsätzlich zum Ermitteln einer Korrektur einsetzten. Die auf diese Weise empirisch oder semi-empirisch bestimmten Korrekturen lassen sich beispielsweise im Rahmen einer Tabelle und/oder Matrix und/oder Liste hinterlegen, beispielsweise in einem Datenspeicher und/oder einer Datenverarbeitungsvorrichtung einer Auswertevorrichtung eines Testgeräts. Beispielsweise kann der Datenspeicher einen flüchtigen und/oder einen nicht flüchtigen Datenspeicher umfassen. Beispielsweise kann der Datenspeicher ein EEPROM umfassen. Alternativ oder zusätzlich zu einer Hinterlegung der Korrekturen in einem Datenspeicher und/oder einer Datenverarbeitungsvorrichtung einer Auswertevorrichtung eines Testgeräts können eine oder mehrere der genannten Korrekturen auch von dem Testgerät eingelesen werden, beispielsweise über mindestens eine Schnittstelle, beispielsweise einer drahtgebundenen und/oder einer drahtlosen Schnittstelle. Beispielsweise können eine oder mehrere Korrekturen auch in einem externen Gerät und/oder externen Datenspeicher gespeichert sein und an das Testgerät übertragen werden. Beispielsweise kann als externer Datenspeicher mindestens ein so genannter ROM-Key verwendet werden, welcher eine oder mehrere Korrekturen in gespeicherter Form enthält und welcher zum Zweck einer Datenübertragung mit einer Schnittstelle des Testgeräts verbunden werden kann. Alternativ oder zusätzlich kann auch beispielsweise eine oder mehrere Korrekturen auf einem RFID-Chip als externem Datenspeicher gespeichert sein, so dass beispielsweise eine Funkübertragung der mindestens einen Korrektur an das Testgerät erfolgen kann. Wiederum alternativ oder zusätzlich kann auch mindestens ein Datenübertragungsnetzwerk verwendet werden, um mindestens eine Korrektur an das Testgerät zu übertragen, beispielsweise ein drahtgebundenes und/oder ein drahtloses Datenübertragungsnetzwerk, insbesondere das Internet und/oder ein drahtloses Telekommunikationsnetzwerk. Wie oben ausgeführt, kann die Korrektur insbesondere eine Abhängigkeit der optischen Messgröße von der Störgröße umfassen, insbesondere einen Zusammenhang zwischen einem Remissionswert der Testchemie und einem Hämatokrit. Auch andere Ausgestaltungen sind möglich.

Weitere mögliche Ausgestaltungen betreffen insbesondere die Bestimmung der Konzentration des Analyten aus dem zeitlichen Verlauf der optischen Messgröße während des zweiten Zeitabschnitts. Wie oben beschrieben, kann zu diesem Zweck insbesondere mindestens eine "zweite" charakteristische Größe aus dem zeitlichen Verlauf abgeleitet werden, aus der sich die Analytkonzentration bestimmen lässt. Insbesondere kann, wie oben ausgeführt, eine Veränderung der optischen Messgröße in dem zweiten Zeitabschnitt erfasst werden, wobei zur Bestimmung der Konzentration des Analyten die optische Messgröße zu einem Zeitpunkt verwendet wird, der auch als Endzeitpunkt bezeichnet werden kann und zu welchem die Nachweisreaktion im Wesentlichen vollständig abgelaufen ist. Insbesondere kann der Zeitpunkt, zu welchem die optische Messgröße erfasst und zur Bestimmung der Konzentration des Analyten verwendet wird, derart gewählt werden, dass zu diesem Zeitpunkt eine zeitlichen Änderung der optischen Messgröße unterhalb eines vorgegebenen Schwellwerts liegt.

Weitere mögliche Ausgestaltungen des erfindungsgemäßen Verfahrens betreffen die Verwendung bevorzugter Testelemente. Insbesondere kann das Verfahren derart durchgeführt werden, dass das Blut an mindestens einer Aufgabestellung auf das Testelement aufgebracht wird. Diese Aufgabestelle kann unmittelbar an der Testchemie angeordnet sein oder auch von der Testchemie beabstandet angeordnet sein, beispielsweise um mindestens 1 mm, insbesondere um mindestens 2 mm und beispielsweise um 3 bis 10 mm. Insbesondere wenn die Aufgabestelle beabstandet von der Testchemie angeordnet ist, können das Blut oder Bestandteile des Bluts von der Aufgabestelle zu der Testchemie transferiert werden. Zu diesem Zweck kann mindestens ein Transferelement vorgesehen sein, welches Bestandteil des Testelements sein kann oder welches auch Bestandteil eines mit dem Testelement zusammen wirkenden Testgeräts sein kann. Besonders bevorzugt ist es, wenn das Transferelement mindestens ein Kapillarelement aufweist, welches Bestandteil des Testelements sein kann und welches eingerichtet sein kann, um mittels Kapillarkräften das Blut oder Bestandteile des Bluts von der mindestens einen Aufgabestelle zu der Testchemie, beispielsweise einem die Testchemie umfassenden Testchemiefeld, zu transferieren.

Weiterhin kann das Verfahren derart durchgeführt werden, dass das Blut oder die Bestandteile des Bluts auf dem Weg zwischen der Aufgabestelle und der Testchemie mindestens ein Abtrennelement passieren, wobei in dem Abtrennelement mindestens ein Bestandteil des Bluts aus dem Blut abgetrennt werden kann. Dieses mindestens einen Abtrennelement kann beispielsweise mindestens eine Abtrennschicht und/oder mindestens ein Sieb und/oder mindestens ein Gewebe und/oder mindestens ein Flies umfassen. Das mindestens eine Abtrennelement kann auch insbesondere Bestandteil eines Schichtaufbaus sein, welcher auch die Testchemie umfassen kann. So kann beispielsweise das Testelement ein Testchemiefeld umfassen, welches in einem Schichtaufbau das mindestens eine Abtrennelement und mindestens eine Testchemieschicht umfasst. Beispielsweise kann das mindestens eine Abtrennelement als Abtrennschicht ausgestaltet sein, wobei das Blut und/oder Bestandteile des Bluts auf dem Weg von der Aufgabestelle zu der Testchemieschicht zunächst die mindestens eine Abtrennschicht passieren müssen. Derartige Abtrennelemente sind aus dem Stand der Technik der Testelemente, beispielsweise dem oben beschriebenen Stand der Technik, grundsätzlich bekannt. Insbesondere kann das Abtrennelement mindestens eine mit der Testchemie in Kontakt stehende Abtrennschicht umfassen.

Zwischen der Aufgabestelle und der Testchemie kann, wie oben ausgeführt, insbesondere mindestens ein Kapillarelement vorgesehen sein. Insbesondere kann dieses Kapillarelement einen Kapillarspalt umfassen. Das Kapillarelement kann insbesondere eine Länge von mindestens 0,5 mm und besonderes bevorzugt von mindestens 5 mm aufweisen, beispielsweise eine Länge von 5 mm bis 50 mm und insbesondere eine Länge von 10 mm bis 20 mm. Auch andere Ausgestaltungen sind möglich.

Das erfindungsgemäße Verfahren in einer oder mehreren der oben beschriebenen oder nachfolgend noch näher beschriebenen Ausgestaltungen kann insbesondere ganz oder teilweise unter Verwendung eines Computers und/oder eines Computernetzwerks und/oder eines Computerprogramms durchgeführt werden. So wird in einem weiteren Aspekt der vorliegenden Erfindung ein Computerprogramm vorgeschlagen, welches eingerichtet ist, um nach einem Laden in einen Arbeitsspeicher eines Computers oder Prozessors oder Computernetzwerks das erfindungsgemäße Verfahren vollständig oder teilweise durchzuführen. Insbesondere kann die Erfassung des zeitlichen Verlaufs der mindestens einen optischen Messgröße der Testchemie vollständig oder teilweise computerimplementiert oder computerunterstützt durchgeführt werden. Alternativ oder zusätzlich kann auch der Verfahrensschritt, in welchem aus dem mindestens einen ersten Zeitabschnitt des zeitlichen Verlaufs der optischen Messgröße auf die mindestens eine Störgröße des Bluts geschlossen wird, vollständig oder teilweise computerimplementiert oder computerunterstützt durchgeführt werden. Wiederum alternativ oder zusätzlich kann der Verfahrensschritt, in welchem aus dem mindestens einen zweiten Zeitabschnitt des zeitlichen Verlaufs auf die Konzentration des Analyten geschlossen wird, vollständig oder teilweise computerimplementiert oder computerunterstützt durchgeführt werden. Wiederum alternativ oder zusätzlich

können Kombinationen der genannten Verfahrensschritte und/oder anderer Verfahrensschritte durch das Computerprogramm durchführbar sein.

Das vorgeschlagene Computerprogramm kann insbesondere in einer Auswertevorrichtung einer unten noch näher beschriebenen Vorrichtung durchgeführt werden. Entsprechend kann die Auswertevorrichtung programmtechnisch eingerichtet sein, um das vorgeschlagene Computerprogramm durchzuführen. Beispielsweise kann die Auswertevorrichtung mindestens eine Datenverarbeitungsvorrichtung zur Durchführung des Computerprogramms aufweisen, wenn das Computerprogramm in die Auswertevorrichtung oder die Datenverarbeitungsvorrichtung geladen wird. Die Datenverarbeitungsvorrichtung kann beispielsweise einen Mikroprozessor umfassen. Alternativ oder zusätzlich zur Durchführung in der Auswertungsvorrichtung kann das Computerprogramm auch ganz oder teilweise in einer anderen Art von Computer oder Computernetzwerk durchgeführt werden. Beispielsweise kann es sich hierbei um einen Arztcomputer und/oder einen Patientencomputer handeln. Die Auswertung kann also auch ganz oder teilweise getrennt von der Messung erfolgen. So können beispielsweise die Messdaten, also insbesondere der zeitliche Verlauf der mindestens einen optischen Messgröße, in den Computer geladen werden, beispielsweise einen flüchtigen oder nicht flüchtigen Speicher des Computers. Anschließend oder gleichzeitig kann eine Auswertung unter Durchführung des vorgeschlagenen Computerprogramms erfolgen.

In einem weiteren Aspekt wird ein Datenträger vorgeschlagen, beispielsweise ein flüchtiger oder nicht-flüchtiger Datenträger, auf welchem eine Programmstruktur gespeichert ist, mittels derer nach einem Laden der Programmstruktur in einen Arbeitsspeicher eines Computers oder Computernetzwerks das erfindungsgemäße Verfahren oder Teile desselben von dem Computer oder Computernetzwerk ausführbar sind.

In einem weiteren Aspekt wird ein Computer oder Computernetzwerk vorgeschlagen, welches eingerichtet ist, um das vorgeschlagene Verfahren vollständig oder teilweise, beispielsweise die oben genannten Verfahrensschritte, in einer oder mehreren der oben beschriebenen oder nachfolgend noch näher beschriebenen Ausgestaltungen durchzuführen. Beispielsweise können der Computer oder das Computernetzwerk mindestens einen Mikroprozessor zur Durchführung des Verfahrens oder von Teilen desselben umfassen.

In einem weiteren Aspekt der vorliegenden Erfindung wird, wie oben ausgeführt, eine Vorrichtung zur Bestimmung mindestens einer Konzentration mindestens eines Analyten in Blut vorgeschlagen. Die Vorrichtung kann insbesondere, wie oben ausgeführt, eingerichtet sein, um ein erfindungsgemäßes Verfahren in einer oder mehreren der dargestellten Varianten oder der im Folgenden noch darzustellenden Varianten durchzuführen. Dabei können Verfahrensschritte auch einzeln durchgeführt werden. Die Vorrichtung kann dementsprechend einzeln durchgeführt werden. Die Vorrichtung kann dementsprechend entsprechende Einrichtungen umfassen, um alle, mehrere oder einzelne der genannten Verfahrensschritte durchzuführen.

Die Vorrichtung umfasst mindestens ein Testelement, wobei das Testelement mindestens eine Testchemie aufweist. Die Testchemie ist eingerichtet, um bei Anwesenheit des Analyten mindestens eine optisch nachweisbare Nachweisreaktion durchzuführen. Das Blut ist auf das Testelement aufbringbar. Bezüglich möglicher Ausgestaltungen des Testelements, der Testchemie und des Aufbringens des Bluts kann auf die obige Beschreibung verwiesen werden.

Die Vorrichtung weist weiterhin mindestens eine optische Nachweisvorrichtung auf. Die optische Nachweisvorrichtung ist eingerichtet, um einen zeitlichen Verlauf mindestens einer optischen Messgröße der Testchemie zu erfassen. Unter einer optischen Nachweisvorrichtung ist dabei allgemein eine Vorrichtung zu verstehen, welche zu mehreren Zeitpunkten kontinuierlich oder diskontinuierlich die mindestens eine optische Messgröße, beispielsweise mindestens eine optische Messgröße gemäß der obigen Beschreibung, erfassen kann und vorzugsweise abspeichern kann, beispielswiese in einem Datenspeicher. Zu diesem Zweck kann, wie unten noch näher ausgeführt wird, die optische Nachweisvorrichtung beispielsweise eine oder mehrere Lichtquellen zur Bestrahlung und/oder Beleuchtung der Testchemie und/oder zur Beaufschlagung der Testchemie mit mindestens einem Lichtstrahl umfassen. Beispielsweise können diese eine oder mehreren Lichtquellen mindestens eine Halbleiterlichtquelle umfassen, beispielsweise mindestens eine Leuchtdiode und/oder mindestens eine Laserdiode. Weiterhin kann die optische Nachweisvorrichtung, alternativ oder zusätzlich, mindestens einen Detektor zur Erfassung mindestens eines von der Testchemie ausgehenden Lichts aufweisen, vorzugsweise wiederum mindestens einen Halbleiterdetektor, beispielsweise mindestens eine Fotodiode. Weiterhin kann die Nachweisvorrichtung weitere Elemente umfassen, beispielsweise mindestens ein optisches Element, wie beispielsweise mindestens eine Linse und/oder mindestens ein Umlenkelement, wie beispielsweise mindesten einen Spiegel und/oder mindestens ein Prisma. Weiterhin kann die Nachweisvorrichtung eine oder mehrere elektronische Komponenten umfassen, beispielsweise zur Ansteuerung und/oder auch Auswertung der mindestens einen optionalen Lichtquelle und/oder des mindestens einen optionalen Detektors und/oder zur Speicherung und/oder Verarbeitung von Signalen des mindestens einen optionalen Detektors.

Die Vorrichtung umfasst weiterhin mindestens eine Auswertevorrichtung. Die Auswertevorrichtung ist eingerichtet, um aus mindestens einem ersten Zeitabschnitt des zeitlichen Verlaufs der optischen Messgröße auf mindestens eine Störgröße des Bluts, insbesondere einen Hämatokrit des Bluts, zu schließen. Weiterhin ist die Auswertevorrichtung eingerichtet, um aus mindestens einem zweiten Zeitabschnitt des zeitlichen Verlaufs auf die Konzentration des Analyten zu schließen. Bezüglich möglicher einsetzbarer Verfahren, um aus dem ersten Zeitabschnitt auf die Störgröße und aus

dem zweiten Zeitabschnitt auf die Konzentration des Analyten zu schließen, kann beispielsweise auf die obige Beschreibung oder auf die nachfolgende Beschreibung bevorzugter Ausführungsbeispiele verwiesen werden. Die Auswertevorrichtung kann insbesondere eine oder mehrere elektronische Komponenten umfassen, beispielsweise mindestens eine Datenverarbeitungsvorrichtung wie beispielsweise einen Mikrocomputer. Die Datenverarbeitungsvorrichtung kann insbesondere programmtechnisch eingerichtet sein, beispielsweise um einen oder mehrere der oben genannten Verfahrensabschnitte durchzuführen. So kann die Datenverarbeitungsvorrichtung mittels eines Programmcodes eingerichtet sein, um ein Verfahren in einer oder mehreren der oben beschriebenen Ausgestaltungen umzusetzen, um aus dem ersten Zeitabschnitt des zeitlichen Verlaufs der optischen Messgröße auf die mindestens eine Störgröße des Bluts zu schließen. Weiterhin kann die Datenverarbeitungsvorrichtung mittels eines Programmcodes, beispielsweise mittels desselben Programmcodes, programmtechnisch eingerichtet sein, um aus dem mindestens einen zweiten Zeitabschnitt des zeitlichen Verlaufs der optischen Messgröße auf die Konzentration des Analyten zu schließen. Die Datenverarbeitungsvorrichtung kann weiterhin mindestens einen flüchtigen und/oder nicht flüchtigen Datenspeicher umfassen. In diesem Datenspeicher können beispielsweise Analytkonzentrationen gespeichert werden und/oder es kann eine Datenbank in dem Datenspeicher abgelegt werden. Weiterhin können auch beispielsweise Vergleichsmuster und/oder Vergleichskurven zur Auswertung des zeitlichen Verlaufs der optischen Messgröße in dem ersten Zeitabschnitt und/oder in dem zweiten Zeitabschnitt in dem Datenspeicher hinterlegt sein, beispielsweise um ein oder mehrere der oben beschriebenen Mustervergleichsschritte oder Auswertungsschritte durchzuführen. Weiterhin können in dem Datenspeicher ein oder mehrere Korrekturen hinterlegt sein, beispielsweise Korrekturtabellen, um entsprechend der ermittelten Störgröße des Bluts optional eine Korrektur der Analytkonzentration durchzuführen, beispielsweise gemäß der obigen Beschreibung.

Die Nachweisvorrichtung und/oder die Auswertevorrichtung können insbesondere Bestandteile eines Testgeräts der Vorrichtung sein. So kann die Vorrichtung ein Testgerät umfassen, welches mit dem mindestens einen Testelement zusammenwirken kann und welches vorzugsweise die Nachweisvorrichtung und die Auswertevorrichtung umfasst. Um mit dem Testelement zusammen zu wirken, kann das Testgerät beispielsweise eine Testelement-Aufnahme und/oder eine Testelement-Ausgabe aufweisen, welche eingerichtet ist, um ein Testelement in eine Applikationsposition zu bringen, in welcher Blut auf das Testelement aufbringbar ist. Alternativ oder zusätzlich kann auch ein Aufbringen des Bluts in einem Zustand erfolgen, in welchem das Testelement von dem Testgerät getrennt ist. Auch Mischformen sind denkbar, beispielsweise eine Einrichtung, bei welcher zunächst das Testelement mit dem Testgerät verbunden wird, beispielsweise um das Testgerät zu initiieren und optional um eine oder mehrere Leerwertmessungen und/oder Kalibrationsmessungen durchzuführen, wobei anschließend das Testelement wieder von dem Testgerät getrennt wird, um die Blutprobe aufzubringen, und wobei anschließend das Testelement mit dem aufgebrachten Blut wieder mit dem Testgerät verbunden wird.

Das Testgerät kann als Einzel-Testelement-Testgerät ausgestaltet sein, wobei beispielsweise Testelemente einzeln nacheinander und beispielsweise händisch mit dem Testgerät verbunden werden können, beispielsweise indem Testelemente nacheinander in eine Teststreifenaufnahme des Testgeräts von außen eingeschoben werden. Alternativ kann das Testgerät auch als Mehr-Testelemente-Testgerät ausgestaltet sein, beispielsweise indem das Testgerät ein Testelement-Magazin aufweist, aus welchem nacheinander eine Mehrzahl von Testelementen, beispielsweise einzeln, bereitgestellt werden können. Entsprechend der möglichen Ausgestaltungen des Testelements als Teststreifen, Testband, Teststäbchen, Testblättchen, Testscheibe oder anderen Ausgestaltungen, wobei jeweils pro Testelement ein oder mehrere Testchemiefelder optional vorgesehen sein können, kann auch das Magazin ausgestaltet sein, beispielsweise als Teststreifenmagazin (beispielsweise als Stapelmagazin), als Bandkassette, als Scheibenmagazin oder auf andere Weise. Derartige Ausgestaltungen von Magazinen und Testelementen sowie Testgeräten sind dem Fachmann grundsätzlich bekannt. Dementsprechend lässt sich die vorgeschlagene Erfassung des zeitlichen Verlaufs der optischen Messgröße und die Auswertung des ersten Zeitabschnitts des zeitlichen Verlaufs zur Bestimmung der mindestens einen Störgröße und des zweiten Zeitabschnitts des zeitlichen Verlaufs zur Bestimmung der Analytkonzentration auch beispielsweise durch entsprechende programmtechnische Modifikation einer Auswertevorrichtung in bestehende Testgeräte integrieren.

Wie oben dargestellt, kann die Vorrichtung insbesondere eingerichtet sein, um eine Verfahren gemäß einer oder mehreren der erfindungsgemäß vorgeschlagenen Ausgestaltungen durchzuführen. Wie dargestellt, kann dies insbesondere durch eine programmtechnische Einrichtung mindestens einer Datenverarbeitungsvorrichtung der mindestens einen Auswertevorrichtung erfolgen.

Wie oben ebenfalls dargestellt, kann die Nachweisvorrichtung beispielsweise mindestens einen Detektor und/oder mindestens eine Lichtquelle umfassen. Beispielsweise kann die Nachweisvorrichtung mindestens eine Abfragelichtquelle zur Bestrahlung der Testchemie mit mindestens einem Abfragelichtstrahl umfassen. Unter einem Abfragelichtstrahl ist dabei allgemein ein Lichtstrahl zu verstehen, mittels dessen die Testchemie bestrahlt werden kann, und welcher geeignet ist, um eine wie auch immer geartete Antwort der Testchemie hervorzurufen. Diese Antwort kann beispielsweise in einer Reflektion des Abfragelichtstrahls und/oder in einer Streuung des Abfragelichtstrahls und/oder in einer vollständigen oder teilweisen Absorption des Abfragelichtstrahls, gefolgt von einer Re-Emission und/oder einer

Emission eines anderen Lichtstrahls, bestehen. Die Abfragelichtquelle kann insbesondere eine Halbleiterlichtquelle umfassen, beispielsweise mindestens eine Leuchtdiode, vorzugsweise eine Leuchtdiode zur Emission von Licht im ultravioletten und/oder sichtbaren und/oder infraroten Spektralbereich. Wie oben ausgeführt, kann eine Wellenlänge des Abfragelichtstrahls beispielsweise ausgewählt sein aus der Gruppe bestehend aus 635 nm, 660 nm, 770 nm, 815 nm und 880 nm. Grundsätzlich sind jedoch eine Vielzahl von Wellenlängen möglich.

Weiterhin umfasst die Nachweisvorrichtung vorzugsweise mindestens einen Detektor zur Erfassung mindestens eines von der Testchemie ausgehenden Antwortlichtstrahls. Unter einem Antwortlichtstrahl ist dabei allgemein ein Lichtstrahl zu verstehen, welcher von der Testchemie, beispielsweise einem die Testchemie umfassenden Testchemiefeld, ausgesandt wird, in Antwort auf den Abfragelichtstrahl. Dieses Aussenden kann dabei unmittelbar von der Testchemie ausgehen, beispielsweise indem ein oder mehrere Moleküle der Testchemie unmittelbar Photonen des Antwortlichtstrahls emittieren, beispielsweise im Rahmen einer Fluoreszenz und/oder Phosphoreszenz. Alternativ oder zusätzlich kann der Antwortlichtstrahl auch derart von der Testchemie ausgehen, dass dieser lediglich einen reflektierten oder gestreuten Abfragelichtstrahl umfasst. Auch diese soll vom Begriff "ausgehen" umfasst sein. Insbesondere kann der Antwortlichtstrahl somit an der Testchemie reflektiertes oder diffusiv gestreutes Licht des Abfragelichtstrahls umfassen.

Weitere mögliche Ausgestaltungen betreffen die Ausgestaltungen des Testelements. Wie oben beschrieben, kann das Testelement insbesondere ein Teststreifen sein. Dabei sind beispielsweise Vorrichtungen mit einzelnen Teststreifen einsetzbar oder auch Vorrichtungen mit mehreren Teststreifen. Die Vorrichtung kann insbesondere mindestens eine Teststreifenaufhahme aufweisen, also eine mechanische Vorrichtung, welche eingerichtet ist, um den Teststreifen zu haltern. Dabei ist mindestens ein Teststreifen in der Teststreifenaufhahme in eine Applikationsposition bringbar, wobei in der Applikationsposition mindestens eine Aufgabestelle des Teststreifens durch einen Benutzer mit Blut beaufschlagbar ist. Der mindestens eine Teststreifen kann dabei beispielsweise von außen, beispielsweise von außerhalb eines Testgeräts, in die Teststreifenaufnahme und in die Applikationsposition gebracht werden, beispielsweise händisch durch einen Benutzer, insbesondere durch Einschieben eines Teststreifens in einen Teststreifenschlitz der Teststreifenaufnahme. Alternativ oder zusätzlich kann ein Teststreifen auch beispielsweise aus einem Innenraum eines Testgeräts der Vorrichtung und/oder aus einem Magazin, welches beispielsweise Bestandteil des Testgeräts sein kann, in die Teststreifenaufhahme und in die Applikation ausgeschoben werden.

Vorzugsweise weist der Teststreifen mindestens ein Kapillarelement auf, um das Blut oder Bestandteile des Bluts von der Aufgabestelle zu der Testchemie zu transferieren. Beispielsweise kann das Kapillarelement einen Querschnitt von weniger als 1 mm aufweisen, insbesondere einen Querschnitt von 50 $\mu$m bis 1000 $\mu$m. Beispielsweise kann das Kapillarelement einen Kapillarkanal mit rechteckigem, polygonalem oder rundem Querschnitt aufweisen, beispielsweise mit einem Durchmesser oder Äquivalentdurchmesser von weniger als 1 mm, insbesondere weniger als 200 $\mu$m. Beispielsweise können Kapillaren mit einem rechteckigen Querschnitt eingesetzt werden, beispielsweise mit einer Kantenlänge von 1,5 mm x 75 $\mu$m oder mit kleineren Abmessungen, beispielsweise 0,5 mm x 50 $\mu$m.

Insbesondere kann zwischen dem Kapillarelement und der Testchemie mindestens ein Abtrennelement zur Abtrennung mindestens eines Bestandteils des Bluts aus dem Blut angeordnet sein. Bezüglich möglicher Ausgestaltungen des Abtrennelements kann insbesondere auf die obige Beschreibung verwiesen werden. Das Abtrennelement kann insbesondere mindestens eine Abtrennschicht umfassen. Beispielsweise kann der Kapillarkanal derart ausgestaltet sein, dass dieser an einer Kante und/oder an einer Fläche der Abtrennschicht mündet und Blut oder Blutbestandteile dorthin transferiert. Anschließend können von dort aus Blut oder Blutbestandteile die Abtrennschicht durchdringen. Nach dem Durchdringen der Abtrennschicht können das Blut oder die Blutbestandteile, ggf. nach Abtrennung eines oder mehrerer Bestandteile, direkt oder indirekt beispielsweise in eine Testchemieschicht gelangen, welche die Testchemie umfasst. So kann beispielsweise ein Schichtaufbau gewählt werden, bei welchem, ausgehend von der Kapillare, zunächst mindestens eine Abtrennschicht verwendet wird, gefolgt von mindestens einer Testchemieschicht. Das Abtrennelement und/oder die Abtrennschicht können beispielsweise frei von Nachweischemie sein, also an der oben beschriebenen Nachweisreaktion vorzugsweise nicht beteiligt sein. Bezüglich möglicher Ausgestaltungen des Abtrennelements und/oder der Abtrennschicht kann insbesondere auf den oben genannten Stand der Technik herkömmlicher Teststreifen verwiesen werden.

Der Teststreifen kann insbesondere derart eingerichtet sein, dass ein Abfragelichtstrahl in die Testchemie eingestrahlt werden kann. Zu diesem Zweck kann beispielsweise ein Testchemiefeld eine der Nachweisvorrichtung und insbesondere der Abfragelichtquelle zuweisende Oberfläche aufweisen. Der Teststreifen kann insbesondere derart eingerichtet sein, dass ein in die Testchemie eingestrahlter Abfragelichtstrahl zunächst die Testchemie passiert, beispielsweise mindestens eine Testchemieschicht, dann das Abtrennelement passiert, beispielsweise mindestens eine Abtrennschicht, anschließend an mindestens einer reflektierenden Oberfläche reflektiert wird, anschließend erneut das Abtrennelement passiert, dann wiederum die Testchemie passiert und schließlich wieder aus dem Teststreifen austritt, beispielsweise als Antwortlichtstrahl. Die Reflektion an der reflektierenden Oberfläche kann beispielsweise eine Reflektion an einer reflektierenden Oberfläche eines Trägerelements des Teststreifens sein. Dieses Trägerelement kann beispielsweise ganz oder teilweise hergestellt sein aus einem oder mehreren dem Materialien Kunststoff, Keramik, Papier. Auch Laminate sind grundsätzlich möglich. Zur Erhöhung einer Reflektivität der Oberfläche kann das Trägerelement

beispielsweise ein oder mehrere Pigmente enthalten, beispielsweise Titandioxid.

Durch den oben beschriebenen reflektiven Aufbau passiert das Licht auf seinem Weg durch das Testelement die Testchemie mindestens zweifach, da Einfach- oder auch Mehrfachreflektionen möglich sind. Dementsprechend wird das Licht mindestens zweimal durch die Testchemie beeinflusst, was die Sensitivität des optischen Nachweises erhöhen kann. Weiterhin passiert das Licht jedoch auch das Abtrennelement mehrfach, so dass auch beispielsweise abgetrennte Bestandteile des Bluts, welche sich an oder in dem Abtrennelement anlagern können, das Licht mehrfach beeinflussen können. Dementsprechend kann das Licht auch in verstärktem Maße beispielsweise durch eine sich in oder an dem Abtrennelement anlagernde Störkomponente beeinflusst werden, beispielsweise durch rote Blutkörperchen. Dies wiederum bewirkt, dass auch die Sensitivität des optischen Nachweises der Störkomponente während des ersten Zeitabschnitts erhöht werden kann.

Die reflektierende Oberfläche kann beispielsweise unmittelbar an dem Abtrennelement angeordnet sein. Alternativ kann die reflektierende Oberfläche auch beabstandet zu dem Abtrennelement angeordnet sein. So kann beispielsweise zwischen dem Abtrennelement, beispielsweise eine Abtrennschicht, und der reflektierende Oberfläche ein Abschnitt des Kapillarelements angeordnet sein, so dass beispielsweise das Licht zwischen dem Abtrennelement und der reflektierenden Oberfläche einen Teil eines Kapillarkanals des Kapillarelements passieren kann.

Weitere mögliche Ausgestaltungen betreffen das Testelement. So kann das Testelement, insbesondere der Teststreifen, vorzugsweise mindestens ein von der Außenseite des Testelements sichtbares Testchemiefeld der Testchemie aufweisen. Die optische Nachweisvorrichtung kann insbesondere eingerichtet sein, um die mindestens eine optische Eigenschaft der Testchemie an dem Testchemiefeld zu erfassen. So kann das Testchemiefeld die oben genannte Oberfläche bereitstellen, über welche Abfragelicht in die Testchemie eingestrahlt werden kann und über welche Antwortlicht aus der Testchemie ausgestrahlt werden kann.

Weitere mögliche Ausgestaltungen betreffen die Testchemie. Die Testchemie kann insbesondere derart stabil ausgewählt werden, dass diese zumindest kurzzeitig stabil gegenüber Feuchtigkeit und Temperaturbelastungen ist, insbesondere gegenüber Temperaturen von mindestens 60 °C, mindestens 80 °C oder sogar mindestens 100° C.

Allgemein kann bezüglich möglicher Testchemien beispielsweise auf den Stand der Technik verwiesen werden, beispielsweise auf WO 2007/118647, EP 0 354 441 B1, WO 2010/094426 A1, WO 2010/094427 A1, auf J. Hönes et al.: Diabetes Technology and Therapeutics, Vol. 10, Supplement 1, 2008, S-10 bis S-26 oder auf A. v. Ketteler et al.: Fluorescence Properties of Carba Nicotinamide Adenine Dinucleotide for Glucose Sensing, ChemPhysChem 2012, 13, 1302-1306.

Die Testchemie kann insbesondere mindestens ein Enzym und vorzugsweise mindestens ein Coenzym enthalten, welche vorzugsweise gemeinsam gelagert sind. Beispielsweise kann das Enzym Glukose-Dehydrogenase (GDH) und/oder eine Mutante dieses Enzyms aufweisen. Beispielsweise ist in WO2007/118647 eine Testchemie beschrieben, welche auf der Verwendung einer vom Coenzym PQQ abhängigen GDH-Mutante (EC 1.1.5.2) basiert. Die nachfolgend beschriebenen Messungen wurden vorwiegend mit dieser Testchemie durchgeführt. Weitere Beispiele einer PQQ-abhängigen Testchemie, welche ebenfalls im Rahmen der vorliegenden Erfindung einsetzbar ist, sind in EP 0 354 441 B1 beschrieben.

Besonders bevorzugt ist, wie oben ausgeführt, eine temperaturstabile Testchemie, also eine Testchemie, welche zumindest kurzzeitig stabil gegenüber Temperaturen von 100° C ist, insbesondere gegen Temperaturen von 110° C und besonders bevorzugt gegenüber Temperaturen von 120° C. Unter einer zumindest kurzzeitig stabil gegenüber den genannten Temperaturen ausgestalteten Testchemie wird eine Testchemie verstanden, die bei einer Beaufschlagungsdauer von mindestens 1 Minute, vorzugsweise von mindestens 5 Minuten, mit den genannten Temperaturen in ihrer Aktivität um vorzugsweise weniger als 50 %, insbesondere um weniger als 30 % und besonders bevorzugt um weniger als 20 % abnimmt. Beispielsweise kann die Testchemie, vorzugsweise als Trockenchemie auf dem Trägerelement, zum Test dieser Eigenschaften für die genannten Zeitdauern, beispielsweise für 1 Minute oder 5 Minuten, den genannten Temperaturen ausgesetzt werden. Vor oder nach dieser Temperaturbeaufschlagung wird die Aktivität erfasst. Die Aktivität kann dabei grundsätzlich mittels eines beliebigen, aus dem Stand der Technik bekannten Verfahrens bestimmt werden, da im Rahmen der vorliegenden Definition lediglich die prozentuale Abnahme der Aktivität während der Temperaturbelastung von Relevanz ist. Die Aktivität kann sich dabei insbesondere auf eine Enzymaktivität der Testchemie, insbesondere einer Trockenchemie, beziehen, insbesondere in einem Teststreifen. Beispielsweise sind Verfahren bekannt, die zur Messung der Enzymaktivität das Enzym aus der Testchemie bzw. dem Testelement extrahieren und anschließend beispielsweise mittels einer Ultraviolett-Absorption die Aktivität bestimmen. Diesbezüglich kann beispielsweise auf H. U. Bergmeyer: Methoden der enzymatischen Analyse, Verlag Chemie, 2. Auflage 1970, S. 417 oder auf Banauch et al.: A glucose dehydrogenase for the determination of glucose concentrations in body fluids, Z. Klin. Chem. Klin. Biochem. 1975 Mar; 13(3):101-7 verwiesen werden. Beispielsweise kann für den Test der Stabilität und/oder der Aktivitätsabnahme ein Testelement, beispielsweise ein Teststreifen, mit der Testchemie hergestellt werden. Anschließend kann mit einem üblichen Verfahren die Enzymaktivität eines Enzyms der Testchemie gemessen werden, dann die oben beschriebene Lagerung bei erhöhter Temperatur durchgeführt werden, und anschließend erneut dasselbe Verfahren zur Messung der Enzymaktivität durchgeführt werden. Die Prozedur wird üblicherweise mit einem repräsentativen Kollektiv an Testelementen bzw. Testchemien durchgeführt.

Als Beispiel für temperaturstabile Testchemien kann beispielsweise auf WO 2007/012494 A1 sowie auf die oben bereits zitierten WO 2010/094426 A1, WO 2010/094427 A1 und A. v. Ketteler et al.: Fluorescence Properties of Carba Nicotinamide Adenine Dinucleotide for Glucose Sensing, ChemPhysChem 2012, 13, 1302-1306 verwiesen werden. Die dort dargestellten Testchemien sind auch im Rahmen der vorliegenden Erfindung einsetzbar, allein oder auch in Kombination mit einer oder mehreren anderen Testchemien. Alternativ oder zusätzlich sind jedoch auch andere Testchemien einsetzbar.

Die Testchemie kann, wie oben ausgeführt, insbesondere mindestens ein Enzym und mindestens ein Coenzym, beispielsweise mindestens ein stabiles Coenzym, enthalten, welche gemeinsam gelagert sind. Mit Hilfe eines stabilen Coenzyms kann insbesondere eine Temperaturstabilisierung und/oder eine Langzeitstabilisierung von mehreren Wochen bzw. Monaten bei hoher relativer Feuchte oder sogar in flüssiger Phase und bei erhöhten Temperaturen realisiert werden. Diese Erkenntnis ist überraschend, da bekannt ist, dass Enzyme in Gegenwart von nativem Coenzym zwar eine erhöhte Kurzzeitstabilität für einige Stunden besitzen, aber eine geringere Haltbarkeit über einen längeren Zeitraum aufweisen. Gegenüber diesen Erkenntnissen gegenüber dem Stand der Technik war es überraschend, dass ein Enzym in Gegenwart eines stabilen Coenzyms eine deutlich erhöhte Temperatur- und Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Das Enzym, insbesondere das durch das Coenzym stabilisierte Enzym, kann insbesondere ein Coenzym-abhängiges Enzym sein. Geeignete Enzyme sind z.B. Dehydrogenasen, ausgewählt aus einer Glukose-Dehydrogenase (E.C.1.1.1.47 und/oder E.C.1.1.5.2), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glukoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase. Vorzugsweise ist das Enzym Glukose-Dehydrogenase.

Als besonders bevorzugt hat sich der Einsatz einer mutierten Glukose-Dehydrogenase erwiesen. Beispielsweise kann diesbezüglich auf die oben bereits erwähnte WO 2007/118647 verwiesen werden. Auch andere Mutanten sind jedoch grundsätzlich alternativ oder zusätzlich einsetzbar.

Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nichtortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf.

Die mutierte Glukose-Dehydrogenase kann die gegenüber der korrespondierenden Wildtyp-Glukose-Dehydrogenase veränderte(n) Aminosäure(n) grundsätzlich an einer beliebigen Position ihrer Aminosäuresequenz enthalten. Vorzugsweise umfasst die mutierte Glukose-Dehydrogenase eine Mutation an mindestens einer der Positionen 96, 170 und 252 der Aminosäuresequenz der Wildtyp-Glukose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glukose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z.B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glukose-Dehydrogenase kann durch Mutation einer aus einer beliebigen biologischen Quelle stammenden Wildtyp-Glukose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glukose-Dehydrogenase einem Bakterium, wobei es sich besonders bevorzugt um eine Glukose-Dehydrogenase aus Bacillus megaterium, Bacillus subtilis oder Bacillus thuringiensis, insbesondere aus Bacillus subtilis, handelt.

In einer besonders bevorzugten Ausführungsform der vorliegenden Erfindung handelt es sich bei der mutierten Glukose-Dehydrogenase um eine durch Mutation von Wildtyp-Glukose-Dehydrogenase aus Bacillus subtilis erhaltene Glukose-Dehydrogenase, welche die in SEQ ID NO: 1 (GlucDH_E96G_E170K) oder die in SEQ ID NO: 2 (GlucDH_E170K_K252L) dargestellte Aminosäuresequenz besitzt.

Das stabile Coenzym ist vorzugsweise ein gegenüber dem nativen Coenzym chemisch verändertes Coenzym, welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Bevorzugte Beispiele für stabile Coenzyme sind stabile Derivate von Nicotinamid-Adenin-Dinukleotid (NAD/NADH) oder Nicotinamid-Adenin-Dinukleotidphosphat (NADP/NADPH), oder verkürzte NAD-Derivate, z.B. ohne AMP-Teil bzw. mit nicht nukleosidischen Resten, z.B. hydrophoben Resten. Ebenfalls bevorzugt ist als stabiles Coenzym im Sinne der vorliegenden Erfindung die Verbindung der Formel (I).

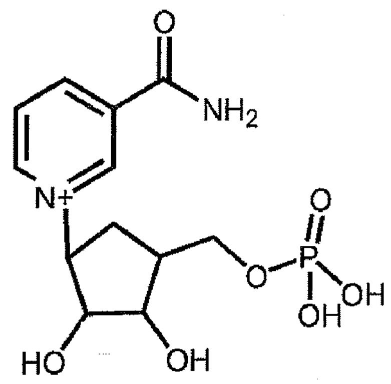

(I).

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (II) ausgewählt:

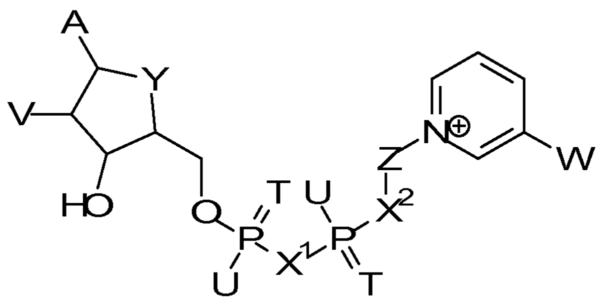

(II)

mit

A = Adenin oder ein Analog davon,
T = jeweils unabhängig O, S,
U = jeweils unabhängig OH, SH, $BH_3^-$, $BCNH_2^-$,
V = jeweils unabhängig OH oder eine Phosphatgruppe, oder zwei Gruppen, die eine cyclische Phosphatgruppe bilden;
W = COOR, $CON(R)_2$, COR, $CSN(R)_2$ mit R = jeweils unabhängig H oder $C_1$-$C_2$-Alkyl,
$X^1$, $X^2$ = jeweils unabhängig O, $CH_2$, $CHCH_3$, $C(CH_3)_2$, NH, $NCH_3$,

Y = NH, S, O, $CH_2$,
Z = ein linearer oder cyclischer organischer Rest ist,

mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest $CR^4_2$, wobei $CR^4_2$ an die cyclische Gruppe und an $X^2$ gebunden ist, mit $R^4$ = jeweils unabhängig H, F, Cl, $CH_3$.

Besonders bevorzugt ist Z ein gesättigter oder ungesättigter carbocyclischer oder heterocyclischer Fünfring, insbesondere eine Verbindung der allgemeinen Formel (III)

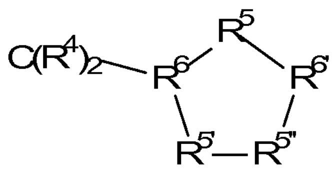

(III)

wobei zwischen $R^{5'}$ und $R^{5''}$ eine Einfach- oder Doppelbindung vorliegen kann, mit

$R^4$ = jeweils unabhängig H, F, Cl, $CH_3$,
$R^5$ =$CR^4_2$,
wobei $R^{5'}$ = O, S, NH, $NC_1$-$C_2$-Alkyl, $CR^4_2$, CHOH, $CHOCH_3$, und
$R^{5''}$ = $CR^4_2$, CHOH, $CHOCH_3$, wenn zwischen $R^{5'}$ und $R^{5''}$ eine Einfachbindung vorliegt, und
wobei $R^{5'}$=$R^{5''}$=$CR^4$, wenn zwischen $R^{5'}$ und $R^{5''}$ eine Doppelbindung vorliegt, und
$R^6$, $R^{6'}$ = jeweils unabhängig CH oder $CCH_3$.

In einer bevorzugten Ausführungsform enthalten die erfindungsgemäßen Verbindungen Adenin oder Adenin-Analoga, wie z.B. $C_8$- und $N_6$-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, $C_1$-$C_6$-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe ersetzt sein, beispielsweise isotronisch und/oder isovalent und/oder isoelektronisch ersetzt sein, wie z.B. $O^-$ durch $S^-$ bzw. $BH_3^-$, O durch NH, $NCH_3$ bzw. $CH_2$ und =O durch =S.
In den erfindungsgemäßen Verbindungen der Formel (II) ist W vorzugsweise $CONH_2$ oder $COCH_3$.

In den Gruppen der Formel (III) ist $R^5$ vorzugsweise $CH_2$. Weiterhin ist bevorzugt, dass $R^{5'}$ ausgewählt ist aus $CH_2$, CHOH und NH. In einer besonders bevorzugten Ausführungsform sind $R^{5'}$ und $R^{5''}$ jeweils CHOH. In noch einer weiteren bevorzugten Ausfuhrungsform ist $R^{5'}$ NH und $R^{5''}$ $CH_2$.

In der am stärksten bevorzugten Ausführungsform handelt es sich bei dem Coenzym, insbesondere dem stabilen Coenzym, um cNAD oder auch "carbaNAD", insbesondere nach einer oder mehreren der oben genannten Formeln (I), (II) oder (III). Alternativ oder zusätzlich kann das Coenzym auch ein oder mehrere Coenzyme umfassen, ausgewählt aus der Gruppe bestehend aus: NAD, cNAD, PQQ und FAD.

Die bevorzugte Testchemie ist insbesondere derart ausgestaltet, dass die in diesen enthaltenen Enzymen Langzeit-stabilisiert sind. Dies bedeutet, dass man das mit einem stabilen Coenzym stabilisierte Enzym, z.B. als Trockensubstanz, beispielsweise über eine Dauer von mindestens zwei Wochen, vorzugsweise von mindestens vier Wochen und besonders bevorzugt von mindestens acht Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.

Durch die Stabilisierung ist es möglich, das mit einem stabilen Coenzym stabilisierte Enzym auch ohne Trocknungsreagenz für eine lange Zeit, wie oben angegeben, und/oder bei hohen Temperaturen, wie oben angegeben, zu

lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in dem Testelement, das zur Bestimmung eines Analyten geeignet ist. Das mit einem stabilen Coenzym stabilisierte Enzym ist dabei Bestandteil der bevorzugten Testchemie, welche gegebenenfalls noch weitere Bestandteile wie etwa Salze, Puffer, etc. enthalten kann. Vorzugsweise ist die Testchemie dabei frei von einem Mediator.

Das mit einem stabilen Coenzym stabilisierte Enzym kann allgemein zum Nachweis von Analyten, beispielsweise Parametern in Körperflüssigkeiten wie etwa Blut, Serum, Plasma oder Urin bzw. in Abwasserproben oder Lebensmitteln eingesetzt werden.

Als Analyten können beliebige biologische oder chemische Substanzen bestimmt werden, die durch eine Redoxreaktion nachgewiesen werden können, z.B. Substanzen, bei denen es sich um Substrate eines Coenzym-abhängigen Enzyms handelt oder Coenzym-abhängige Enzyme selbst. Bevorzugte Beispiele für Analyten sind Glukose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glukose. Besonders bevorzugt ist hierbei der Nachweis von Glukose mit Hilfe von Glukose-Dehydrogenase (GlucDH).

Die Veränderung der Testchemie, beispielsweise des stabilen Coenzyms, durch Reaktion mit dem Analyten kann grundsätzlich auf beliebige Art und Weise nachgewiesen werden, unter Verwendung der mindestens einen optischen Messgröße. Hier können grundsätzlich alle aus dem Stand der Technik bekannten Methoden eingesetzt werden, beispielsweise Methoden zum Nachweis enzymatischer Reaktionen. Vorzugsweise wird jedoch die Veränderung des Coenzyms durch optische Methoden nachgewiesen. Optische Nachweismethoden umfassen beispielsweise die Messung von Reflexion und/oder Remission, Absorption, Fluoreszenz, Circulardichroismus (CD), optische Rotationsdispersion (ORD), Refraktometrie etc.

Ein optisches Nachweisverfahren, welches im Rahmen der vorliegenden Anmeldung bevorzugt Anwendung findet, ist die Photometrie. Zur photometrischen Messung einer Veränderung des Coenzyms infolge Umsetzung mit dem Analyten kann insbesondere in der Testchemie optional mindestens ein Mediator verwendet werden, welcher die Reaktivität des reduzierten Coenzyms erhöhen kann und welcher eine Übertragung von Elektronen auf einen geeigneten optischen Indikator und/oder ein geeignetes optisches Indikatorsystem ermöglichen oder unterstützen kann. Alternativ kann jedoch auch ein direkter Nachweis, ohne Anwesenheit eines Mediators, erfolgen.

Als Mediatoren, welche optional für die Zwecke der vorliegenden Erfindung geeignet sind und in der Testchemie enthalten sein können, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage. Bevorzugte Beispiele für Phenanthrolinchinone umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind.

Als optischer Indikator oder als optisches Indikatorsystem, welches in der Testchemie enthalten sein kann, kann insbesondere eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

Eine besonders bevorzugte Testchemie umfasst die Verwendung des Enzyms Glukose-Dehydrogenase mit NAD und/oder mit einem stabilen NAD-Derivat, beispielsweise cNAD, zum Nachweis von Glukose, wobei vorzugsweise ein Derivat des reduzierten Coenzyms NADH gebildet wird. Alternativ oder zusätzlich können auch ein oder mehrere der Coenzyme NAD, PQQ und FAD verwendet werden. Auch andere Coenzyme sind grundsätzlich einsetzbar. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Insbesondere kann die Testchemie derart ausgestaltet sein, dass diese ein mit einem stabilen Coenzym stabilisiertes Enzym umfasst, wobei das stabilisierte Enzym bei einer Lagerung von vorzugsweise mindestens zwei Wochen, besonders bevorzugt mindestens vier Wochen und am meisten bevorzugt mindestens acht Wochen bei einer Temperatur

von vorzugsweise mindestens 20° C, besonders bevorzugt mindestens 25° C und am meisten bevorzugt mindestens 30° C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Das Testelement kann insbesondere mindestens ein Trägerelement aufweisen, wobei die Testchemie vorzugsweise mit dem Trägerelement verbunden ist. Dieses Verbinden kann beispielsweise dadurch erfolgen, dass die Testchemie direkt oder indirekt in Form mindestens einer Testchemieschicht auf das Trägerelement aufgebracht ist. Beispielsweise kann das Aufbringen durch ein Verfahren erfolgen, ausgewählt aus der Gruppe bestehend aus Rakeln, Drucken (insbesondere Siebdruck, Schablonendruck oder Tampondruck) und Aufschleudern.

Das Trägerelement kann insbesondere ganz oder teilweise aus mindestens einem Kunststoffinaterial hergestellt sein. Insbesondere kann es sich bei diesem Kunststoffmaterial um ein Kunststoffinaterial mit einer Erweichungstemperatur, bestimmbar nach DIN EN ISO 306, von mindestens 100° C, vorzugsweise von mindestens 110° C und besonders bevorzugt von mindestens 120° C handeln, beispielsweise mit einer Erweichungstemperatur von mindestens 130° C oder sogar mindestens 140° C oder mindestens 150° C. Beispiele derartiger Kunststoffe sind Acrylnitril-Butadien-Styrol (ABS), Polymethylmethacrylat (PMMA), Polypropylen (PP), Polyester und Polycarbonat (PC) oder Kombinationen der genannten und/oder anderer Kunststoffe. Auch andere Kunststoffe sind jedoch grundsätzlich einsetzbar.

Das Trägerelement kann insbesondere mindestens ein Folienelement umfassen. Insbesondere kann es sich hierbei um eine Kunststofffolie handeln. Diese Folie kann einschichtig oder auch mehrschichtig aufgebaut sein. Das Trägerelement kann eine oder mehrere Testchemien tragen. Beispielsweise kann die Testchemie mindestens eine für eine Probenaufgabe zugängliche Testfeldoberfläche bilden.

Weitere mögliche Ausgestaltungen der Vorrichtung betreffen die Nachweisvorrichtung. Wie oben ausgeführt, ist es besonders bevorzugt, wenn die Nachweisvorrichtung eingerichtet ist, um die mindestens eine Störgröße und die Konzentration des mindestens einen Analyten bei derselben Wellenlänge zu erfassen. Dementsprechend kann die optische Nachweisvorrichtung insbesondere eingerichtet sein, um die optische Messgröße in dem ersten Zeitabschnitt und dem zweiten Zeitabschnitt bei derselben mindestens einen Wellenlänge zu erfassen. Dementsprechend können beispielsweise zur Erfassung der optischen Messgröße während des ersten Zeitabschnitts und des zweiten Zeitabschnitts dieselbe Lichtquelle und/oder derselbe Detektor, beispielsweise dieselbe Abfragelichtquelle und derselbe Detektor verwendet werden.

Wie oben ausgeführt, kann die optische Nachweisvorrichtung insbesondere eine oder mehrere Halbleiterlichtquellen, insbesondere eine oder mehrere Leuchtdioden, aufweisen. Insbesondere kann die Nachweisvorrichtung mindestens eine erste Leuchtdiode und mindestens eine zweite Leuchtdiode sowie mindestens eine Fotodiode zur Erfassung von an der Testchemie diffusiv reflektiertem Licht der Leuchtdioden aufweisen. Sind mehrere Leuchtdioden vorgesehen, so können diese gleichzeitig oder auch zeitversetzt oder zeitlich überlappend emittieren. Insbesondere kann die Vorrichtung eingerichtet sein, um abwechselnd reflektiertes Licht der Leuchtdioden mit demselben Detektor, insbesondere derselben Fotodiode, zu erfassen.

Sind mehrere Leuchtdioden vorgesehen, so kann die Auswertevorrichtung insbesondere eingerichtet sein, um reflektiertes Licht der zweiten Leuchtdiode zur Erkennung einer Benetzung der Testchemie durch das Blut oder Blutbestandteile zu verwenden. Diese Erkennung der Benetzung kann beispielsweise durch eine Überwachung eines Remissionswerts erfolgen. So kann beispielsweise eine Remission des von der zweiten Leuchtdiode emittierten Lichts an einem Testchemiefeld mit einem oder mehreren Schwellwerten verglichen werden, wobei eine starke Änderung der Remission auf einen Start einer Benetzung und einen Start eines Benetzungssprungs schließen lässt. Die Auswertevorrichtung kann weiterhin eingerichtet sein, um reflektiertes Licht der ersten Leuchtdiode zur Erfassung der optischen Messgröße, insbesondere einer Remission, während des ersten Zeitabschnitts und des zweiten Zeitabschnitts zu verwenden, beispielsweise gemäß dem oben beschriebenen Algorithmus, und daraus auf die mindestens eine Störgröße des Bluts wie die Konzentration des mindestens einen Analyten zu schließen. Dementsprechend kann die erste Leuchtdiode beispielsweise eingesetzt werden, um das Verfahren gemäß einer oder mehrerer der oben beschriebenen Ausgestaltungen durchzuführen. Die zweite Leuchtdiode kann beispielsweise eingesetzt werden, um einen Startpunkt eines Remissionssprungs zu ermitteln und diesen Startpunkt beispielsweise als Startzeitpunkt des ersten Zeitabschnitts zu wählen. Auch andere Ausgestaltungen sind jedoch möglich.

In einem weiteren Aspekt der vorliegenden Erfindung wird eine Verwendung eines Benetzungssprungs im zeitlichen Verlauf einer an einem Testelement, insbesondere einem Testchemiefeld eines Testelements, erfassten optischen Messgröße, insbesondere einer Remission, zur Bestimmung einer Störgröße in Blut vorgeschlagen, insbesondere zur Bestimmung eins Anteils einer Störkomponente in dem Blut und besonders bevorzugt zur Bestimmung eines Hämatokrits. Die Verwendung kann insbesondere derart ausgestaltet sein, dass die derart bestimmte Störgröße weiterhin zur Korrektur einer Bestimmung einer Konzentration mindestens eines Analyten in dem Blut verwendet wird, insbesondere zur Korrektur einer Bestimmung einer Blutglukosekonzentration. Zur Bestimmung der Störgröße und zur Bestimmung der Konzentration des Analyten kann insbesondere dieselbe optische Messgröße verwendet werden, insbesondere eine optische Remission bei derselben Wellenlänge. Für weitere mögliche Ausgestaltungen der Verwendung kann auf die obige Beschreibung des Verfahrens und auf die obige Beschreibung der Vorrichtung sowie die nachfolgend noch näher beschriebenen Ausführungsbeispiele verwiesen werden.

Das vorgeschlagene Verfahren, die vorgeschlagene Vorrichtung und die Verwendung weisen gegenüber bekannten Verfahren, Vorrichtungen und Verwendungen zahlreiche Vorteile auf. Insbesondere lässt sich eine effiziente Korrektur von Störeinflüssen, insbesondere eine effiziente Hämatokritkorrektur, bei einer optischen Blutzuckermessung realisieren. Im Gegensatz zu herkömmlichen Verfahren, bei welchen üblicherweise an eine Abtrennung von Erythrozyten vor einem Test hohe Anforderungen zu stellen sind, lässt sich der apparative Aufwand zur Umsetzung des vorgeschlagenen Verfahrens auf Seiten des Testelements und auf Seiten eines möglichen Testgeräts äußerst gering halten. Das vorgeschlagene Verfahren lässt sich insbesondere im Wesentlichen durch eine entsprechende Software auch in bekannten Geräten implementieren.

Im Gegensatz zu EP 2 325 624 A1, welche, wie sich beispielsweise aus Absatz [0030] ausdrücklich ergibt, das Remissionsverhalten während der Benetzung als konstant ansieht, kann erfindungsgemäß durch die Unterteilung des zeitlichen Verlaufs der optischen Messgröße der Benetzungssprung gerade zur Bestimmung der Störkomponente, insbesondere des Hematokrits, herangezogen werden. Die EP 2 325 624 A1 setzt sich lediglich zum Ziel, eine Änderung des Übertragungsverhaltens eines optischen Übertragungssystems durch Auftrag der Probe zu korrigieren. So wird in EP 2 325 624 A1 zwar eine Zeitachse in mehrere Zeitabschnitte eingeteilt. Im den Zeitabschnitt zwischen den Zeitpunkten t1 und t2 werden jedoch keine Messwerte erfasst, wie sich beispielsweise aus Spalte 6, Zeilen 2-6 ausdrücklich ergibt. In diesem Zeitabschnitt werden lediglich die nachfolgenden Messkurven extrapoliert. Insofern offenbart die EP 2 325 624 A1 ausdrücklich nicht das erfindungsgemäße Merkmal, dass ein zeitlicher Verlauf mindestens einer optischen Messgröße der Testchemie in einem ersten Zeitabschnitt erfasst wird und aus diesem zeitlichen Verlauf in dem ersten Zeitabschnitt auf mindestens eine Störgröße des Bluts geschlossen wird. So wird in der EP 2 325 624 A1 in dem Zeitabschnitt zwischen t1 und t2 keine Messkurve erfasst. Lediglich die Extrapolationskurven sind in diesem Zeitabschnitt gültig. Weiterhin wird mittels dieser Extrapolationskurven in EP 2 325 624 A1 nicht auf eine Störgröße des Bluts geschlossen, sondern es wird auf einen Startzeitpunkt und eine Veränderung des Übertragungsverhaltens des optischen Übertragungssystems geschlossen. Wie oben ausgeführt, kann im Rahmen der vorliegenden Erfindung unter einer Störgröße des Bluts insbesondere eine beliebige Einflussgröße, welche eine Eigenschaft des Bluts ist, mit Ausnahme der Konzentration des nachzuweisenden Analyten selbst, verstanden werden, wobei die Eigenschaft des Bluts die Bestimmung der Konzentration des Analyten beeinflussen kann. Durch das erfindungsgemäß vorgeschlagene Verfahren können also, durch Unterteilung der eigentlichen Messkurve in mindestens zwei Zeitabschnitte, Störgrößen des Blutes selbst ermittelt und/oder deren Einfluss auf die Bestimmung der Analytkonzentration verringert, kompensiert oder korrigiert werden.

Im Gegensatz beispielsweise zur US 2010/0159570 A1 oder zu JP 2007/303968 A wird im Rahmen des vorgeschlagenen Verfahrens ein zeitlicher Verlauf einer optischen Messgröße der Testchemie selbst erfasst. Dabei kann die optische Messgröße beispielsweise über das gesamte Testchemiefeld und/oder über einen Messbereich auf dem Testchemiefeld hinweg homogen und/oder räumlich gemittelt erfasst werden, vorzugsweise ohne Ortsauflösung. Beispielsweise kann eine einzige optische Messgröße an einem Ort eines die Testchemie umfassenden Testchemiefeldes oder räumlich gemittelt über einen oder mehrere Bereiche des Testchemiefeldes zeitaufgelöst erfasst werden und aus dem zeitlichen Verlauf dieser optischen Messgröße in dem ersten Zeitabschnitt auf die Störgröße geschlossen werden und in dem zweiten Zeitabschnitt auf die Konzentration des Analyten. In anderen Worten kann das vorgeschlagene Verfahren insbesondere ohne eine ortsaufgelöste Messung der mindestens einen optischen Messgröße durchgeführt werden und/oder lediglich mit einer räumlich gemittelten Erfassung der mindestens einen optischen Messgröße. Insbesondere kann das erfindungsgemäße Verfahren ohne Laufzeitmessung und/oder ohne Füllzeitmessung des Testelements durchgeführt werden, insbesondere ohne zeitaufgelöste Messung an unterschiedlichen Orten zur Bestimmung einer Laufzeit oder Füllzeit. Derartige einfache optische Messungen mindestens einer optischen Messgröße der Testchemie, welche ohnehin bei optischen Testelementen in der Regel vorgesehen sind, lassen sich leicht und ohne größere apparative Veränderungen entsprechender Testgeräte umsetzen, im Gegensatz beispielsweise zu der in US 2010/0159570 A1 oder JP 2007/303968 A beschriebenen Füllzeitmessung. Eine Ortsauflösung mit hoher Präzision, welche für Füllzeitmessungen erforderlich wäre und welche apparativ aufwändig umzusetzen wäre, kann entfallen. Dementsprechend können ein oder mehrere vergleichsweise einfach gestaltete Detektoren verwendet werden, beispielsweise lediglich mit einer einzelnen Lichtquelle, beispielsweise einer einzelnen Leuchtdiode. Füllzeitmessungen sind zudem apparativ stets mit der Problematik verbunden, dass ein Aufgabezeitpunkt der Probe in der Regel unbekannt ist und dass dem-entsprechend beispielsweise Differenzmessungen auf festgelegten Laufstrecken durchgeführt werden müssen.

Bei der erfindungsgemäßen Erfassung des zeitlichen Verlaufs der mindestens einen optischen Messgröße hingegen wird zudem der Einfluss des Hämatokrits direkt an der Testchemie erfasst. Hierbei werden in der Regel eine Vielzahl physikalisch-chemischer Vorgänge gleichzeitig erfasst, wie beispielsweise diffusive Vorgänge, Solvatation, Brechungsindexänderungen und andere Vorgänge, welche durch den Hämatokrit beeinflusst werden. Die Erfassung erfolgt unmittelbar dort, wo die Einflussnahme des Hämatokrit bestimmt werden soll, nämlich an der Testchemie, im

Gegensatz zu indirekten Messungen wie beispielsweise den genannten Füllzeitmessungen. Zudem werden in der Regel unmittelbar diejenigen physikalisch-chemischen Reaktionen und Vorgänge erfasst, welche aufgrund der Störgröße, insbesondere der Störkomponente, den Nachweis des Analyten in der Testchemie beeinflussen können.

Im Gegensatz beispielsweise zu US 4,935,346 lässt sich mittels des vorgeschlagenen Verfahrens, insbesondere durch die vorgeschlagene Unterteilung eines zeitlichen Verlaufs der optischen Messgröße in mindestens einen ersten Zeitabschnitt und mindestens einen zweiten Zeitabschnitt, eine wirksame Trennung der Bestimmung der Störgröße und der Bestimmung der Analytkonzentration herbeiführen. Überraschenderweise wurde gefunden, dass durch diese zeitliche Trennung eine gute Erfassung der Störgröße ohne Beeinflussung durch den Analytgehalt auch bei ein und derselben Wellenlänge möglich ist. Im Gegensatz dazu sind in US 4,935,346 sowohl die optischen Messungen bei 700 nm als auch bei 635 nm - wenn auch in unterschiedlichem Maße - beeinflusst sowohl durch den Hämatokrit als auch durch den Analytgehalt. Eine Trennung dieser beiden Effekte ist in der Praxis nur schwer möglich. Durch die erfindungsgemäß vorgeschlagene zeitliche Trennung wird diese Problematik auf einfache und elegante Weise überwunden.

Insbesondere lässt sich auf einfache Weise eine Messung des zeitlichen Verlaufs der mindestens einen optischen Messgröße bei lediglich einer Wellenlänge durchführen und hierdurch beispielsweise der sowohl Glukosegehalt als auch der Hämatokrit einer einzelnen Probe bestimmen. Aufgrund eines bekannten oder zumindest bestimmbaren Einflusses des Hämatokrits auf die Analytmessung, beispielsweise die Glukosemessung, kann eine effizientere Korrektur beispielsweise eine rechnerische Korrektur, des experimentell begründeten Analytgehalts vorgenommen werden, entweder bereits bei der Auswertung oder nachträglich in Form einer nachträglichen Korrektur. Die Möglichkeit einer Erfassung des optischen Messwerts bei lediglich einer Wellenlänge bedingt einen deutlichen apparativen Vorteil, da eine Implementierung mehrerer Lichtquellen vermieden werden kann. Gleichwohl können, wie oben ausgeführt, mehrere Lichtquellen nach wie vor noch vorgesehen sein, beispielsweise zum Erkennen eines Starts eines Benetzungssprungs und damit zur Festlegung eines definierten Zeitpunktes, beispielsweise eines Startzeitpunkts des ersten Zeitintervalls.

Überraschenderweise wurde festgestellt, was unten durch Experimente noch näher belegt wird, dass ein Hämatokrit beispielsweise alleine anhand eines Benetzungssprungs zuverlässig bestimmt werden kann, beispielsweise bei einer Wellenlänge von 880 nm. Da beispielsweise die Bestimmung einer Glukosekonzentration weitgehend flexibel ist hinsichtlich der Wahl der entsprechenden Wellenlänge, können für die Erfassung der optischen Messgröße während des ersten Zeitabschnitts und des zweiten Zeitabschnitts dieselben Wellenlängen verwendet werden. Es ist somit lediglich noch die Auswertung eines einzigen zeitlichen Verlaufs einer einzigen optischen Messgröße erforderlich, was auch den Auswertungsaufwand und somit die Rechenleistung und den Ressourcenbedarf einer Auswertevorrichtung erheblich verringern kann.

Weiterhin lässt sich durch das erfindungsgemäße Verfahren, die erfindungsgemäße Vorrichtung und die erfindungsgemäße Verwendung beispielsweise eine Hämatokritabhängigkeit fotometrischer Systeme zum Analytnachweis, beispielsweise fotometrischer Systeme zum Blutglukosenachweis, deutlich reduzieren. Hierdurch lassen sich zulässige Hämatokritbereiche, in denen die Vorrichtung und das Verfahren verwendet werden dürfen, deutlich erhöhen, ohne dass der apparative Aufwand signifikant gesteigert würde. Hierdurch lässt sich ein Anwendungsbereich bekannter Vorrichtungen durch Implementierung der erfindungsgemäßen Ausgestaltungen und Verfahren deutlich erweitern. Diese Erweiterung kann auf einfache Weise realisiert werden, beispielsweise durch Implementierung einer neuartigen Auswertungssoftware, mittels derer das erfindungsgemäße Verfahren umgesetzt wird.

Der zeitliche Verlauf der mindestens einen optischen Messgröße, beispielsweise der Remission oder der relativen Remission (eine Remission angegeben im Verhältnis zu einer Remission eines Remissions-Leerwerts) wird häufig auch als "Kinetik" bezeichnet, da dieser zeitliche Verlauf, zumindest im zweiten Zeitabschnitt, den Verlauf der Nachweisreaktion zum Nachweis des Analyten kennzeichnet. Überraschenderweise wurde, wie oben ausgeführt, herausgefunden, dass sich insbesondere aus einem Benetzungssprung, beispielsweise einem anfänglichen starken Abfall einer Remission, bedingt durch die Benetzung der Testchemie mit Blut oder Blutbestandteilen, auf einfach und zuverlässige Weise auf die Störgröße, insbesondere die Konzentration der Störkomponente und besonders bevorzugt des Hämatokrits, schließen lässt. Die Auswertung und Analyse der Kinetikdaten, welche exemplarisch nachfolgend noch näher beschrieben wird, zeigt einen zuverlässigen Zusammenhang beispielsweise zwischen einem Hämatokrit und diesem Benetzungssprung. Diese Abhängigkeit wurde an verschiedenen Proben mit hoher Reproduzierbarkeit festgestellt. Auf dieser Basis kann insbesondere eine Bestimmung des Hämatokrits der verwendeten Probe möglich sein, um, beispielsweise nachfolgend oder bereits bei der Auswertung, eine Korrektur des Messwerts der Analytkonzentration, beispielsweise der Glukosekonzentration, zu berücksichtigen. Insbesondere kann auf diese Weise, beispielsweise anhand eines Benetzungssprungs in einer Remission, ein Hämatokrit mit einer Genauigkeit von plus minus 5 % bis 10 %, insbesondere genauer als 5 % bestimmt werden. Bei einer bekannten Abhängigkeit des Messwerts der Analykonzentration vom Hämatokrit kann hieraus eine rechnerische Korrektur oder eine Korrektur mittels einer entsprechenden Korrekturfunktion und/oder mittels Korrekturfaktoren und/oder mittels Korrektur-Offsets, auf einfache und zuverlässige Weise erfolgen.

Zusammenfassend sind im Rahmen der vorliegenden Erfindung folgende Ausführungsformen besonders bevorzugt:

Ausführungsform 1: Verfahren zur Bestimmung mindestens einer Konzentration mindestens eines Analyten in Blut, insbesondere zur Bestimmung einer Blutglukosekonzentration, wobei ein Testelement verwendet wird, wobei das Testelement mindestens eine Testchemie aufweist, wobei die Testchemie eingerichtet ist, um bei Anwesenheit des Analyten mindestens eine optisch nachweisbare Nachweisreaktion durchzuführen, wobei das Blut auf das Testelement aufgebracht wird, wobei ein zeitlicher Verlauf mindestens einer optischen Messgröße der Testchemie erfasst wird, wobei aus mindestens einem ersten Zeitabschnitt des zeitlichen Verlaufs der optischen Messgröße auf mindestens eine Störgröße des Bluts, insbesondere eine Konzentration einer Störkomponente und vorzugsweise einen Hämatokrit des Bluts, geschlossen wird und wobei aus mindestens einem zweiten Zeitabschnitt des zeitlichen Verlaufs auf die Konzentration des Analyten geschlossen wird.

Ausführungsform 2: Verfahren nach der vorhergehenden Ausführungsform, wobei der erste Zeitabschnitt ein anfänglicher Zeitabschnitt des zeitlichen Verlaufs ist, wobei der zweite Zeitabschnitt dem ersten Zeitabschnitt zeitlich nachgeordnet ist.

Ausführungsform 3: Verfahren nach einer der vorhergehenden Ausführungsformen, wobei der zeitliche Verlauf der optischen Messgröße in dem ersten Zeitabschnitt einen Benetzungssprung der optischen Messgröße umfasst.

Ausführungsform 4: Verfahren nach einer der vorhergehenden Ausführungsformen, wobei die optische Messgröße bei mindestens einer ersten Wellenlänge erfasst wird, wobei aus einer starken Änderung der optischen Messgröße nach dem Aufbringen des Bluts auf das Testelement auf einen Benetzungszeitpunkt geschlossen wird, zu welchem das Blut die Testchemie erreicht und benetzt.

Ausführungsform 5: Verfahren nach der vorhergehenden Ausführungsform, wobei der erste Zeitabschnitt und der zweite Zeitabschnitt zumindest teilweise dem Benetzungszeitpunkt zeitlich nachgeordnet sind.

Ausführungsform 6: Verfahren nach einer der beiden vorhergehenden Ausführungsformen, wobei die optische Messgröße in dem ersten Zeitabschnitt und in dem zweiten Zeitabschnitt bei mindestens einer zweiten Wellenlänge erfasst wird, wobei die zweite Wellenlänge gleich der ersten Wellenlänge ist.

Ausführungsform 7: Verfahren nach einer der vorhergehenden Ausführungsformen, wobei die optische Messgröße mindestens einen Remissionswert der Testchemie umfasst.

Ausführungsform 8: Verfahren nach einer der vorhergehenden Ausführungsformen, wobei zur Erfassung der optischen Messgröße in dem ersten Zeitabschnitt und in dem zweiten Zeitabschnitt jeweils mittels mindestens einer Abfragelichtquelle mindestens ein Abfragelichtstrahl auf die Testchemie eingestrahlt werden und wobei jeweils mittels mindestens eines Detektors mindestens ein von der Testchemie ausgehender Antwortlichtstrahl erfasst werden.

Ausführungsform 9: Verfahren nach der vorhergehenden Ausführungsform, wobei der Abfragelichtstrahl in dem ersten Zeitabschnitt und in dem zweiten Zeitabschnitt dieselbe Wellenlänge und/oder dieselben spektralen Eigenschaften aufweist.

Ausführungsform 10: Verfahren nach einer der beiden vorhergehenden Ausführungsformen, wobei der Antwortlichtstrahl in dem ersten Zeitabschnitt und dem zweiten Zeitabschnitt dieselbe Wellenlänge und/oder dieselben spektralen Eigenschaften aufweist.

Ausführungsform 11: Verfahren nach einer der drei vorhergehenden Ausführungsformen, wobei der Antwortlichtstrahl durch Reflexion und/oder Streuung des Abfragelichtstrahls an der Testchemie gebildet wird.

Ausführungsform 12: Verfahren nach einer der vier vorhergehenden Ausführungsformen, wobei der Abfragelichtstrahl in dem ersten Zeitabschnitt und/oder dem zweiten Zeitabschnitt eine Wellenlänge im Bereich von 300 bis 1100 nm aufweist, insbesondere eine Wellenlänge ausgewählt aus der Gruppe bestehend aus: 365 nm, 375 nm, 440 nm, 525 nm, 550 nm, 580 nm, 635 nm, 660 nm, 740 nm, 770 nm, 815 nm und 880 nm.

Ausführungsform 13: Verfahren nach einer der vorhergehenden Ausführungsformen, wobei mittels der Steuergröße, insbesondere des Hämatokrits, mindestens eine Korrektur ermittelt wird, wobei aus dem zweiten Zeitabschnitt unter

Berücksichtigung der Korrektur eine korrigierte Konzentration des Analyten ermittelt wird.

Ausführungsform 14: Verfahren nach einer der vorhergehenden Ausführungsformen, wobei eine Veränderung der optischen Messgröße in dem zweiten Zeitabschnitt erfasst wird, wobei zur Bestimmung der Konzentration des Analyten die optische Messgröße zu einem Zeitpunkt verwendet wird, zu welchem eine zeitliche Änderung der optischen Messgröße unterhalb eines vorgegebenen Schwellwertes liegt.

Ausführungsform 15: Verfahren nach einer der vorhergehenden Ausführungsformen, wobei das Blut an mindestens einer Aufgabestelle auf das Testelement aufgebracht wird, wobei das Blut oder Bestandteile des Bluts von der Aufgabestelle zu der Testchemie transferiert werden.

Ausführungsform 16: Verfahren nach der vorhergehenden Ausführungsform, wobei das Blut oder die Bestandteile des Bluts auf dem Weg zwischen der Aufgabestelle und der Testchemie mindestens ein Abtrennelement passieren, wobei in dem Abtrennelement mindestens ein Bestandteil des Bluts aus dem Blut abgetrennt wird.

Ausführungsform 17: Verfahren nach der vorhergehenden Ausführungsform, wobei das Abtrennelement mindestens eine mit der Testchemie in Kontakt stehende Abtrennschicht umfasst.

Ausführungsform 18: Verfahren nach einer der drei vorhergehenden Ausführungsformen, wobei zwischen der Aufgabestelle und der Testchemie mindestens ein Kapillarelement vorgesehen ist, insbesondere ein Kapillarelement mit einer Länge von mindestens 1 mm und besonders bevorzugt von mindestens 3 mm oder sogar mit einer Länge von mindestens 10 mm.

Ausführungsform 19: Vorrichtung zur Bestimmung mindestens einer Konzentration mindestens eines Analyten in Blut, insbesondere zur Bestimmung einer Blutglukosekonzentration, wobei die Vorrichtung mindestens ein Testelement umfasst, wobei das Testelement mindestens eine Testchemie aufweist, wobei die Testchemie eingerichtet ist, um bei Anwesenheit des Analyten mindestens eine optisch nachweisbare Nachweisreaktion durchzuführen, wobei das Blut auf das Testelement aufbringbar ist, wobei die Vorrichtung mindestens eine optische Nachweisvorrichtung aufweist, wobei die optischen Nachweisvorrichtung eingerichtet ist, um einen zeitlichen Verlauf mindestens einer optischen Messgröße der Testchemie zu erfassen, wobei die Vorrichtung weiterhin mindestens eine Auswertevorrichtung aufweist, wobei die Auswertevorrichtung eingerichtet ist, um aus mindestens einem ersten Zeitabschnitt des zeitlichen Verlaufs der optischen Messgröße auf mindestens eine Störgröße des Bluts, insbesondere einen Hämatokrit des Bluts, zu schließen und wobei die Auswertevorrichtung weiterhin eingerichtet ist, um aus mindestens einem zweiten Zeitabschnitt des zeitlichen Verlaufs auf die Konzentration des Analyten zu schließen.

Ausführungsform 20: Vorrichtung nach der vorhergehenden Ausführungsform, wobei die Vorrichtung eingerichtet ist, um ein Verfahren nach einem der vorhergehenden Verfahrensansprüche durchzuführen.

Ausführungsform 21: Vorrichtung nach einer der vorhergehenden Ausführungsformen, wobei die Nachweisvorrichtung mindestens eine Abfragelichtquelle zur Bestrahlung der Testchemie mit mindestens einem Abfragelichtstrahl und mindestens einen Detektor zur Erfassung mindestens eines von der Testchemie ausgehenden Antwortlichtstrahls aufweist.

Ausführungsform 22: Vorrichtung nach einer der vorhergehenden Ausführungsformen, wobei die Auswertevorrichtung mindestens eine Datenverarbeitungsvorrichtung, insbesondere mindestens einen Mikrocomputer, umfasst.

Ausführungsform 23: Vorrichtung nach einer der vorhergehenden Ausführungsformen, wobei das Testelement ein Teststreifen ist, wobei die Vorrichtung mindestens eine Teststreifenaufnahme aufweist, wobei mindestens ein Teststreifen in der Teststreifenaufnahme in eine Applikationsposition bringbar ist, wobei in der Applikationsposition mindestens eine Aufgabestelle des Teststreifens durch einen Benutzer mit Blut beaufschlagbar ist, wobei der Teststreifen mindestens ein Kapillarelement aufweist, um das Blut oder Bestandteile des Bluts von der Aufgabestelle zu der Testchemie zu transferieren.

Ausführungsform 24: Vorrichtung nach der vorhergehenden Ausführungsform, wobei zwischen dem Kapillarelement und der Testchemie mindestens ein Abtrennelement zur Abtrennung mindestens eines Bestandteils des Bluts aus dem Blut angeordnet ist.

Ausführungsform 25: Vorrichtung nach der vorhergehenden Ausführungsform, wobei das Abtrennelement mindestens eine Abtrennschicht umfasst.

Ausführungsform 26: Vorrichtung nach einer der drei vorhergehenden Ausführungsformen, wobei der Teststreifen derart eingerichtet ist, dass ein in die Testchemie eingestrahlter Abfragelichtstrahl zunächst die Testchemie passiert, dann zumindest teilweise das Abtrennelement passiert, anschließend an mindestens einer reflektierenden Oberfläche, insbesondere der Abtrennschicht oder einer dahinter liegenden reflektierenden Oberfläche eines Trägerelements, reflektiert wird, anschließend optional erneut das Abtrennelement passiert, dann wiederum die Testchemie passiert und schließlich wieder aus dem Teststreifen austritt.

Ausführungsform 27: Vorrichtung nach einer der vorhergehenden Ausführungsformen, wobei das Testelement, insbesondere der Teststreifen, mindestens ein von einer Außenseite des Testelements sichtbares Testchemiefeld der Testchemie aufweist, wobei die optische Nachweisvorrichtung eingerichtet ist, um die mindestens eine optische Eigenschaft der Testchemie an dem Testchemiefeld zu erfassen.

Ausführungsform 28: Vorrichtung nach einer der vorhergehenden Ausführungsformen, wobei die optische Nachweisvorrichtung eingerichtet ist, um die optische Messgröße in dem ersten Zeitabschnitt und dem zweiten Zeitabschnitt bei derselben Wellenlänge zu erfassen.

Ausführungsform 29: Vorrichtung nach einer der vorhergehenden Ausführungsformen, wobei die optische Nachweisvorrichtung mindestens eine erste Leuchtdiode und mindestens eine zweite Leuchtdiode sowie mindestens eine Fotodiode zur Erfassung von an der Testchemie diffusiv reflektiertem Licht der Leuchtdioden aufweist. Ausführungsform 30: Vorrichtung nach der vorhergehenden Ausführungsform, wobei die Vorrichtung eingerichtet ist, um abwechselnd reflektiertes Licht der Leuchtdioden mit derselben Fotodiode zu erfassen.

Ausführungsform 31: Vorrichtung nach einer der beiden vorhergehenden Ausführungsformen, wobei die Auswertevorrichtung eingerichtet ist, um reflektiertes Licht der zweiten Leuchtdiode zur Erkennung einer Benetzung der Testchemie durch das Blut oder Blutbestandteile zu verwenden, wobei die Auswertevorrichtung weiterhin eingerichtet ist, um reflektiertes Licht der ersten Leuchtdiode zur Erfassung der optischen Messgröße, insbesondere einer Remission, während des ersten Zeitabschnitts und des zweiten Zeitabschnitts zu verwenden und daraus auf die mindestens eine Störgröße des Bluts sowie die Konzentration des Analyten zu schließen.

Ausführungsform 32: Verwendung eines Benetzungssprungs im zeitlichen Verlauf einer an einem Testelement erfassten optischen Messgröße, insbesondere einer Remission, zur Bestimmung einer Störgröße in Blut, insbesondere zur Bestimmung eines Hämatokrits.

Ausführungsform 33: Verwendung nach der vorhergehenden Ausführungsform, wobei die derart bestimmte Störgröße weiterhin zur Korrektur einer Bestimmung einer Konzentration mindestens eines Analyten in dem Blut verwendet wird, insbesondere zur Korrektur einer Bestimmung einer Blutglukosekonzentration.

Ausführungsform 34: Verwendung nach der vorhergehenden Ausführungsform, wobei zur Bestimmung der Störgröße und zur Bestimmung der Konzentration des Analyten dieselbe optische Messgröße verwendet wird, insbesondere eine optische Remission bei derselben Wellenlänge.

## Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.
Im Einzelnen zeigen:

Figur 1 eine schematische Schnittdarstellung einer erfindungsgemäßen Vorrichtung mit einem Testelement und einem Testgerät;

Figur 2 eine schematische Darstellung einer optischen Nachweisvorrichtung in Draufsicht;

| | |
|---|---|
| Figur 3 | eine schematische Darstellung eines zeitlichen Verlaufs einer relativen Remission und einer Unterteilung in eine ersten Zeitabschnitt und einen zweiten Zeitabschnitt; |
| Figur 4 | eine schematische Darstellung eines erfindungsgemäß verwendbaren Teststreifens in Draufsicht; |
| Figur 5 | eine schematische Querschnittdarstellung gemäß Figur 4; |
| Figur 6 | eine Messung eines zeitlichen Verlaufs einer relativen Remission für zwei Blutproben mit unterschiedlichem Hämatokrit; |
| Figur 7 | ein Zusammenhang eines Ausmaßes eines Benetzungssprungs und eines Hämatokrits; |
| Figur 8 | ein künstlich generiertes Beispiel einer Abhängigkeit der Abweichung einer Glukosekonzentration gegen eine Referenzmethode in Abhängigkeit vom Hämatokrit als Störgröße; |
| Figur 9 | ein zeitlicher Verlauf normierter Remissionswerte bei einer Messung an cNAD-Testchemie bei unterschiedlichen Blutglukosekonzentrationen und Hämatokritwerten; und |
| Figur 10 | ein Zusammenhang zwischen Benetzungssprung und Hämatokritwert bei einer Messung an Proben mit unterschiedlichen Blutglukosekonzentrationen. |

Ausführungsbeispiele

In Figur 1 ist ein Ausführungsbeispiel einer erfindungsgemäßen Vorrichtung 110 zur Bestimmung einer Konzentration eines Analyten in Blut, insbesondere zur Bestimmung einer Blutglukosekonzentration, in einer schematischen Schnittdarstellung gezeigt. Die Vorrichtung 110 umfasst in diesem Ausführungsbeispiel ein Testgerät 112, welches beispielsweise als Handgerät ausgestaltet sein kann, sowie ein Testelement 114, in diesem Fall beispielsweise einen Teststreifen 116. Das Testgerät 112 umfasst eine Testelementaufnahme 118, in welcher ein Teststreifen 116 aufgenommen und in eine Applikationsposition (in Figur 1 dargestellt) bringbar ist. In dieser Applikationsposition kann eine Blutprobe 120, beispielsweise ein Tropfen, an einer Aufgabestelle 122 auf das Testelement 114 aufgebracht werden.

Das Testelement 114 umfasst, wie in Figur 1 erkennbar, eine Testchemie 124, welche beispielsweise Bestandteil eines Testchemiefeldes 126 sein kann.

Weiterhin umfasst die Vorrichtung 110 bei dem in Figur 1 dargestellten Ausführungsbeispiel eine optische Nachweisvorrichtung 128, mittels derer mindestens eine optische Messgröße der Testchemie 124 und insbesondere ein zeitlicher Verlauf der optischen Messgröße erfassbar sind. Zu diesem Zweck kann die optische Nachweisvorrichtung 128 beispielsweise mindestens eine Lichtquelle 130 zur Erzeugung mindestens eines Abfragelichtstrahl 132 sowie mindestens einen Detektor 134 zur Detektion mindestens eines Antwortlichtstrahls 136 aufweisen, wobei der Antwortlichtstrahl 136 beispielsweise an der Testchemie 124 gestreutes oder diffusiv reflektiertes Abfragelicht sein kann. Weitere mögliche Einzelheiten der optischen Nachweisvorrichtung 128 werden unten noch näher erläutert.

Weiterhin umfasst die Vorrichtung 110 in dem dargestellten Ausführungsbeispiel mindestens eine Auswertevorrichtung 138. Diese Auswertevorrichtung 138 kann unidirektional oder bidirektional über mindestens eine Datenleitung 140 mit der optischen Nachweisvorrichtung 128 in Verbindung stehen und/oder kann auch ganz oder teilweise mit der optischen Nachweisvorrichtung 128 zusammengefasst sein, beispielsweise indem die Auswertevorrichtung 138 ganz oder teilweise in die optische Nachweisvorrichtung 128 integriert ist. Auch andere Ausgestaltungen sind möglich, beispielsweise dezentrale Ausgestaltungen. Die Auswertevorrichtung 138 kann beispielsweise mindestens eine Datenverarbeitungsvorrichtung 142 umfassen, beispielsweise einen Mikrocomputer. Die Datenverarbeitungsvorrichtung 142 kann beispielsweise programmtechnisch eingerichtet sein, um einen Programmablauf einer erfindungsgemäßen Ausgestaltung eines Verfahrens zur Bestimmung mindestens einer Konzentration mindestens eines Analyten in dem Blut 120 zu steuern und/oder auszuwerten. Weiterhin kann die Auswertevorrichtung 138 auch mindestens einen flüchtigen und/oder mindestens einen nicht-flüchtigen Datenspeicher 144 umfassen.

Die Vorrichtung 110 gemäß dem in Figur 1 dargestellten Ausführungsbeispiel kann darüber hinaus weitere Elemente umfassen, beispielsweise um eine Schnittstelle zwischen einem Benutzer der Vorrichtung 110 und der Vorrichtung 110 bereit zu stellen. Dementsprechend können beispielsweise ein oder mehrere Bedienelemente 146, beispielsweise eine oder mehrere Tasten, und/oder ein oder mehrere Anzeigeelemente 148 vorgesehen sein, welche ebenfalls, wie in Figur 1 dargestellt, unidirektional und bidirektional mit der Auswertevorrichtung 138 in Verbindung stehen können. Darüber hinaus kann die Vorrichtung 110 optional ein oder mehrere weitere Schnittstellen umfassen, beispielsweise ein oder mehrere Datenschnittstellen, mittels derer beispielsweise Daten zwischen der Vorrichtung 110 und einer oder mehreren weiteren Vorrichtungen ausgetauscht werden können, beispielswiese Messdaten und/oder

Messergebnisse.

In Figur 2 ist in Draufsicht eine optische Nachweisvorrichtung 128 in einer möglichen Ausgestaltung gezeigt. Die optische Nachweisvorrichtung 128 umfasst wiederum mindestens eine Abfragelichtquelle 130 und mindestens einen Detektor 134. Diese Elemente können insgesamt insbesondere mit einem Halbleiterbauelemente ausgestaltet sein. Die Abfragelichtquelle 130 umfasst in dem dargestellten Ausführungsbeispiel optional mindestens eine erste Leuchtdiode 150 und mindestens eine zweite Leuchtdiode 152. Die erste Leuchtdiode 150 ist im Folgenden auch mit LED 1 bezeichnet und die zweite Leuchtdiode 152 mit LED 2. Wie in Figur 2 erkennbar, kann insbesondere die erste Leuchtdiode auch mehrteilig ausgestaltet sein und umfasst in diesem Ausführungsbeispiel quer zu einer Längserstreckungsrichtung des Testelements 114 eine erste Leuchtdiode A (Bezugsziffer 154, LED1A) und eine erste Leuchtdiode B (Bezugsziffer 156, LED1B). Diese mehrteilige Ausgestaltung der ersten Leuchtdiode 150 kann beispielsweise dazu dienen, eine bessere Abdeckung des Testchemiefeldes 126 mit Abfragelicht zu bewirken. Während die erste Leuchtdiode 150, welche beispielsweise bei 660 Nanometern emittieren kann, zur Bestimmung des Remissionswertes zum Zweck einer Bestimmung der Analytkonzentration eingesetzt wird, kann die zweite, optionale Leuchtdiode 152 beispielsweise eingesetzt werden, um einen Start eines Benetzungssprungs zu erkennen. Die zweite Leuchtdiode 152 kann insbesondere beispielsweise bei 880 nm emittieren. Das von dem Testchemiefeld 126 remittierte, d.h. diffusiv gestreute Abfragelicht 132 in Form des Antwortlichtstrahls 136 wird von dem Detektor 134, beispielsweise einer Fotodiode, detektiert. In Figur 3 ist exemplarische ein Zeitverlauf eines von dem Detektor 134 typischerweise erfassten Signals als Beispiel eines zeitlichen Verlaufs eines optischen Messwerts dargestellt. Aufgetragen ist hierbei die so genannte relative Remission (rR) angegeben in Prozent, als Funktion der Zeit t.

Aus dieser schematischen Darstellung in Figur 3 geht hervor, dass die Remission zunächst, vor Aufbringen einer Probe des Bluts 120, bei einem so genannten Leerwert verbleibt, welcher willkürlich auf 100 % relative Remission gesetzt wird und welcher als Bezugswert für die nachfolgend erfassten Signale verwendet wird.

Nachdem das Blut oder Bestandteile des Bluts die Testchemie 124 erreicht haben, beginnt eine Benetzungsphase, welche sich in dem in Figur 3 dargestellten zeitlichen Verlauf bemerkbar macht. Diese Benetzungsphase ist optional mittels der zweiten Leuchtdiode 152 erkennbar. Beispielsweise können die erste Leuchtdiode 150 und die zweite Leuchtdiode 152 abwechselnd eingeschaltet werden, beispielsweise in einem gepulsten intermittierenden Betrieb. Alternativ oder zusätzlich kann der Beginn einer Benetzungsphase auch mittels der ersten Leuchtdiode 150 erfasst werden, so dass insgesamt nur eine Leuchtdiode erforderlich ist.

Mit dem Beginn der Benetzungsphase fällt die relative Remission in Figur 3 stark ab. Dieser Zeitpunkt ist in Figur 3 mit $t_0$ bezeichnet. Dieser Zeitpunkt kann beispielsweise durch einen Schwellwertvergleich der Remission und/oder durch eine Betrachtung einer Änderung der Remission erkannt werden.

Der Zeitpunkt $t_0$ oder beispielsweise ein früherer Zeitpunkt als $t_0$ kann allgemein beispielsweise als Anfangszeitpunkt eines ersten Zeitabschnitts angesetzt werden, welcher in Figur 3 mit der Bezugsziffer 158 bezeichnet ist. Der Zeitpunkt $t_0$ kann beispielsweise durch einen Vergleich der relativen Remission oder einer Ableitung des zeitlichen Verlaufs der relativen Remission mit mindestens einem Schwellwert und/oder durch eine Betrachtung der Ableitung des zeitlichen Verlaufs der relativen Remission bestimmt werden, beispielsweise automatisch. Grundsätzlich ist eine genaue Bestimmung des Zeitpunkts $t_0$ jedoch in der Regel nicht erforderlich, da sich die relative Remission vor dem Zeitpunkt $t_0$ in der Regel kaum ändert, so dass auch frühere Zeitpunkte als $t_0$ als Anfangszeitpunkt des ersten Zeitabschnitts 158 verwendet werden können. Am Zeitpunkt $t_0$ tritt üblicherweise ein deutlicher erster Knick auf. Beginnend mit dem Zeitpunkt $t_0$ erfolgt ein Abfall der relativen Remission um einen Betrag $\Delta rR$, welcher auch als Benetzungssprung bezeichnet wird und welcher in Figur 3 mit der Bezugsziffer 160 bezeichnet ist.

Am Ende des Benetzungssprungs 160, nachdem die relative Remission um den Betrag $\Delta rR$ abgefallen ist, zeigt der zeitliche Verlauf der relativen Remission in der Regel einen deutlichen zweiten Knick 162. Dieser Knick, welcher beispielswiese wiederum durch eine Betrachtung einer Ableitung des zeitlichen Verlaufs in Figur 3 und/oder durch Vergleich der Absolutwerte der relativen Remission und/oder der zeitlichen Änderung der relativen Remission mit mindestens einem vorgegebenen Schwellwert automatisch erkannt werden kann, tritt zu einem Zeitpunkt auf, welcher in Figur 3 mit $t_1$ bezeichnet wird. Dieser Zeitpunkt $t_1$, welcher den Ende des Benetzungssprungs 160 charakterisiert, kann als Endzeitpunkt des ersten Zeitabschnitts 158 angesetzt werden. Gleichzeitig kann dieser Zeitpunkt $t_1$ als Anfangszeitpunkt eines zweiten Zeitabschnitts angesetzt werden, welcher in Figur 3 mit der Bezugsziffer 164 bezeichnet ist. Während dem ersten Zeitabschnitt der zeitliche Verlauf in Figur 3 durch Benetzungseffekte der Testchemie 124 dominiert ist, ist im zweiten Zeitabschnitt 164 der zeitliche Verlauf der relativen Remission durch einen Fortschritt der optischen Nachweisreaktion dominiert, welche innerhalb der Testchemie 124 abläuft. Der zweite Zeitabschnitt 164 kann als offenes Intervall ausgestaltet werden und kann beispielsweise den gesamten zeitlichen Verlauf für Zeiten $t \geq t_1$ umfassen. Alternativ kann der zweite Zeitabschnitt 164 auch zu einem vorgegebenen Zeitpunkt enden, beispielsweise zum Zeitpunkt $t_2$ in Figur 3, welcher unten noch näher erläutert wird. Allgemein wird darauf hingewiesen, dass die Zeitabschnitte 158, 164 als geschlossene Intervalle, halb-offene Intervalle oder auch als offene Zeitintervalle ausgestaltet sein können.

Bei der Auswertung des zeitlichen Verlaufs der optischen Messgröße, in Figur 3 exemplarische der relativen Remission, wird aus dem ersten Zeitabschnitt 158 auf eine Störgröße, beispielsweise dem Hämatokrit, geschlossen.

Hierzu kann beispielsweise der Sprung in der Remission betrachtet werden, welcher während des Benetzungssprungs 160 auftritt und welcher in Figur 3 mit $\Delta rR$ bezeichnet ist. Dieses $\Delta rR$ kann als charakteristische Größe verwendet werden, um hieraus, gemäß beispielsweise einem nachfolgend noch näher beschriebenen Algorithmus, auf die Störgröße, insbesondere den Hämatokrit, zu schließen. Aus dem zweiten Zeitabschnitt 164 hingegen wird die Analytkonzentration, beispielsweise die Glukosekonzentration, abgeleitet. Dies kann beispielsweise derart erfolgen, dass zu einem festen Zeitpunkt innerhalb des zweiten Zeitabschnitts 164 ein Remissionswert rR erfasst wird, beispielsweise zu einer fest vorgegebenen Zeit nach dem Zeitpunkt $t_0$ oder nach dem Zeitpunkt $t_1$. Die dann erfasste Remission rR kann beispielsweise als zweite charakteristische Größe angesetzt werden, aus welcher die Analytkonzentration bestimmt wird. Alternativ oder zusätzlich kann der Zeitpunkt $t_2$, zu welchem die Remission zur Bestimmung der Analytkonzentration erfasst wird, auch als so genannter "Endzeitpunkt" ausgestaltet sein und variabel gewählt werden. Als Endzeitpunkt wird dabei allgemein ein Zeitpunkt verstanden, zu welchem die Nachweisreaktion im Wesentlichen vollständig abgelaufen ist. Dieser im Wesentlichen vollständige Ablauf der Nachweisreaktion kann beispielsweise dadurch erfasst werden, dass geprüft wird, ob innerhalb eines vorgegebenen geringen Zeitintervalls $\Delta t$ sich die relative Remission $\Delta rR$ um nicht mehr als einen vorgegebenen Schwellwert oder um weniger als einen vorgegebenen Schwellwert ändert. Durch eine derartige Schwellwertbedingung kann der Endzeitpunkt $t_2$ festgestellt werden und die Remission rR von $t_2$ als weitere charakteristische Größe oder zweite charakteristische Größe zur Bestimmung der Analytkonzentration aus dem Zeitverlauf des zweiten Zeitabschnitts 164 bestimmt werden.

In den Figuren 4 und 5 ist in einer Draufsicht (Figur 4) und in einer Schnittdarstellung (Figur 5) ein Ausführungsbeispiel eines erfindungsgemäß bevorzugt einsetzbaren Testelements 114 in Form eines Teststreifens 116 gezeigt. Der Teststreifen 116 ist in diesem Ausführungsbeispiel als Kapillar-Teststreifen ausgestaltet und umfasst ein in Figur 4 gestrichelt dargestelltes Kapillarelement 166. Die Schnittebene in Figur 5 ist derart gewählt, dass diese längs durch das Kapillarelement 166 verläuft. Das Kapillarelement 166 ist eingerichtet, um Blut von einer Aufgabestelle 122, an welcher die Probe aufgebracht wird, zu der Testchemie 124, insbesondere einem Testchemiefeld 126, durch Kapillarkräfte zu transferieren. Wie in Figur 5 erkennbar, kann das Kapillarelement 166 beispielsweise durch einen Schichtaufbau in dem Teststreifen 116 realisiert werden. Zu diesem Zweck kann der Schichtaufbau beispielsweise ein Trägerelement 168 umfassen, auf welches ein oder mehrere Abstandhalter 170 (Spacer) aufgebracht sind. Diese Abstandhalter 170 sind mittig voneinander beabstandet, so dass zwischen den Abstandhaltern 170 das Kapillarelement 166 ausgebildet wird. Die Abstandhalter 170 sind wiederum durch mindestens eine Deckfolie 172 abgedeckt, welche das Kapillarelement 166 verschließt. In der Deckfolie 172 ist eine Öffnung 174 vorgesehen (siehe Figur 5) beispielsweise ein Fenster. Diese Öffnung 174 ist in dem dargestellten Ausführungsbeispiel abgedeckt durch das Testchemiefeld 126 mit der Testchemie 124 zwischen dem Kapillarelement 166 und dem Testchemiefeld 126 kann optional mindestens ein Abtrennelement 176 vorgesehen sein, beispielsweise mindestens eine Abtrennschicht 178. Diese dienen zum Abtrennen von Blutbestandteilen, beispielsweise roten Blutkörperchen, um diese zumindest weitgehend von der Testchemie 124 fern zu halten.

Wie in Figur 5 erkennbar, durchstrahlt der Abfragelichtstrahl 132 das Testchemiefeld 126 und optional die Abtrennschicht 178. Unmittelbar an der Testchemie 124 und/oder an dem Abtrennelement 176 und/oder, wie in Figur 5 erkennbar, an einer reflektierenden Oberfläche 180 des Trägerelements 178 wird der Abfragelichtstrahl 132 optional reflektiert und/oder gestreut. Zu diesem Zweck können das Trägerelement 168 und/oder die reflektierende Oberfläche 180 zusätzlich mit reflektierenden Oberflächen mit reflektierenden Eigenschaften ausgestaltet sein, beispielsweise indem das Trägerelement 168 weiß ausgestaltet wird und/oder mit weißen Pigmenten versetzt wird, beispielsweise Titandioxid. Lagern sich in der Abtrennschicht 178 und/oder beispielsweise an einer Grenzfläche zwischen der Abtrennschicht 178 und dem Kapillarelement 166 Störkomponenten an, beispielsweise rote Blutkörperchen, so werden diese Anlagerungen bei dem optischen Aufbau gemäß Figur 5 erkennbar mindestens zweimal durchdrungen, so dass der optische Nachweis in erheblichen Maße beispielsweise durch den Hämatokrit beeinflusst werden kann. Insofern kann eine Hämatokritkorrektur gemäß der vorliegenden Erfindung gerade bei Aufbauten gemäß den Figuren 4 und 5 zu einer erheblichen Steigerung der Nachweisgenauigkeit beitragen.

In Figur 6 ist ein Messbeispiel gezeigt, anhand dessen erkennbar ist, dass ein Remissionssprung während eines ersten Zeitabschnitts 158 tatsächlich abhängig ist von einem Hämatokrit. Dieses Ausführungsbeispiel zeigt, dass für die Durchführung eines erfindungsgemäßen Verfahrens eine exakte Bestimmung der Grenzzeitpunkte des ersten Zeitabschnitts 158 und des zweiten Zeitabschnitts 164 nicht ohne Weiteres erforderlich ist.

In Figur 6 sind relative Remissionen analog zu der Darstellung in Figur 3 aufgetragen. Die Zeit t ist hier in willkürlichen Einheiten eines Messtakts einer Messung aufgetragen, beispielsweise entsprechend einer internen Uhr (Clock) des Testgeräts 112 und/oder der Datenverarbeitungsvorrichtung 142. Der Zeitpunkt, zu welchem die Probe 120 auf das Testelement 114 aufgebracht wurde, wurde willkürlich als Nullpunkt der Zeitachse angesetzt.

Aufgetragen sind in Figur 6 zwei Messkurven, die bei unterschiedlichen Blutproben 120 gewonnen wurden. So zeigt Bezugsziffer 182 eine Messung an einer Blutprobe 120 mit einem Hämatokrit von 25 %, und Kurve 184 zeigt eine Messung an einer Blutprobe 120 mit einem Hämatokrit von 55 %. Der Glukosegehalt in beiden Proben wurde dabei gleich gewählt. Für den Aufbau der verwendeten Testelemente 114 und der Testchemie 124 kann exemplarisch auf EP 1 035 921 B1, auf EP 1 039 298 B1, auf WO 2007/118647 oder auf EP 0 821 234 B1 verwiesen werden. Alternative

Ausgestaltungen, welche ebenfalls eingesetzt werden könnten, sind beispielsweise in EP 1 035 919 B1 oder in EP 1 035 920 B1 beschrieben.

Deutlich ist in Figur 6 erkennbar, dass die beiden Proben zu einem unterschiedlichen Benetzungssprung $\Delta rR$ führen. Während die Kurve 182 einen Benetzungssprung $\Delta rR$ von näherungsweise 12 % aufweist, zeigt die Kurve 184 einen Benetzungssprung von näherungsweise 20 %.

In Figur 7 sind systematische Messungen einer Abhängigkeit der Remissionsänderung $\Delta rR$ während des Benetzungssprungs vom Hämatokrit Hct angegeben. Pro Hämatokritwert wurden jeweils zehn Blutproben mit jeweils 50, 120 und 300 mg/dL Glukose hergestellt und ausgewertet. Diese unterschiedlichen Glukosekonzentrationen sind in Figur 7 jeweils mit unterschiedlichen Symbolen bezeichnet. Dabei bezeichnen die Kreuze Konzentrationen von 50 mg/dL, die nicht ausgefüllten Kreise Konzentrationen von 150 mg/dL und die waagerechten Striche Konzentrationen von 300 mg/dL Glukose. Die durchgezogene Linie stellt eine Anpassungsgerade durch sämtliche Messpunkte dar, deren Gleichung rechts oben in Figur 7 angegeben ist.

Die Messergebnisse in Figur 7 zeigen, dass der Benetzungssprung $\Delta rR$ mit hoher Reproduzierbarkeit vom Hämatokrit abhängig ist. Die gestrichelte Gerade sowie die Angaben im rechten oberen Feld der Figur 7 zeigen eine willkürliche Anpassung einer Geraden an den Messverlauf der Punkte in Figur 7. Es zeigt sich, dass die Abhängigkeit des Benetzungssprungs $\Delta rR$ vom Hämatokrit sich in guter Näherung in Form einer Geraden darstellen lässt.

Der Hämatokrit HKT (auch bezeichnet als HK oder HCT) kann aus dem Abfall der Remission am Benetzungssprung $\Delta Rem$ (entsprechend beispielsweise $\Delta rR$ in Figur 6) daher beispielsweise berechnet werden gemäß der folgenden Formel:

$$HKT = a * \Delta Rem\,(\text{Benetzungssprung})^{\,b} + c. \qquad (1)$$

Die Glieder a, b und c können beispielsweise für eine spezifische Testchemie einmalig ermittelt werden. Beispielsweise können diese Glieder a, b und c in dem Datenspeicher 144 der Datenverarbeitungsvorrichtung 142 hinterlegt werden und/oder auf andere Weise bereitgestellt werden, beispielsweise indem diese, wie oben ausgeführt, als Korrekturen an die Vorrichtung 110 und/oder das Testgerät 112 übermittelt werden, beispielsweise über mindestens eine Schnittstelle und/oder mittels mindestens eines ROM-Keys und/oder mittels mindestens eines RFID-Chips. Alternativ oder zusätzlich können die genannten Glieder nach Prüfung auch nachgestellt und so mitgegeben werden. Alternativ oder zusätzlich sind auch andere Möglichkeiten denkbar.

Da auf diese Weise anhand des zeitlichen Verlaufs der Remission mit hoher Präzision nunmehr ein Hämatokrit bestimmt werden kann, kann eine Korrektur der aus dem zweiten Zeitabschnitt 164 gewonnenen Glukosemesswerte aufgrund des bekannten Hämatokrits erfolgen. Wie oben ausgeführt, kann diese Korrektur nachfolgend durchgeführt werden oder kann bereits bei der ersten Berechnung des Analytgehalts in eine Umrechnung einfließen.

Die Korrektur der Glukosekonzentration unter Berücksichtigung des Hämatokrits und/oder einer anderen Störgröße kann, wie oben ausgeführt, insbesondere durch Verwendung einer oder mehrerer Korrekturfunktionen erfolgen. Beispielsweise kann eine Korrekturfunktion in Form einer Korrekturgleichung verwendet werden, bei welcher die gemessene Glukosekonzentration beispielsweise mit vom Hämatokrit und/oder anderen Störgrößen abhängigen Korrekturgliedern korrigiert wird. Beispielsweise können dabei ein oder mehrere Korrekturfaktoren und/oder ein oder mehrere Korrektur-Offsets verwendet werden. Wie oben ausgeführt, kann ein Korrektur-Offset, welcher vom Hämatokrit abhängig sein kann, beispielsweise auch als Störbetrag bezeichnet werden. So kann eine Korrektur der Glukosekonzentration durch den Hämatokrit beispielsweise durch eine Addition oder Subtraktion eines Korrektur-Offsets erfolgen, wobei der Korrektur-Offset eine Funktion des Hämatokrits und/oder einer anderen Art von Störgröße ist. In der Praxis hat es sich gezeigt, dass die Korrektur der Glukosekonzentration durch den Hämatokrit beispielsweise nach folgender Formel erfolgen kann:

$$c(Gluc)_{korr} = c(Gluc) + m * HKT^{\,i} + n \qquad (2)$$

Dabei stellt $c(Gluc)_{korr}$ die korrigierte Glukosekonzentration dar und c(Gluc) die gemessene Glukosekonzentration. Die Glieder m und i sind experimentell zu ermittelnde Korrekturglieder der Korrekturfunktion, welche beispielsweise auch von der Temperatur und der Glukosekonzentration selbst abhängen können.

Es wird darauf hingewiesen, dass das oben genannte Beispiel einer Korrektur des Hämatokrits und/oder einer anderen Störgröße lediglich exemplarisch zu verstehen ist. Zahlreiche andere mögliche Korrekturen sind denkbar. So ist oben beispielsweise ein zweistufiges Verfahren dargestellt, bei welchem zunächst nach der Formel (1) aus dem Benetzungssprung auf den Hämatokrit geschlossen wird und anschließend nach der Formel (2) aus dem Hämatokrit auf eine korrigierte Glukosekonzentration geschlossen wird. Es ist unmittelbar ersichtlich, dass die Formeln (1) und (2) auch zusammengefasst werden könnten zu einem einstufigen Verfahren, bei welchem unmittelbar aus dem Benetzungssprung und der Glukosekonzentration eine korrigierte Glukosekonzentration berechnet wird. Zahlreiche weitere Möglichkeiten sind denkbar. In einer weiteren Vereinfachungsstufe könnte beispielsweise auch die Hämatokritabhängigkeit des Systems unabhängig von der Glukosekonzentration aus dem Remissionsabfällen am Benetzungssprung bestimmt werden.

Eine technische Implementierung der Korrektur kann auf einfache Weise erfolgen, beispielsweise unter Verwendung der Datenverarbeitungsvorrichtung 142. Wie oben ausgeführt, kann die Korrektur beispielsweise in einer einfachen Addition oder Subtraktion eines oder mehrerer in Versuchen ermittelten Abweichungen je Glukosekonzentration und Hämatokrit zu einer Glukose-Referenzmessung bestehen. Zu diesem Zweck können beispielsweise im Testgerät 112, beispielsweise in dem Datenspeicher 144, ein oder mehrere für die Korrektur benötigte Korrekturglieder und/oder Korrekturfunktionen hinterlegt werden. Beispielsweise können ein oder mehrere Abhängigkeitskurven und/oder Abhängigkeitstabellen, welche die Korrektur beschreiben, hinterlegt werden, beispielsweise in Form von Polygonzügen oder Hyperflächen. Anhand des ermittelten HKTs und der am Ende der Messung bekannten Glukosekonzentration wird dieser Korrekturbetrag beispielsweise addiert oder subtrahiert.

Exemplarisch ist diese Hinterlegung von Korrekturen in Figur 8 dargestellt. Figur 8 zeigt ein künstlich generiertes Beispiel einer Abhängigkeit einer Abweichung $\Delta$ der gemessenen, unkorrigierten Glukosekonzentration von einer mittels einer verlässlichen Referenzmethode ermittelten tatsächlichen Glukosekonzentration. Dabei werden beispielsweise Blutproben mit verschiedenen Hämatokritwerten erzeugt, wobei beispielsweise Proben mit Hämatokriten von 25, 30, 35, 43, 50, 55, 60 und 65 % verwendet werden können, also Hämatokrite, welche einen in der Praxis typischerweise maximal auftretenden Bereich abdecken sollten. Derartige Blutproben werden mit unterschiedlichen Glukosekonzentrationen erzeugt, beispielsweise mit Glukosekonzentrationen von 0-20 mg/dL, 50 mg/dL, 100 mg/dL, 300 mg/dL und 450 mg/dL. Die tatsächlichen Glukosekonzentrationen werden beispielsweise mit einem Laborgerät oder auf andere Weise gemessen, und es wird die Abweichung $\Delta$ zur jeweiligen, mittels des mindestens einen optischen Messwerts (beispielsweise der Remission) ermittelten unkorrigierten Glukosekonzentration bestimmt. So sind in Figur 8 exemplarisch für eine Probe mit einem Hämatokrit von 30 % auf der horizontalen Achse die mittels der verlässlichen Referenzmethode ermittelten tatsächlichen Glukosekonzentrationen c der Probe in mg/dL aufgetragen. Auf der vertikalen Achse ist jeweils für eine Vielzahl unterschiedlicher Experimente die Abweichung zwischen der unkorrigierten, mittels der Remission bestimmten Glukosekonzentration und der tatsächlichen Glukosekonzentration aufgetragen. Für tatsächliche Glukosekonzentrationen unterhalb von 100 mg/dL sind dabei die Abweichungen $\Delta$ als Absolutwerte in mg/dL angegeben, und für tatsächliche Glukosekonzentrationen oberhalb von 100 mg/dL in Prozent. Derartige Kurven oder Polygonzüge, wie in Figur 8 dargestellt, lassen sich nacheinander für eine Vielzahl von Hämatokriten bestimmen, so dass, wenn die Kurven aneinander gesetzt werden, eine Hyperfläche entsteht, bei welcher beispielsweise die tatsächliche Konzentration c auf einer ersten Achse aufgetragen wird, der Hämatokrit auf einer zweiten Achse und die Abweichung $\Delta$ auf einer dritten Achse. Derartige Hyperflächen lassen sich beispielsweise mittels einzelner Werte tabellarisch, analytisch oder auf andere Weise im Datenspeicher 144 hinterlegen, so dass jeweils für jeden Hämatokrit und jede Glukosekonzentration das zugehörige $\Delta$ ermittelt und gemäß der oben genannten Gleichungen (1) und (2) der Hämatokritkorrektur von der unkorrigierten gemessenen Glukosekonzentration abgezogen oder zu selbiger hinzugezählt werden kann, um zu einem korrigierten Wert der Glukosekonzentration zu gelangen. Die Hämatokrit-Abhängigkeit ist über unterschiedliche Chargen der Testchemie 124 zumindest weitgehend stabil. Die ermittelten Korrekturwerte gelten somit universell für eine Kombination aus einem Testgerät 112 und einem Testelement 114 mit einer bestimmten Testchemie 124.

In den Figuren 9 und 10 sind weitere Ausführungsbeispiele von Messungen dargestellt, welche das Funktionsprinzip der vorliegenden Erfindung verdeutlichen. Bei diesen Messungen wurde wiederum, wie auch in den zuvor dargestellten Messungen, ein Testelement 114 verändert, welches jedoch in dem dargestellten Ausführungsbeispiel eine Testchemie 124 mit einem cNAD-Coenzym aufwies, wie beispielsweise in A. v. Ketteler et al.: Fluorescence Properties of Carba Nicotinamide Adenine Dinucleotide for Glucose Sensing, ChemPhysChem 2012, 13, 1302-1306 beschrieben.

Wiederum wurde, analog zu den oben dargestellten Figuren 3 und 6, ein zeitlicher Verlauf einer Remissionskurve aufgenommen, und es wurde der Benetzungssprung $\Delta rR$ gemessen. Dabei zeigt Figur 9 einen zeitlichen Verlauf einer normierten Remission $R_N$, also Remissionskurven, welche anfänglich auf den Remissionswert 1 normiert sind. Auf der horizontalen Achse ist die Zeit t in Sekunden s aufgetragen. Die Messungen wurden an der oben genannten cNAD-Testchemie durchgeführt, wobei die dargestellten zeitlichen Verläufe und Benetzungssprünge mit einer Wellenlänge von 600 nm erfasst wurden. Die zeitlichen Verläufe sind dargestellt für verschiedene, in der Legende angegebene Blutglukosekonzentrationen von 50 mg/dl, 100 mg/dl und 350 mg/dl, wobei für jede Blutglukosekonzentration jeweils Proben mit einem Hämatokrit (hier bezeichnet als HK) von 20 %, 40 % und 60 % verwendet wurden. Die Blutglukosekonzentrationen wurden mit einer Anregungswellenlänge von 365 nm bestimmt.

In den Messungen gemäß Figur 9 ist bereits deutlich erkennbar, dass der Benetzungssprung in seiner Höhe und seinem Zeitpunkt stark variiert. So tritt der Benetzungssprung für Proben mit der gleichen Blutglukosekonzentration bei höheren Hämatokritwerten später ein als bei niedrigeren Hämatokritwerten. Zudem fällt für Proben mit der gleichen Blutglukosekonzentration bei höheren Hämatokritwerten der Benetzungssprung erheblich ausgeprägter aus als bei

Proben mit niedrigerem Hämatokritwert.

Dieser Zusammenhang ist in Figur 10 nochmals verdeutlicht. In dieser Figur ist, ebenfalls für eine cNAD-Testchemie, jeweils der Benetzungssprung $\Delta rR$, angegeben in % relative Remission (rR), auf der vertikalen Achse aufgetragen. Auf der horizontalen Achse ist der zugehörige Hämatokritwert HKT, angegeben in Prozent, dargestellt. Die Messungen des Benetzungssprungs wurden in diesem Fall mit einer infraroten Leuchtdiode mit einer Emissionswellenlänge von 880 nm durchgeführt. Die Messungen wurden an Blutproben mit Hämatokritwerten von 20 %, 30 %, 40 %, 50 % und 60 % durchgeführt, jeweils für Blutglukosekonzentrationen von 0 mg/dl und 550 mg/dl, wie in der Legende angegeben.

Die Messungen in Figur 10 verdeutlichen das bereits in Figur 9 erkennbare Ergebnis, dass der Benetzungssprung, abgesehen von unvermeidlichen Ausreißern in den Messwerten, im Wesentlichen unabhängig ist von der Blutglukosekonzentration, selbst über den gesamten, in der Praxis relevanten Bereich bis hin zu Blutglukosekonzentrationen von 550 mg/dl. Der Benetzungssprung ist jedoch, wie bereits bei den Messungen in Figur 9, deutlich abhängig von den jeweiligen Hämatokritwert. Wiederum kann somit, wie bereits zuvor erläutert, auch bei der cNAD-Testchemie aufgrund einer Erfassung des Benetzungssprungs eine von der Blutglukosekonzentration unabhängige Aussage über den Hämatokritwert getroffen werden. Diese unabhängige Aussage kann dann wiederum, beispielsweise mittels eines Korrekturalgorithmus gemäß Gleichung (2) oben, zur Ermittlung einer korrigierten Blutglukosekonzentration $c(Gluc)_{korr}$ genutzt werden.

Bezugszeichenliste

| | |
|---|---|
| 110 | Vorrichtung zur Bestimmung einer Konzentration eines Analyten in Blut |
| 112 | Testgerät |
| 114 | Testelement |
| 116 | Teststreifen |
| 118 | Testelementaufnahme |
| 120 | Blutprobe |
| 122 | Aufgabestelle |
| 124 | Testchemie |
| 126 | Testchemiefeld |
| 128 | optische Nachweisvorrichtung |
| 130 | Abfragelichtquelle |
| 132 | Abfragelichtstrahl |
| 134 | Detektor |
| 136 | Antwortlichtstrahl |
| 138 | Auswertevorrichtung |
| 140 | Datenleitung |
| 142 | Datenverarbeitungsvorrichtung |
| 144 | Datenspeicher |
| 146 | Bedienelement |
| 148 | Anzeigeelement |
| 150 | erste Leuchtdiode (LED1) |
| 152 | zweite Leuchtdiode (LED2) |
| 154 | erste Leuchtdiode A (LED1A) |
| 156 | erste Leuchtdiode B (LED1B) |
| 158 | erster Zeitabschnitt |
| 160 | Benetzungssprung |
| 162 | Knick |
| 164 | zweiter Zeitabschnitt |
| 166 | Kapillarelement |
| 168 | Trägerelement |
| 170 | Abstandhalter |
| 172 | Deckfolie |
| 174 | Öffnung |
| 176 | Abtrennelement |
| 178 | Abtrennschicht |
| 180 | refaktierende Oberfläche |
| 182 | Hämatokrit 25 % |
| 184 | Hämatokrit 55 % |

## Patentansprüche

1. Verfahren zur Bestimmung mindestens einer Konzentration mindestens eines Analyten in Blut, insbesondere zur Bestimmung einer Blutglukosekonzentration, wobei ein Testelement (114) verwendet wird, wobei das Testelement (114) mindestens eine Testchemie (124) aufweist, wobei die Testchemie (124) eingerichtet ist, um bei Anwesenheit des Analyten mindestens eine optisch nachweisbare Nachweisreaktion durchzuführen, wobei das Blut auf das Testelement (114) aufgebracht wird, wobei ein zeitlicher Verlauf mindestens einer optischen Messgröße der Testchemie (124) erfasst wird, wobei aus mindestens einem ersten Zeitabschnitt (158) des zeitlichen Verlaufs der optischen Messgröße auf mindestens eine Störgröße des Bluts geschlossen wird und wobei aus mindestens einem zweiten Zeitabschnitt (164) des zeitlichen Verlaufs auf die Konzentration des Analyten geschlossen wird, wobei ein Zeitabschnitt (158, 164) jeweils eine mindestens zwei Messzeitpunkte umfassende Menge an Messzeitpunkten ist, wobei zur Erfassung der optischen Messgröße in dem ersten Zeitabschnitt (158) und in dem zweiten Zeitabschnitt (164) jeweils mittels mindestens einer Abfragelichtquelle (130) mindestens ein Abfragelichtstrahl (132) auf die Testchemie (124) eingestrahlt werden und wobei jeweils mittels mindestens eines Detektors mindestens ein von der Testchemie (124) ausgehender Antwortlichtstrahl erfasst werden, **dadurch gekennzeichnet, dass** die Störgröße ein Hämatokrit des Bluts ist, wobei der zeitliche Verlauf der optischen Messgröße in dem ersten Zeitabschnitt (158) einen Benetzungssprung (160) der optischen Messgröße umfasst, wobei der Benetzungssprung (160) eine sprunghafte Veränderung der optischen Messgröße ist, welche auf eine Benetzung der Testchemie (124) mit dem Blut zurückzuführen ist, wobei aus dem zeitlichen Verlauf der optischen Messgröße während des ersten Zeitabschnitts (158) mindestens eine charakteristische Größe bestimmt wird, wobei die charakteristische Größe eine Remissionswertänderung während des Benetzungssprungs (160) oder während eines Teils des Benetzungssprungs (160) ist, wobei ein vorgegebener und/oder bestimmbarer Zusammenhang zwischen der charakteristischen Größe und der Störgröße verwendet wird, um aus der charakteristischen Größe auf die mindestens eine Störgröße zu schließen.

2. Verfahren nach dem vorhergehenden Anspruch, wobei der erste Zeitabschnitt (158) ein anfänglicher Zeitabschnitt des zeitlichen Verlaufs ist, wobei der zweite Zeitabschnitt (164) dem ersten Zeitabschnitt (158) zeitlich nachgeordnet ist.

3. Verfahren nach einem der vorhergehenden Ansprüche, wobei die optische Messgröße bei mindestens einer ersten Wellenlänge erfasst wird, wobei aus einer starken Änderung der optischen Messgröße nach dem Aufbringen des Bluts auf das Testelement (114) auf einen Benetzungszeitpunkt geschlossen wird, zu welchem das Blut die Testchemie (124) erreicht und benetzt.

4. Verfahren nach dem vorhergehenden Anspruch, wobei der Abfragelichtstrahl (132) in dem ersten Zeitabschnitt (158) und in dem zweiten Zeitabschnitt (164) dieselbe Wellenlänge und/oder dieselben spektralen Eigenschaften aufweist.

5. Verfahren nach einem der beiden vorhergehenden Ansprüche, wobei der Antwortlichtstrahl (136) in dem ersten Zeitabschnitt (158) und dem zweiten Zeitabschnitt (164) dieselbe Wellenlänge und/oder dieselben spektralen Eigenschaften aufweist.

6. Verfahren nach einem der vorhergehenden Ansprüche, wobei mittels der Störgröße, insbesondere des Hämatokrits, mindestens eine Korrektur ermittelt wird, wobei aus dem zweiten Zeitabschnitt (164) unter Berücksichtigung der Korrektur eine korrigierte Konzentration des Analyten ermittelt wird.

7. Verfahren nach einem der vorhergehenden Ansprüche, wobei eine Veränderung der optischen Messgröße in dem zweiten Zeitabschnitt (164) erfasst wird, wobei zur Bestimmung der Konzentration des Analyten die optische Messgröße zu einem Zeitpunkt verwendet wird, zu welchem eine zeitliche Änderung der optischen Messgröße unterhalb eines vorgegebenen Schwellwertes liegt.

8. Verfahren nach einem der vorhergehenden Ansprüche, wobei das Blut an mindestens einer Aufgabestelle (122) auf das Testelement (114) aufgebracht wird, wobei das Blut oder Bestandteile des Bluts von der Aufgabestelle (122) zu der Testchemie (124) transferiert werden.

9. Vorrichtung (110) zur Bestimmung mindestens einer Konzentration mindestens eines Analyten in Blut, insbesondere zur Bestimmung einer Blutglukosekonzentration, wobei die Vorrichtung (110) mindestens ein Testelement (114) umfasst, wobei das Testelement (114) mindestens eine Testchemie (124) aufweist, wobei die Testchemie (124) eingerichtet ist, um bei Anwesenheit des Analyten mindestens eine optisch nachweisbare Nachweisreaktion durchzuführen, wobei das Blut auf das Testelement (114) aufbringbar ist, wobei die Vorrichtung (110) mindestens eine optische Nachweisvorrichtung (128) aufweist, wobei die optischen Nachweisvorrichtung (128) eingerichtet ist, um einen zeitlichen Verlauf mindestens einer optischen Messgröße der Testchemie (124) zu erfassen, wobei die Vorrichtung (110) weiterhin mindestens eine Auswertevorrichtung (138) aufweist, wobei die Auswertevorrichtung (138) eingerichtet ist, um aus mindestens einem ersten Zeitabschnitt (158) des zeitlichen Verlaufs der optischen Messgröße auf mindestens eine Störgröße des Bluts zu schließen und wobei die Auswertevorrichtung (138) weiterhin eingerichtet ist, um aus mindestens einem zweiten Zeitabschnitt (164) des zeitlichen Verlaufs auf die Konzentration des Analyten zu schließen, wobei ein Zeitabschnitt (158, 164) jeweils eine mindestens zwei Messzeitpunkte umfassende Menge an Messzeitpunkten ist, wobei zur Erfassung der optischen Messgröße in dem ersten Zeitabschnitt (158) und in dem zweiten Zeitabschnitt (164) jeweils mittels mindestens einer Abfragelichtquelle (130) mindestens ein Abfragelichtstrahl (132) auf die Testchemie (124) eingestrahlt werden und wobei jeweils mittels mindestens eines Detektors mindestens ein von der Testchemie (124) ausgehender Antwortlichtstrahl erfasst werden, **dadurch gekennzeichnet, dass** die Störgröße ein Hämatokrit des Bluts ist, wobei der zeitliche Verlauf der optischen Messgröße in dem ersten Zeitabschnitt (158) einen Benetzungssprung (160) der optischen Messgröße umfasst, wobei der Benetzungssprung (160) eine sprunghafte Veränderung der optischen Messgröße ist, welche auf eine Benetzung der Testchemie (124) mit dem Blut zurückzuführen ist, wobei die Auswertevorrichtung (138) eingerichtet ist, um aus dem zeitlichen Verlauf der optischen Messgröße während des ersten Zeitabschnitts (158) mindestens eine charakteristische Größe zu bestimmen, wobei die charakteristische Größe eine Remissionswertänderung während des Benetzungssprungs (160) oder während eines Teils des Benetzungssprungs (160) ist, wobei die Auswertevorrichtung (138) eingerichtet ist, um einen vorgegebenen und/oder bestimmbaren Zusammenhang zwischen der charakteristischen Größe und der Störgröße zu verwenden, um aus der charakteristischen Größe auf die mindestens eine Störgröße zu schließen.

**10.** Vorrichtung (110) nach dem vorhergehenden Anspruch, wobei das Testelement (114) ein Teststreifen (116) ist, wobei die Vorrichtung (110) mindestens eine Teststreifenaufnahme (118) aufweist, wobei mindestens ein Teststreifen (116) in der Teststreifenaufnahme (118) in eine Applikationsposition bringbar ist, wobei in der Applikationsposition mindestens eine Aufgabestelle (122) des Teststreifens (116) durch einen Benutzer mit Blut beaufschlagbar ist, wobei der Teststreifen (116) mindestens ein Kapillarelement (166) aufweist, um das Blut oder Bestandteile des Bluts von der Aufgabestelle zu der Testchemie (124) zu transferieren.

**11.** Vorrichtung (110) nach dem vorhergehenden Anspruch, wobei zwischen dem Kapillarelement (166) und der Testchemie (124) mindestens ein Abtrennelement (176) zur Abtrennung mindestens eines Bestandteils des Bluts aus dem Blut angeordnet ist.

**12.** Vorrichtung (110) nach einem der vorhergehenden Vorrichtungsansprüche, wobei die optische Nachweisvorrichtung (128) eingerichtet ist, um die optische Messgröße in dem ersten Zeitabschnitt (158) und dem zweiten Zeitabschnitt (164) bei derselben Wellenlänge zu erfassen.

## Claims

**1.** Method for determining at least one concentration of at least one analyte in the blood, in particular for determining a blood glucose concentration, wherein a test element (114) is used, wherein the test element (114) contains at least one reagent element (124), wherein the reagent element (124) is configured so as to carry out at least one optically detectable detection reaction in the presence of the analyte, wherein the blood is applied to the test element (114), wherein a time course of at least one optical measurement variable of the reagent element (124) is detected, wherein at least one disturbance variable in the blood is determined from at least one first time interval (158) of the time course of the optical measurement variable, and wherein the concentration of the analyte is determined from at least one second time interval (164) of the time course, wherein a time interval (158, 164) respectively is a quantity of measurement times comprising at least two measurement times, wherein, in order to detect the optical measurement variable in the first time interval (158) and in the second time interval(164), the reagent element (124) is irradiated respectively with at least one interrogating light beam (132) using at least one interrogating light source (130) and wherein at least one response light beam emitted by the reagent element (124) is determined by means of at least one detector respectively, **characterized in that** the disturbance variable is a blood haematocrit, wherein the time course of the optical measurement variable in the first time interval (158) comprises a sudden wetting-induced change (160) in the optical measurement variable, wherein the sudden wetting-induced change (160) is a sudden change in the optical measurement variable that is attributable to wetting of the reagent element (124) with the blood, wherein at least one characteristic variable is determined from the time course of the optical measurement

variable during the first time interval (158), wherein the characteristic variable is a change in the reflectance value during the sudden wetting-induced change (160) or during a portion of the sudden wetting-induced change (160), wherein a preset and/or determinable relation between the characteristic variable and the disturbance variable is used in order to determine the at least one disturbance variable from the characteristic variable.

**2.** Method according to the preceding claim, wherein the first time interval (158) is an initial time interval of the time course, and wherein the second time interval (164) is subsequent to the first time interval (158).

**3.** Method according to one of the preceding claims, wherein the optical measurement variable is determined at at least one first wavelength, and wherein a wetting time point at which the blood reaches and wets the reagent element (124) is determined from a major change in the optical measurement variable after the blood is applied to the test element (114).

**4.** Method according to the preceding claim, wherein the interrogating light beam (132) has the same wavelength and/or the same spectral properties in the first time interval (158) and the second time interval (164) .

**5.** Method according to one of the two preceding claims, wherein the response light beam (136) has the same wavelength and/or the same spectral properties in the first time interval (158) and the second time interval (164).

**6.** Method according to one of the preceding claims, wherein at least one correction is ascertained by means of the disturbance variable, in particular the haematocrit value, and wherein a corrected concentration of the analyte is ascertained from the second time interval (164) taking into account the correction.

**7.** Method according to one of the preceding claims, wherein a change in the optical measurement variable in the second time interval (164) is detected, wherein the optical measurement variable, at a time point at which a temporal change in the optical measurement value is below a preset threshold value, is used to determine the concentration of the analyte.

**8.** Method according to one of the preceding claims, wherein the blood is applied to at least one application site (122) on the test element (114), and wherein the blood or blood components are transferred from the application site (122) to the reagent element (124) .

**9.** Device (110) for determining at least one concentration of at least one analyte in the blood, in particular for determining a blood glucose concentration, wherein the device (110) comprises at least one test element (114), wherein the test element (114) has at least one reagent element (124), wherein the reagent element (124) is configured so as to carry out at least one optically detectable detection reaction in the presence of the analyte, wherein the blood may be applied to the test element (114), wherein the device (110) has at least one optical detection device (128), wherein the optical detection device (128) is configured so as to detect a time course of at least one optical measurement variable of the reagent element (124), wherein the device (110) further has at least one evaluation device (138), wherein the evaluation device (138) is configured so as to determine at least one disturbance variable in the blood from at least one first time interval (158) of the time course of the optical measurement variable, and wherein the evaluation device (138) is further configured so as to determine the concentration of the analyte from at least one second time interval (164) of the time course, wherein a time interval (158, 164) respectively is a quantity of measurement times comprising at least two measurement times, wherein, in order to detect the optical measurement variable in the first time interval (158) and in the second time interval(164), the reagent element (124) is irradiated respectively with at least one interrogating light beam (132) using at least one interrogating light source (130) and wherein at least one response light beam emitted by the reagent element (124) is determined by means of at least one detector respectively, **characterized in that** the disturbance variable is a blood haematocrit, wherein the time course of the optical measurement variable in the first time interval (158) comprises a sudden wetting-induced change (160) in the optical measurement variable, wherein the sudden wetting-induced change (160) is a sudden change in the optical measurement variable that is attributable to wetting of the reagent element (124) with the blood, wherein the evaluation device (138) is configured to determine at least one characteristic variable from the time course of the optical measurement variable during the first time interval (158), wherein the characteristic variable is a change in the reflectance value during the sudden wetting-induced change (160) or during a portion of the sudden wetting-induced change (160), wherein the evaluation device (138) is configured to use a preset and/or determinable relation between the characteristic variable and the disturbance variable in order to determine the at least one disturbance variable from the characteristic variable.

10. Device (110) according to the preceding claim, wherein the test element (114) is a test strip (116), wherein the device (110) has at least one test strip holder (118), wherein at least one test strip (116) in the test strip holder (118) can be placed in an application position, wherein in the application position, a user can apply blood to at least one application site (122) of the test strip (116), wherein the test strip (116) has at least one capillary element (166) for transferring the blood or blood components from the application site to the reagent element (124).

11. Device (110) according to the preceding claim, wherein at least one separating element (176) for separating at least one blood component from the blood is arranged between the capillary element (166) and the reagent element (124).

12. Device (110) according to one of the preceding device claims, wherein the optical detection device (128) is configured so as to detect the optical measurement variable in the first time interval (158) and the second time interval (164) at the same wavelength.

## Revendications

1. Procédé de détermination d'au moins une concentration d'au moins un analyte dans le sang, notamment de détermination d'une concentration de glucose dans le sang, un élément de test (114) étant utilisé, l'élément de test (114) possédant au moins une chimie de test (124), la chimie de test (124) étant conçue pour effectuer au moins une réaction de décèlement optiquement décelable en cas de présence de l'analyte, le sang étant appliqué sur l'élément de test (114), une courbe dans le temps d'au moins une grandeur de mesure optique de la chimie de test (124) étant acquise, la présence d'au moins une grandeur perturbatrice du sang étant déduite à partir d'au moins une première portion de temps (158) de la courbe dans le temps de la grandeur de mesure optique et la concentration de l'analyte étant déduite à partir d'au moins une deuxième portion de temps (164) de la courbe dans le temps, une portion de temps (158, 164) étant respectivement une quantité d'instants de mesure comprenant au moins deux instants de mesure, l'acquisition de la grandeur de mesure optique dans la première portion de temps (158) et dans la deuxième portion de temps (164) s'effectuant par l'incidence d'au moins un rayon lumineux d'interrogation (132) sur la chimie de test (124) respectivement au moyen d'au moins une source de lumière d'interrogation (130) et au moins un rayon lumineux de réponse émanant de la chimie de test (124) étant respectivement acquis au moyen d'au moins un détecteur, **caractérisé en ce que** la grandeur perturbatrice est un hématocrite du sang, la courbe dans le temps de la grandeur de mesure optique dans la première portion de temps (158) comportant un saut de mouillage (160) de la grandeur de mesure optique, le saut de mouillage (160) étant une modification soudaine de la grandeur de mesure optique qui est à attribuer à un mouillage de la chimie de test (124) avec le sang, au moins une grandeur caractéristique étant déterminée à partir de la courbe dans le temps de la grandeur de mesure optique pendant la première portion de temps (158), la grandeur caractéristique étant une modification de la valeur de rémission pendant le saut de mouillage (160) ou pendant une partie du saut de mouillage (160), une relation prédéfinie et/ou pouvant être déterminée entre la grandeur caractéristique et la grandeur perturbatrice étant utilisée en vue de déduire la présence d'au moins une grandeur perturbatrice à partir de la grandeur caractéristique.

2. Procédé selon la revendication précédente, la première portion de temps (158) étant une portion de temps initiale de la courbe dans le temps, la deuxième portion de temps (164) venant chronologiquement après la première portion de temps (158).

3. Procédé selon l'une des revendications précédentes, la grandeur de mesure optique étant acquise à au moins une première longueur d'onde, la présence d'un instant de mouillage, auquel le sang atteint et mouille la chimie de test (124), étant déduite à partir d'une forte variation de la grandeur de mesure optique après l'application du sang sur l'élément de test (114).

4. Procédé selon la revendication précédente, le rayon lumineux d'interrogation (132) possédant la même longueur d'onde et/ou les mêmes propriétés spectrales dans la première portion de temps (158) et dans la deuxième portion de temps (164).

5. Procédé selon l'une des deux revendications précédentes, le rayon lumineux de réponse (136) possédant la même longueur d'onde et/ou les mêmes propriétés spectrales dans la première portion de temps (158) et dans la deuxième portion de temps (164).

6. Procédé selon l'une des revendications précédentes, au moins une correction étant déterminée au moyen de la grandeur perturbatrice, notamment de l'hématocrite, une concentration corrigée de l'analyte étant identifiée à partir

de la deuxième portion de temps (164) en tenant compte de la correction.

7. Procédé selon l'une des revendications précédentes, une variation de la grandeur de mesure optique dans la deuxième portion de temps (164) étant acquise, la détermination de la concentration de l'analyte étant effectuée en utilisant la grandeur de mesure optique à un instant auquel une variation dans le temps de la grandeur de mesure optique est inférieure à une valeur de seuil prédéfinie.

8. Procédé selon l'une des revendications précédentes, le sang étant appliqué au niveau d'au moins un point de chargement (122) sur l'élément de test (114), le sang ou les composantes du sang étant transférés du point de chargement (122) à la chimie de test (124).

9. Dispositif (110) de détermination d'au moins une concentration d'au moins un analyte dans le sang, notamment de détermination d'une concentration de glucose dans le sang, le dispositif (110) comportant au moins un élément de test (114), l'élément de test (114) possédant au moins une chimie de test (124), la chimie de test (124) étant conçue pour effectuer au moins une réaction de décèlement optiquement décelable en cas de présence de l'analyte, le sang pouvant être appliqué sur l'élément de test (114), le dispositif (110) possédant au moins un dispositif de décèlement optique (128), le dispositif de décèlement optique (128) étant conçu pour acquérir une courbe dans le temps d'au moins une grandeur de mesure optique de la chimie de test (124), le dispositif (110) possédant en outre au moins un dispositif d'évaluation (138), le dispositif d'évaluation (138) étant conçu pour déduire la présence d'au moins une grandeur perturbatrice du sang à partir d'au moins une première portion de temps (158) de la courbe dans le temps de la grandeur de mesure optique et le dispositif d'évaluation (138) étant en outre conçu pour déduire la concentration de l'analyte à partir d'au moins une deuxième portion de temps (164) de la courbe dans le temps, une portion de temps (158, 164) étant respectivement une quantité d'instants de mesure comprenant au moins deux instants de mesure, l'acquisition de la grandeur de mesure optique dans la première portion de temps (158) et dans la deuxième portion de temps (164) s'effectuant par l'incidence d'au moins un rayon lumineux d'interrogation (132) sur la chimie de test (124) respectivement au moyen d'au moins une source de lumière d'interrogation (130) et au moins un rayon lumineux de réponse émanant de la chimie de test (124) étant respectivement acquis au moyen d'au moins un détecteur, **caractérisé en ce que** la grandeur perturbatrice est un hématocrite du sang, la courbe dans le temps de la grandeur de mesure optique dans la première portion de temps (158) comportant un saut de mouillage (160) de la grandeur de mesure optique, le saut de mouillage (160) étant une modification soudaine de la grandeur de mesure optique qui est à attribuer à un mouillage de la chimie de test (124) avec le sang, le dispositif d'évaluation (138) étant conçu pour déterminer au moins une grandeur caractéristique à partir de la courbe dans le temps de la grandeur de mesure optique pendant la première portion de temps (158), la grandeur caractéristique étant une modification de la valeur de rémission pendant le saut de mouillage (160) ou pendant une partie du saut de mouillage (160), le dispositif d'évaluation (138) étant conçu pour utiliser une relation prédéfinie et/ou pouvant être déterminée entre la grandeur caractéristique et la grandeur perturbatrice en vue de déduire la présence d'au moins une grandeur perturbatrice à partir de la grandeur caractéristique.

10. Dispositif (110) selon la revendication précédente, l'élément de test (114) étant une bandelette de test (116), le dispositif (110) possédant au moins un logement de bandelette de test (118), au moins une bandelette de test (116) pouvant être amenée dans le logement de bandelette de test (118) dans une position d'application, dans la position d'application au moins un point de chargement (122) de la bandelette de test (116) pouvant être alimenté en sang par un utilisateur, la bandelette de test (116) possédant au moins un élément capillaire (166) pour transférer le sang ou des composantes du sang du point de chargement à la chimie de test (124).

11. Dispositif (110) selon la revendication précédente, au moins un élément de séparation (176) destiné à séparer du sang au moins une composante du sang étant disposé entre l'élément capillaire (166) et la chimie de test (124).

12. Dispositif (110) selon l'une des revendications de dispositif précédentes, le dispositif de décèlement optique (128) étant conçu pour acquérir la grandeur de mesure optique dans la première portion de temps (158) et dans la deuxième portion de temps (164) à la même longueur d'onde.

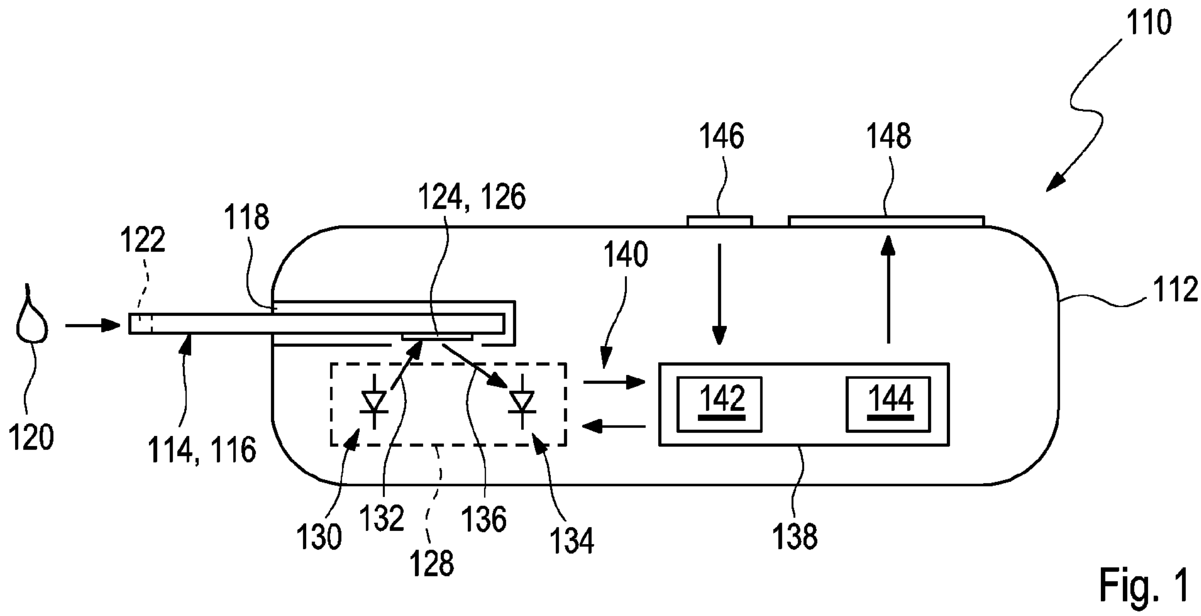


Fig. 1

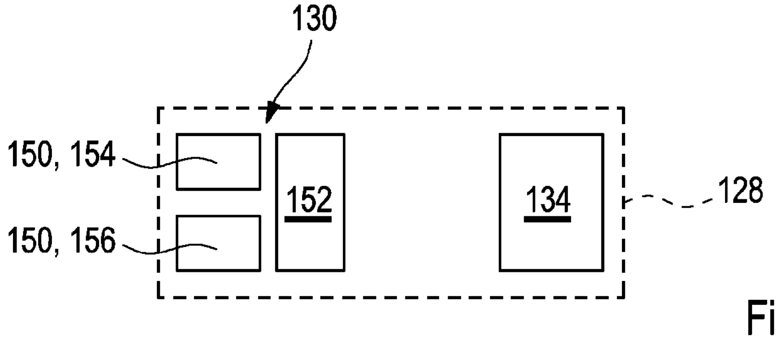


Fig. 2

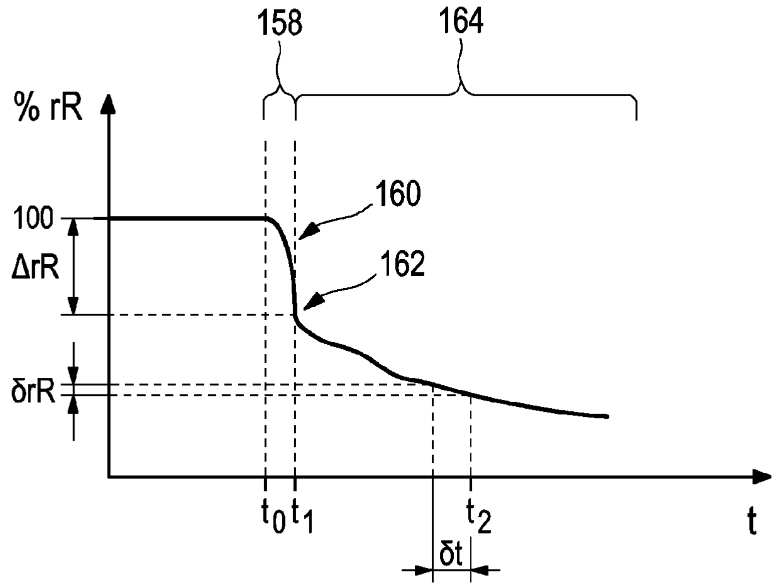


Fig. 3

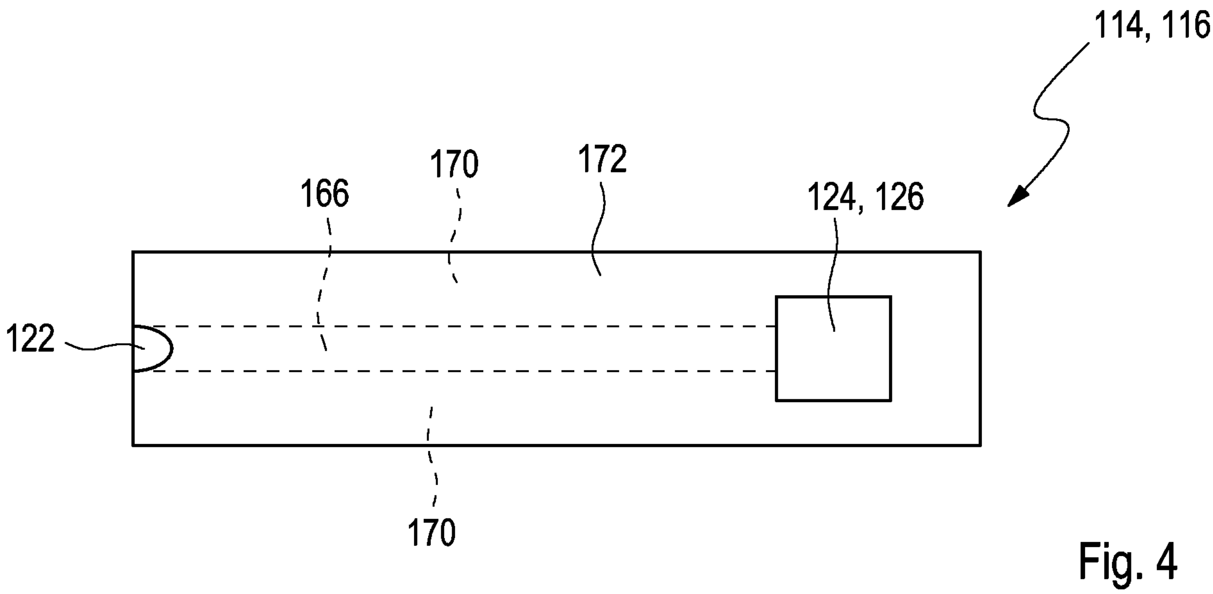
114, 116
166
170
172
124, 126
122
170

Fig. 4

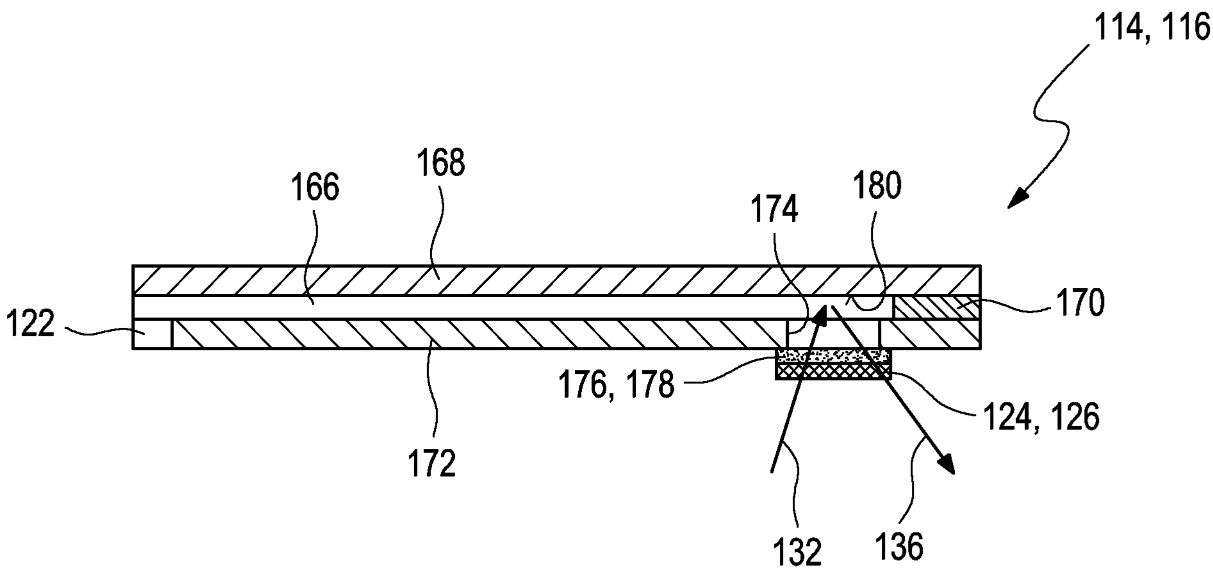
114, 116
166
168
174
180
122
170
176, 178
124, 126
172
132
136

Fig. 5

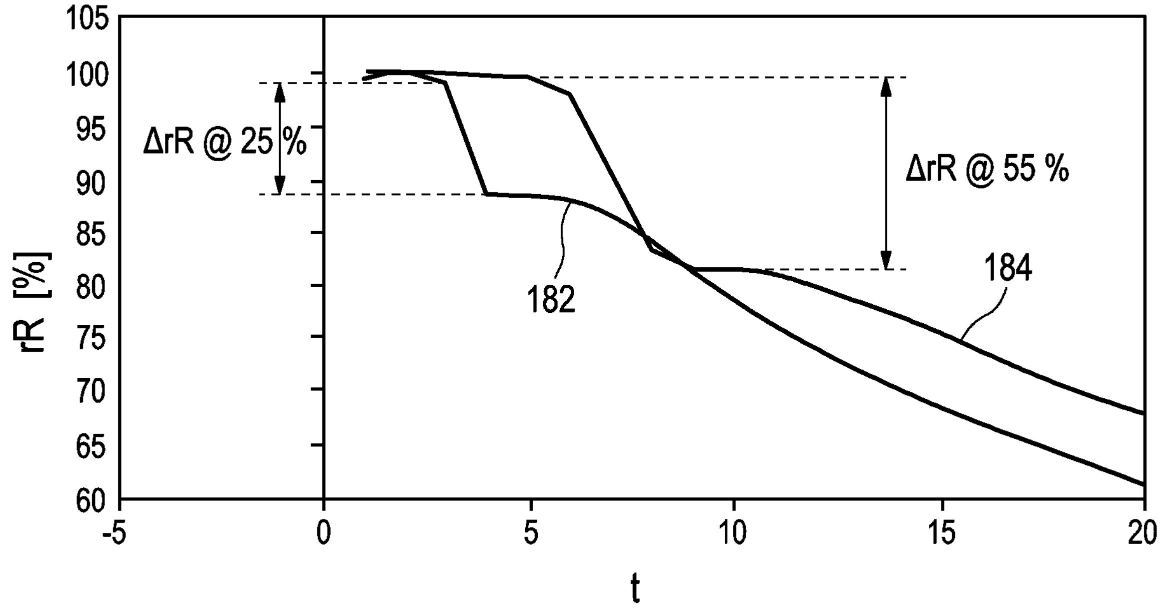
105
100
95
90
85
80
75
70
65
60
rR [%]
ΔrR @ 25 %
ΔrR @ 55 %
182
184
-5
0
5
10
15
20
t

Fig. 6

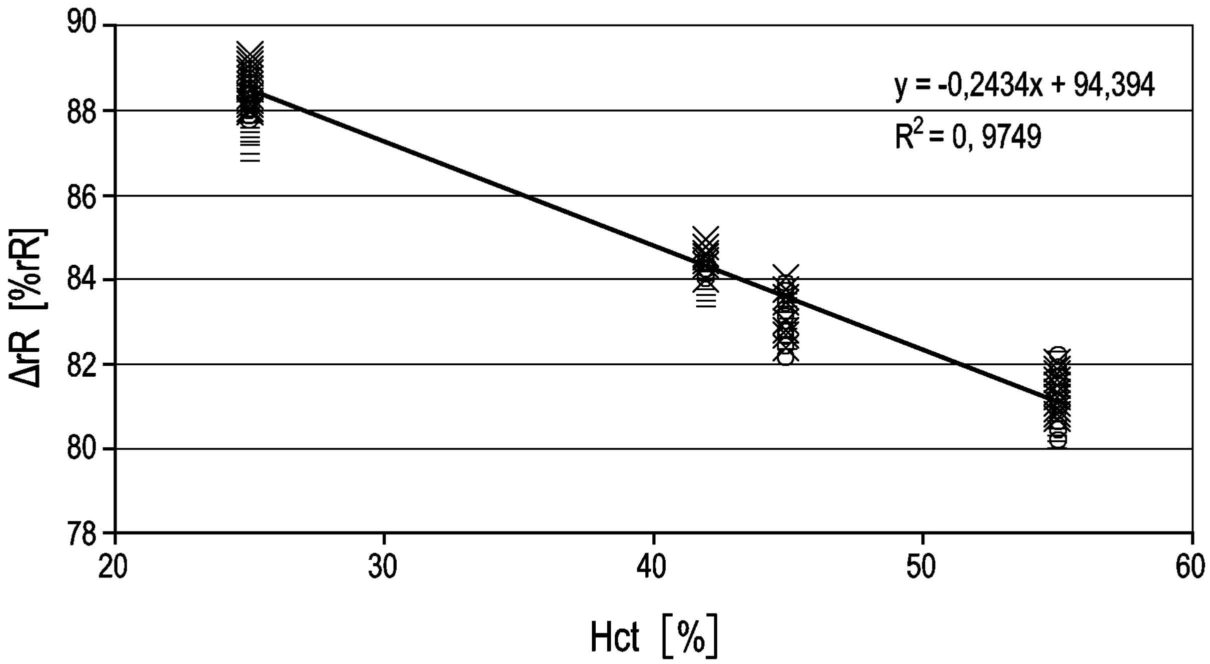
90
88
86
84
82
80
78
ΔrR [%rR]
y = -0,2434x + 94,394
R2 = 0, 9749
20
30
40
50
60
Hct [%]

Fig. 7

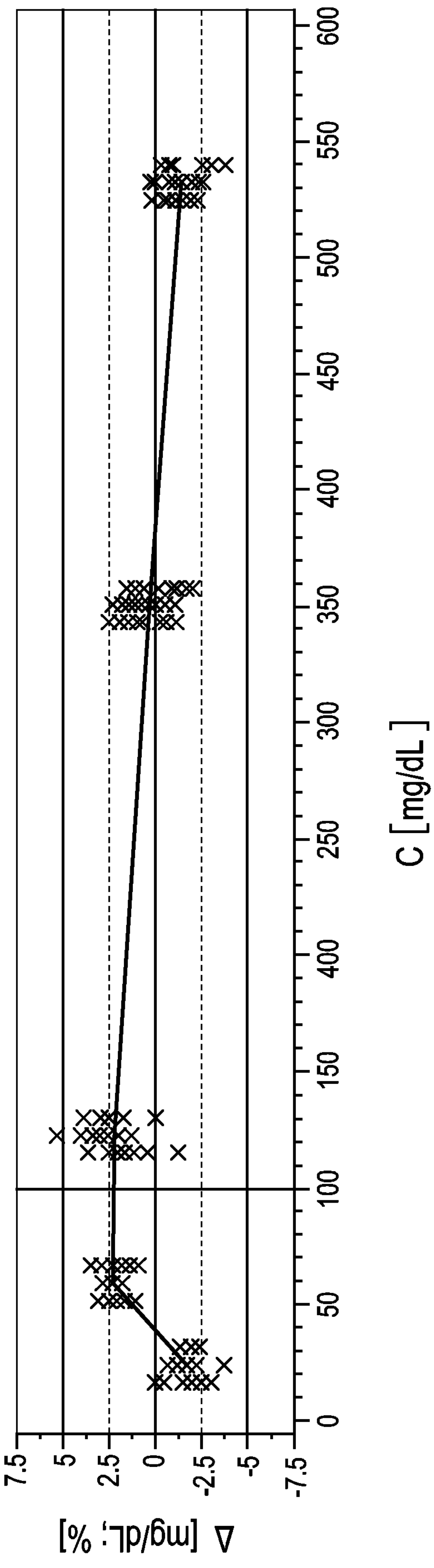
Δ [mg/dL; %]
7.5
5
2.5
0
-2.5
-5
-7.5
C [mg/dL]
0
50
100
150
400
250
300
350
400
450
500
550
600

Fig. 8

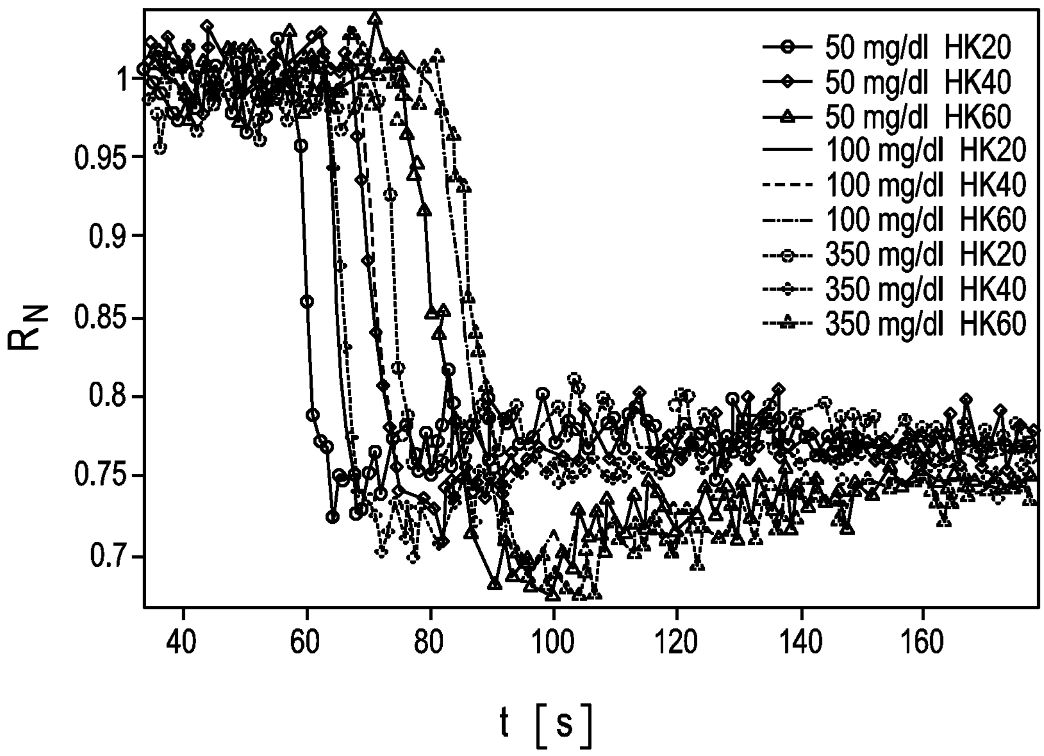
50 mg/dl HK20
50 mg/dl HK40
50 mg/dl HK60
100 mg/dl HK20
100 mg/dl HK40
100 mg/dl HK60
350 mg/dl HK20
350 mg/dl HK40
350 mg/dl HK60
R_N
1
0.95
0.9
0.85
0.8
0.75
0.7
40
60
80
100
120
140
160
t [s]

Fig. 9

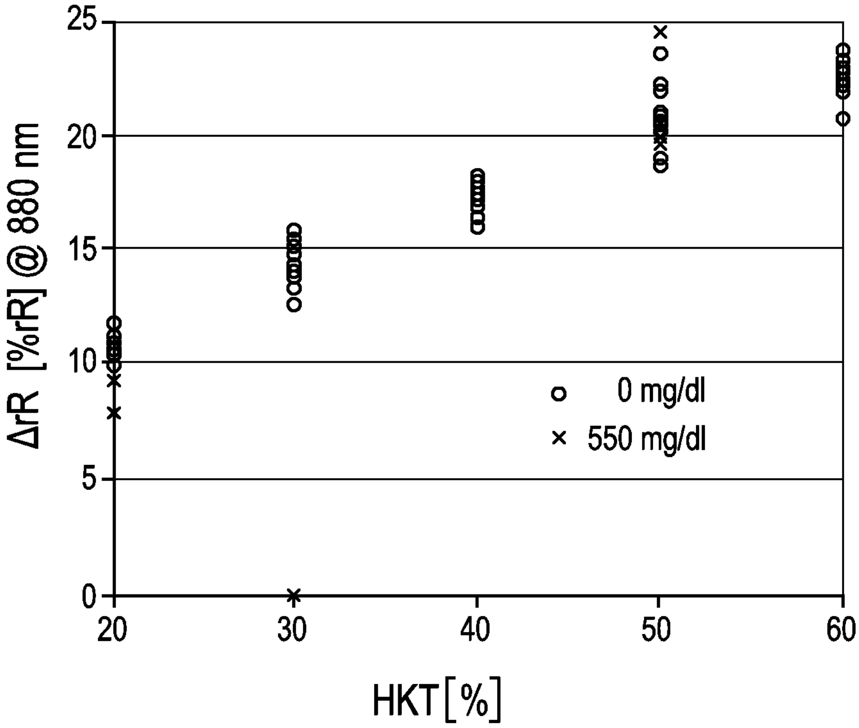
ΔrR [%rR] @ 880 nm
25
20
15
10
5
0
20
30
40
50
60
HKT[%]
0 mg/dl
550 mg/dl

Fig. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010094426 A1
- WO 2010094427 A1
- EP 0302287 A2
- EP 0547710 A2
- EP 1593434 A2
- EP 2325624 A1
- US 5246858 A
- US 5049487 A
- US 20100159570 A1
- JP 2007303968 A
- WO 2006138226 A2
- WO 2008114060 A1
- EP 2259058 A1
- US 4935346 A
- WO 2010052307 A
- EP 0821234 B1
- WO 2007118647 A
- EP 0354441 B1
- WO 2007012494 A1
- WO 2007012494 A
- US 11460366 B
- US 20050214891 A
- EP 1035921 B1
- EP 1039298 B1
- EP 1035919 B1
- EP 1035920 B1


### In der Beschreibung aufgeführte Nicht-Patentliteratur


- **J. HÖNES et al.** *Diabetes Technology and Therapeutics,* 2008, vol. 10 (1), 10-26
- **A. V. KETTELER et al.** Fluorescence Properties of Carba Nicotinamide Adenine Dinucleotide for Glucose Sensing. *ChemPhysChem,* 2012, vol. 13, 1302-1306
- **H. U. BERGMEYER.** Methoden der enzymatischen Analyse. *Verlag Chemie,* 1970, 417
- **BANAUCH et al.** A glucose dehydrogenase for the determination of glucose concentrations in body fluids. *Z. Klin. Chem. Klin. Biochem.,* Marz 1975, vol. 13 (3), 101-7